(19) Europäisches Patentamt / European Patent Office / Office européen des brevets

(11) EP 3 279 289 B1

(12) EUROPÄISCHE PATENTSCHRIFT

(45) Veröffentlichungstag und Bekanntmachung des Hinweises auf die Patenterteilung:
**30.10.2019 Patentblatt 2019/44**

(51) Int Cl.:
***C09K 19/54*** *(2006.01)* ***C07D 211/46*** *(2006.01)*
***C09K 19/30*** *(2006.01)* ***C09K 19/34*** *(2006.01)*

(21) Anmeldenummer: **17184411.1**

(22) Anmeldetag: **02.08.2017**

# (54) PIPERIDIN-DERIVATE UND FLÜSSIGKRISTALLINES MEDIUM

PIPERIDINE DERIVATIVES AND LIQUID CRYSTALLINE MEDIUM

DÉRIVÉS DE PIPERIDINE ET MILIEU CRISTALLIN LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.08.2016 DE 102016009485**

(43) Veröffentlichungstag der Anmeldung:
**07.02.2018 Patentblatt 2018/06**

(73) Patentinhaber: **Merck Patent GmbH**
**64293 Darmstadt (DE)**

(72) Erfinder:
- **Goetz, Achim**
  **64665 ALSBACH-HAEHNLEIN (DE)**
- **Fortte, Rocco**
  **65933 FRANKFURT AM MAIN (DE)**
- **Engel, Martin**
  **64291 DARMSTADT (DE)**
- **Maag, Sabrina**
  **64319 PFUNGSTADT (DE)**
- **Kodek, Thorsten**
  **64546 MOERFELDEN-WALLDORF (DE)**

(56) Entgegenhaltungen:
**EP-A1- 2 982 731** **WO-A1-2015/079797**
**WO-A1-2016/111027** **DE-A1-102015 013 980**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 279 289 B1

## Beschreibung

Die vorliegende Erfindung betrifft Verbindungen der nachfolgend definierten Formel I, flüssigkristalline Medien, welche die Verbindungen der Formel I enthalten, sowie die Verwendung dieser flüssigkristallinen Medien in Flüssigkristallanzeigen.

Das Prinzip der elektrisch kontrollierten Doppelbrechung, der ECB-Effekt oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten $K_3/K_1$, hohe Werte für die optische Anisotropie $\Delta n$ und Werte für die dielektrische Anisotropie $\Delta\varepsilon \leq -0{,}5$ aufweisen müssen, um für hochinformative Anzeigeelemente basierend auf dem ECB-Effekt eingesetzt werden zu können. Auf dem ECB-Effekt basierende elektrooptische Anzeigeelemente weisen eine homöotrope Randorientierung auf (VA-Technologie = Vertical Aligned).

Auch bei Anzeigen, die den so genannten IPS- (In Plane Switching) Effekt verwenden, können dielektrisch negative Flüssigkristallmedien zum Einsatz kommen (S.H. Lee, S. L. Lee, H. Y. Kim, Appl. Phys. Lett. 1998, 73(20), 2881-2883). Entsprechendes gilt auch für Anzeigen, die den so genannten FFS- (Fringe Field Switching) Effekt verwenden.

Für die technische Anwendung dieser Effekte in elektrooptischen Anzeigeelementen werden flüssigkristalline Phasen (FK-Phasen) benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrischen Gleich- und Wechselfeldern.

Ferner wird von technisch verwendbaren FK-Phasen eine flüssigkristalline Mesophase in einem geeigneten Temperaturbereich und eine niedrige Viskosität gefordert.

In den bisher bekannten Reihen von Verbindungen mit flüssigkristalliner Mesophase gibt es keine Einzelverbindung, die allen diesen Erfordernissen entspricht. Es werden daher in der Regel Mischungen von Verbindungen bereitgestellt, um als FK-Phasen verwendbare Materialien zu erhalten.

Matrix-Flüssigkristallanzeigen (MFK-Anzeigen) sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei im Allgemeinen Dünnfilm-Transistoren (TFTs) verwendet werden, die in der Regel auf einer Glasplatte als Substrat angeordnet sind.

Man unterscheidet zwei Technologien, nämlich zwischen TFTs aus Verbindungshalbleitern wie z.B. CdSe und TFTs auf der Basis von polykristallinem und u.a. amorphem Silizium. Letztere Technologie hat derzeit weltweit die größte kommerzielle Bedeutung.

Die TFT-Matrix ist auf der Innenseite der einen Glasplatte der Anzeige aufgebracht, während die andere Glasplatte auf der Innenseite gegebenenfalls die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die bisher am meisten verwendeten TFT-Anzeigen arbeiten üblicherweise mit gekreuzten Polarisatoren in Transmission und sind von hinten beleuchtet. Für TV Anwendungen werden IPS-Zellen oder ECB- (bzw. VAN-) Zellen verwendet, wohingegen für Monitore meist IPS-Zellen oder TN- (Twisted Nematic) Zellen und für "Notebooks", "Laptops" und für mobile Anwendungen meist TN-Zellen Verwendung finden.

Der Begriff MFK-Anzeigen umfaßt Matrix-Displays mit integrierten nichtlinearen Elementen, d.h. neben der aktiven Matrix auch Anzeigen mit passiven Elementen wie Varistoren oder Dioden (MIM = Metall-Isolator-Metall).

MFK-Anzeigen eignen sich insbesondere für TV-Anwendungen, Monitore und "Notebooks" oder für Displays mit hoher Informationsdichte z.B. in Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch einen nicht ausreichend hohen spezifischen Widerstand der Flüssigkristallmischungen, siehe z.B. S. Togashi, K. Sekiguchi, H. Tanabe, E. Yamamoto, K. Sorimachi, E. Tajima, H. Watanabe, H. Shimizu, Proc. Eurodisplay 84, Sept. 1984: A 210-288 Matrix LCD Controlled by Double Stage Diode Rings, S. 141 ff., Paris; M. Stromer, Proc. Eurodisplay 84, Sept. 1984: Design of Thin Film Transistors for Matrix Adressing of Television Liquid Crystal Displays, S. 145 ff., Paris. Mit abnehmendem Widerstand verschlechtert sich der Kontrast einer MFK-Anzeige. Da der spezifische Widerstand der Flüssigkristallmischung durch Wechselwirkung mit den inneren Oberflächen der Anzeige im Allgemeinen über die Lebenszeit einer MFK-Anzeige abnimmt, ist ein hoher (Anfangs-)Widerstand wichtig für Anzeigen, die akzeptable Widerstandswerte über eine lange Betriebsdauer aufweisen müssen.

Anzeigen, die den ECB-Effekt verwenden, haben sich als sogenannte VAN- (Vertically Aligned Nematic) Anzeigen neben IPS-Anzeigen (z.B. S.D. Yeo, Vortrag 15.3: "A LC Display for the TV Application", SID 2004 International Symposium, Digest of Technical Papers, XXXV, Buch II, S. 758 und 759) und den lange bekannten TN-Anzeigen als eine der drei zur Zeit wichtigsten neueren Typen von Flüssigkristallanzeigen insbesondere für Fernsehanwendungen etabliert.

Als wichtigste Bauformen sind hier zu nennen: MVA (Multi-Domain Vertical Alignment, z. B.: H. Yoshide et al., Vortrag 3.1: "MVA LCD for Notebook or Mobile PCs ...", SID 2004 International Symposium, Digest of Technical Papers, XXXV, Buch I, S. 6 bis 9 und C.T. Liu et al., Vortrag 15.1: "A 46-inch TFT-LCD HDTV Technology ...", SID 2004 International Symposium, Digest of Technical Papers, XXXV, Buch II, S. 750 bis 753), PVA (Patterned Vertical Alignment, z. B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763) und ASV (Avanced Super View, z. B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757).

In allgemeiner Form werden die Technologien z.B. in Souk, Jun, SID Seminar 2004, Seminar M-6: "Recent Advances in LCD Technology", Seminar Lecture Notes, M-6/1 bis M-6/26 und Miller, Ian, SID Seminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten insbesondere beim Schalten von Graustufen ein noch nicht zufriedenstellend gelöstes Problem.

ECB-Anzeigen verwenden wie ASV-Anzeigen flüssigkristalline Medien mit negativer dielektrischer Anisotropie ($\Delta\varepsilon$), wohingegen TN- und bislang alle gebräuchlichen IPS-Anzeigen flüssigkristalline Medien mit positiver dielektrischer Anisotropie verwenden. Jedoch können bei Anzeigen, die auf dem IPS- oder FFS-Effekt basieren, alternativ auch dielektrisch negative Flüssigkristallmedien verwendet werden.

In derartigen Flüssigkristallanzeigen werden die Flüssigkristalle als Dielektrika verwendet, deren optische Eigenschaften sich bei Anlegen einer elektrischen Spannung reversibel ändern.

Da bei Anzeigen im Allgemeinen, also auch bei Anzeigen nach diesen erwähnten Effekten, die Betriebsspannung möglichst gering sein soll, werden Flüssigkristallmedien eingesetzt, die in der Regel überwiegend aus Flüssigkristallverbindungen zusammengesetzt sind, die alle das gleiche Vorzeichen der dielektrischen Anisotropie aufweisen und einen möglichst großen Betrag der dielektrischen Anisotropie haben. Es werden in der Regel allenfalls geringere Anteile an neutralen Verbindungen und möglichst keine Verbindungen mit einem Vorzeichen der dielektrischen Anisotropie, das dem des Mediums entgegengesetzt ist, eingesetzt. Bei den Flüssigkristallmedien mit negativer dielektrischer Anisotropie für z.B. ECB-Anzeigen werden somit überwiegend Verbindungen mit negativer dielektrischer Anisotropie eingesetzt. Die eingesetzten Flüssigkristallmedien bestehen in der Regel überwiegend und meist sogar weitestgehend aus Flüssigkristallverbindungen mit negativer dielektrischer Anisotropie.

Für viele praktische Anwendungen in Flüssigkristallanzeigen sind die bekannten Flüssigkristallmedien jedoch nicht stabil genug. Insbesondere ihre Stabilität gegen die Bestrahlung mit UV, aber auch bereits mit den üblichen Hintergrundbeleuchtungen führt zu einer Verschlechterung insbesondere der elektrischen Eigenschaften. So nimmt z. B. die Leitfähigkeit signifikant zu.

Zur Stabilisierung von Flüssigkristallmischungen wurde bereits die Verwendung von sterisch gehinderten Aminen, sogenannten "Hindered Amine Light Stabilizers", kurz HALS, vorgeschlagen.

Nematische Flüssigkristallmischungen mit negativer dielektrischer Anisotropie, die eine geringe Menge an TINUVIN®770, einer Verbindung der Formel

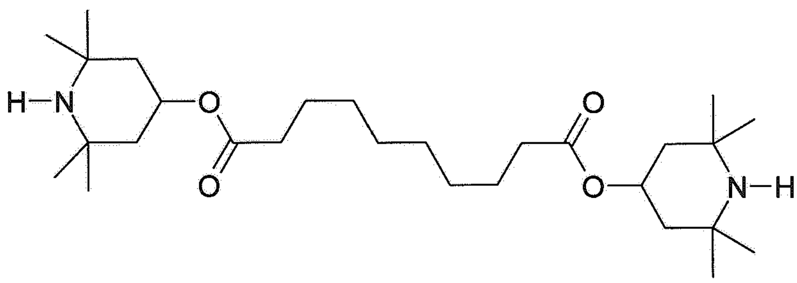

,

als Stabilisator enthalten, werden z.B. in WO 2009/129911 A1 beschrieben. Die entsprechenden Flüssigkristallmischungen haben jedoch für einige praktische Anwendungen nicht ausreichende Eigenschaften. Unter anderem können sie mitunter nicht genügend stabil gegen die Belastung durch die Bestrahlung mit typischen CCFL-(Cold Cathode Fluorescent Lamp) Hintergrundbeleuchtungen sein und/oder Probleme hinsichtlich der Tieftemperaturstabilität aufweisen.

Ähnliche Flüssigkristallmischungen sind z.B. auch aus EP 2 182 046 A1, WO 2008/009417 A1, WO 2009/021671 A1 und WO 2009/115186 A1 bekannt. Diese Flüssigkristallmischungen können gemäß der dortigen Beschreibung optional auch Stabilisatoren verschiedener Arten, wie z. B. Phenole und sterisch gehinderte Amine (HALS), enthalten.

Diese Flüssigkristallmischungen können, je nach Anwendung in unterschiedlichem Ausmaß, ggf. eine Verschlechterung eines oder mehrerer beim Betrieb einer Flüssigkristallanzeige relevanter Parameter zeigen. Insbesondere kann deren Spannungshaltevermögen (Englisch "voltage holding ratio", kurz VHR) nach Belastung sinken. Außerdem kann bei extremen Belastungen eine gelbliche Verfärbung auftreten.

Darüber hinaus wird die Verwendung verschiedener Stabilisatoren in flüssigkristallinen Medien z. B. in JP (S)55-023169 (A), JP (H)05-117324 (A), WO 02/18515 A1 und JP (H) 09-291282 (A) beschrieben. EP 2 982 731 A1, DE 10 2015 013 980 A1, WO 2016/111027 A1 und WO 2015/079797 A1 beschreiben Piperidin-Derivate und deren Verwendung in flüssigkristallinen Medien.

Auch TINUVIN®123, eine Verbindung der Formel

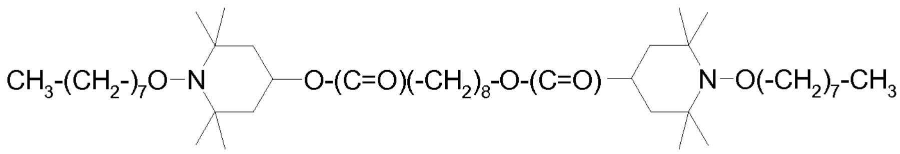

wurde zu Stabilisierungszwecken vorgeschlagen.

HALS mit verschiedenen Substituenten am Stickstoffatom werden in Ohkatsu, Y., J. of Japan Petroleum Institute, 51 (2008), S. 191-204 bezüglich ihrer pKB-Werte verglichen. Dabei werden die folgenden Typen von Strukturformeln beschrieben.

| Typ | Aktive Gruppe des Stabilisators |
|---|---|
| "HALS" | 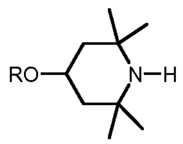 |
| "R-HALS" oder "NR-HALS" | 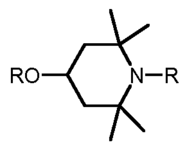 |
| "NOR-HALS" | 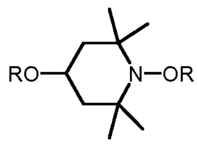 |

Die Verbindung TEMPOL, der folgenden Formel

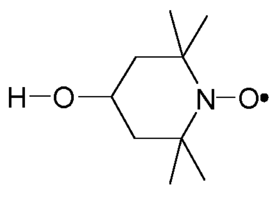

TEMPOL

ist bekannt, sie wird z.B. in Miéville, P. et al., Angew. Chem., 122 (2010), S. 6318-6321 erwähnt. Sie ist von verschiedenen Herstellern kommerziell erhältlich und wird z.B. in Formulierungen für Vorläufer für Polyolefine, Polystyrole, Polyamide, Beschichtungen und PVC als Polymerisationsinhibitor und, insbesondere in Kombination mit UV-Absorbern, als Licht- bzw. UV-Schutz eingesetzt.

Flüssigkristallmedien des Standes der Technik mit entsprechend niedrigen Ansteuerspannungen haben relativ geringe elektrische Widerstände bzw. ein noch nicht ausreichendes, d.h. zu geringes Spannungshaltevermögen (VHR) und führen in den Anzeigen oft zu unerwünschtem "Flicker" und/oder ungenügender Transmission. Außerdem sind sie

nicht ausreichend stabil gegen Temperatur- und/oder UV-Belastung, zumindest dann, wenn sie eine entsprechend hohe Polarität aufweisen, wie sie für niedrige Ansteuerspannungen nötig ist.

Andererseits ist die Ansteuerspannung der Anzeigen des Standes der Technik, die eine hohe VHR aufweisen, oft zu groß, insbesondere für Anzeigen die nicht direkt oder nicht durchgehend an das Stromversorgungsnetz angeschlossen werden, wie z. B. Anzeigen für mobile Anwendungen.

Außerdem muss der Phasenbereich der Flüssigkristallmischung ausreichend breit für beabsichtigte Anzeige-Anwendungen sein.

Des Weiteren sind geeignete Schaltzeiten der Flüssigkristallmedien in den Anzeigen zu beachten. Dies ist besonders für Anzeigen für Fernseh- oder Multimedia-Anwendungen wichtig. Zur Verbesserung der Schaltzeiten ist in der Vergangenheit wiederholt vorgeschlagen worden, die Rotationsviskosität ($\gamma_1$) der Flüssigkristallmedien zu optimieren, also Medien mit einer möglichst geringen Rotationsviskosität zu realisieren. Die dabei erzielten Ergebnisse sind jedoch für viele Anwendungen nicht ausreichend.

Darüber hinaus ist eine ausreichende Stabilität der Medien gegen extreme Belastungen, insbesondere gegen UV- und/oder Temperaturbelastung sicherzustellen, besonders bei Anwendungen in Anzeigen in mobilen Geräten wie z. B. Mobiltelefonen.

Ein Nachteil bisher bekannter MFK-Anzeigen beruht auf ihrem vergleichsweise niedrigen Kontrast, der relativ hohen Blickwinkelabhängigkeit und der Schwierigkeit, in diesen Anzeigen Graustufen zu erzeugen, sowie ihrer ungenügenden VHR und ihrer ungenügenden Lebensdauer.

Es besteht somit immer noch ein großer Bedarf nach MFK-Anzeigen mit sehr hohem spezifischen Widerstand bei gleichzeitig großem Arbeitstemperaturbereich, kurzen Schaltzeiten und niedriger Schwellenspannung, mit deren Hilfe verschiedene Graustufen erzeugt werden können und die insbesondere eine gute und stabile VHR aufweisen.

Der Erfindung liegt die Aufgabe zugrunde, Verbindungen bereitzustellen, die in flüssigkristallinen Mischungen derart zu vorteilhaften Eigenschaften beitragen, dass nützliche und verbesserte MFK-Anzeigen, insbesondere welche auf dem ECB- oder auf dem IPS- oder FFS-Effekt beruhen und auch mobile Anwendungen umfassen, erhältlich werden. Insbesondere besteht eine Aufgabe darin, Verbindungen und flüssigkristalline Medien und diese Medien enthaltende MFK-Anzeigen bereitzustellen, die die oben angegebenen Nachteile nicht oder nur in geringerem Maße und gleichzeitig sehr hohe spezifische Widerstände aufweisen, wobei ferner ein Arbeiten, besonders für mobile Systeme, auch bei extrem hohen und extrem niedrigen Temperaturen ermöglicht wird.

Diese Aufgabe wird durch den Gegenstand der unabhängigen Ansprüche gelöst. Weiterbildungen sind in den entsprechenden Unteransprüchen angegeben, und die Gegenstände, Weiterbildungen und besonderen Merkmale der vorliegenden Erfindung werden im Folgenden beschrieben.

Ein erster Gegenstand der vorliegenden Erfindung ist eine Verbindung der Formel I

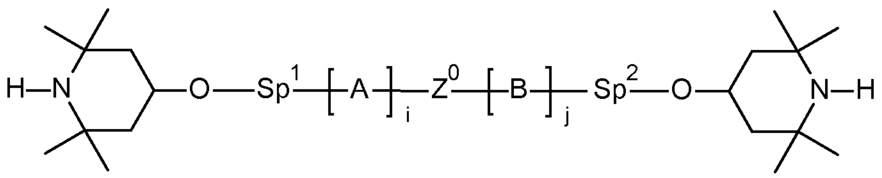

I

wobei

Sp$^1$ und Sp$^2$ die in Anspruch 1 angegebene Bedeutung haben, und

A und B jeweils unabhängig voneinander einen Rest ausgewählt aus den folgenden Gruppen

a) der Gruppe bestehend aus trans-1,4-Cyclohexylen, 1,4-Cyclohexenylen und 1,4'-Bicyclohexylen, worin auch eine oder zwei nicht benachbarte $CH_2$-Gruppen durch -O- und/oder -S- ersetzt sein können und worin auch ein oder mehrere H-Atome durch F ersetzt sein können,

b) der Gruppe bestehend aus 1,4-Phenylen und 1,3-Phenylen, worin auch eine oder zwei nicht benachbarte CH-Gruppen durch N ersetzt sein können und worin auch ein oder mehrere H-Atome durch L ersetzt sein können, und

c) der Gruppe bestehend aus

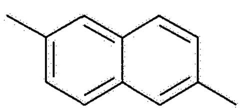

,

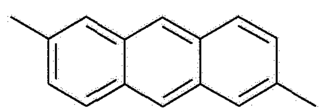

,

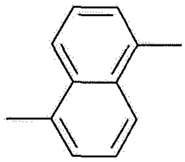,

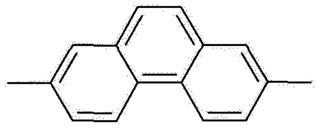,

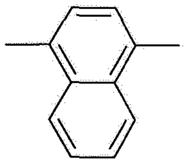,

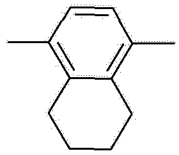,

wobei in diesen Resten auch ein oder mehrere H-Atome durch L ersetzt sein können, und/oder eine oder mehrere Doppelbindungen durch
Einfachbindungen ersetzt sein können, und/oder eine oder mehrere CH-Gruppen durch N ersetzt sein können,

i und j — jeweils unabhängig voneinander 0 oder 1,

$Z^0$ — eine Einfachbindung, $-CH_2-$, $-CF_2-$, -CO-, -O-, -NH-, -NH-(CO)-, $-CH_2CH_2-$, -CH=CH-, $-CF_2O-$, $-OCF_2-$, $-CH_2O-$, $-OCH_2-$, -COO-, -OCO-, $-C_2F_4-$ oder -CF=CF-, vorzugsweise eine Einfachbindung, $-CF_2O-$ oder -COO-,

und

L — bei jedem Auftreten gleich oder verschieden F, Cl, CN oder geradkettiges oder verzweigtes, jeweils optional fluoriertes, Alkyl, Alkoxy, Arylalkyl oder Alkylarylalkyl mit 1 bis 15 C-Atomen, worin auch eine oder mehrere nicht benachbarte $CH_2$-Gruppen durch -O- und/oder -S- ersetzt sein können,

bedeuten.

B bedeutet vorzugsweise trans-1,4-Cyclohexylen, worin auch eine oder mehrere nicht benachbarte $CH_2$-Gruppen durch -O- und/oder -S- ersetzt sein können und worin auch ein oder mehrere H-Atome durch F ersetzt sein können, oder 1,4-Phenylen, worin auch eine oder zwei nicht benachbarte CH-Gruppen durch N ersetzt sein können und worin auch ein oder mehrere H-Atome durch L ersetzt sein können.

In der vorliegenden Erfindung wurde vorteilhaft erkannt, dass die erfindungsgemäßen Verbindungen gemäß Formel I überraschenderweise günstige Eigenschaften aufweisen und insbesondere geeignet sind, Flüssigkristallmischungen sowohl gegen UV-Belastung als auch gegen Temperaturbelastung erheblich, in vielen Fällen ausreichend, zu stabilisieren.

Es wurde ferner gefunden, dass die Verbindungen gemäß Formel I eine ausreichende Polarität sowie Löslichkeit in flüssigkristallinen Medien besitzen. Darüber hinaus können sie eine ausreichende molekulare Flexibilität und günstige Wechselwirkung und Verankerung mit Oberflächen aufweisen. Diese Kombination von Eigenschaften kann vorteilhaft dazu beitragen, eine effektive und effiziente Stabilisierung von flüssigkristallinen Medien zu erreichen.

Es hat sich ferner überraschend gezeigt, dass die erfindungsgemäßen Verbindungen insbesondere geeignet sind, flüssigkristalline Medien mit einer nematischen Phase und einer negativen dielektrischen Anisotropie zu stabilisieren, insbesondere in Gegenwart einer Orientierungsschicht aus z.B. Polyimid.

Ein weiterer Gegenstand der vorliegenden Erfindung betrifft somit die Verwendung einer oder mehrerer Verbindungen gemäß Formel I zur Stabilisierung eines flüssigkristallinen Mediums, vorzugsweise eines flüssigkristallinen Mediums mit einer nematischen Phase und einer negativen dielektrischen Anisotropie.

Als einen weiteren Gegenstand stellt die vorliegende Erfindung ein Verfahren zur Herstellung der erfindungsgemäßen Verbindungen gemäß Formel I zur Verfügung, wobei in dem Verfahren 4-Hydroxy-2,2,6,6-tetramethylpiperidinyloxyl, auch 4-Hydroxy-TEMPO oder TEMPOL genannt, verwendet wird.

In der vorliegenden Erfindung wurde erkannt, dass die erfindungsgemäßen Verbindungen der Formel I in einem überraschend effizienten Verfahren hergestellt werden können, indem TEMPOL als ein Ausgangsstoff eingesetzt wird. Die vorteilhaften Verbindungen gemäß Formel I können somit in zweckmäßiger und nützlicher Weise durch die Verwendung von TEMPOL erhalten werden bzw. sind sie dadurch erhätlich.

Ein weiterer Gegenstand der vorliegenden Erfindung ist ein flüssigkristallines Medium, welches eine oder

mehrere erfindungsgemäße Verbindungen nach Formel I und eine oder mehrere mesogene Verbindungen enthält.

Es wurde vorteilhaft erkannt, dass die Verbindungen gemäß Formel I flüssigkristalline Medien überraschend effektiv und effizient stabilisieren können, insbesondere gegen UV- und/oder Temperaturbelastung. Somit werden erfindungsgemäß flüssigkristalline Medien bereitgestellt, welche eine zuverlässige Funktionsweise und Leistungsfähigkeit auch unter anspruchsvolleren Bedingungen und über längere Zeiträume ermöglichen. So können zum Beispiel unerwünschte Anzeigeeffekte wie das sogenannte Image Sticking oder Mura auch unter extremen Bedingungen oder nach extremen Belastungen vermieden oder deutlich verringert werden.

Eine ausreichende Stabilisierung von Flüssigkristallmischungen sowohl gegen UV-Belastung als auch gegen Temperaturbelastung kann insbesondere auch dann erreicht werden, wenn zusätzlich zu der Verbindung der Formel I oder den Verbindungen der Formel I eine oder mehrere weitere Verbindungen, bevorzugt phenolische Stabilisatoren, in der Flüssigkristallmischung enthalten sind. Diese weiteren Verbindungen werden bevorzugt als Stabilisatoren gegen thermische Belastungen verwendet.

Ein weiterer Gegenstand betrifft elektrooptische Anzeigen oder elektrooptische Komponenten, welche ein erfindungsgemäßes Medium enthalten.

Überraschend wurde gefunden, dass Flüssigkristallanzeigen realisiert werden können, die gute Stabilität gegen Zersetzung durch thermische und durch UV-Belastung und eine stabile hohe VHR aufweisen, u.a. auch schon die initiale VHR, wenn man in diesen Anzeigevorrichtungen die erfindungsgemäßen Flüssigkristallmischungen verwendet.

Die erfindungsgemäßen Medien eignen sich insbesondere für elektrooptische Anzeigen mit einer Aktivmatrix-Adressierung basierend auf dem ECB-Effekt sowie für IPS- und FFS-Anzeigen.

Ohne die vorliegende Erfindung damit einzuschränken, soll die nachfolgende detaillierte Beschreibung der Gegenstände, Weiterbildungen und besonderen Merkmale die Erfindung anschaulich darstellen
und besondere Ausführungsformen detaillierter beschreiben.

Die vorliegende Erfindung stellt Verbindungen der Formel I zur Verfügung, welche vorteilhafte Eigenschaften für die Stabilisierung von insbesondere flüssigkristallinen Medien aufweisen.

Hierbei weisen die erfindungsgemäßen Verbindungen jeweils zwei Tetramethylpiperidinylgruppen auf, welche jeweils über eine Etherbindung sowie die weiteren in der Formel I angegebenen Gruppen miteinander verknüpft sind. Es werden also vorteilhaft zwei ether-gebundene HALS-Gruppen, also spezifische sterisch gehinderte sekundäre Amine, welche keine Wasserstoffatome an den $\alpha$-C-Atomen tragen, pro Molekül so bereitgestellt, dass sie Flüssigkristallmischungen sowohl gegen UV-Belastung als auch gegen Temperaturbelastung effektiv und effizient stabilisieren können.

Die vorliegend bereitgestellte Kombination aus Strukturelementen gemäß Formel I, insbesondere den beiden HALS-Gruppen, den direkten Etherbindungen sowie den weiteren definierten Gruppen, u.a. die Abstandsgruppen und ggf. laterale Substituenten, bietet neben der unmittelbaren Stabilisierungswirkung durch die HALS Gruppen ausreichende Polarität und Löslichkeit in flüssigkristallinen Medien sowie eine ausreichende molekulare Flexibilität und gegebenenfalls eine günstige Wechselwirkung und Verankerung mit Oberflächen.

Diese Kombination von Eigenschaften kann vorteilhaft dazu beitragen, eine ausreichende Stabilisierung von flüssigkristallinen Medien zu erreichen. Es wurde insbesondere erkannt, dass die Verwendung der erfindungsgemäßen Verbindungen und von Medien, welche diese Verbindungen enthalten, in Flüssigkristallanzeigen vorteilhaft ist, worin Oberflächeneinflüsse, z.B. ausgehend von Orientierungsschichten aus Polyimid und die damit verbundenen Wechselwirkungen, beachtlich sein können.

Gemäß der Formel I bedeuten $Sp^1$ und $Sp^2$ jeweils unabhängig voneinander eine wie in Anspruch 1 definierte Abstandsgruppe. Der Begriff "Abstandsgruppe" (engl. "spacer" oder "spacer group") ist dem Fachmann bekannt und wird in der Literatur beschrieben, siehe beispielsweise Pure Appl. Chem. 73(5), 888 (2001) und C. Tschierske, G. Pelzl, S. Diele, Angew. Chem. 2004, 116, 6340-6368.

Hierbei sind die Abstandsgruppen vorteilhaft und bevorzugt flexible Verknüpfungs- oder Brückengruppen, d.h. flexible Segmente. Gemäß der vorliegenden Erfindung sind $Sp^1$ und $Sp^2$ jeweils unabhängig voneinander geradkettiges oder verzweigtes Alkylen, bevorzugt geradkettiges Alkylen, mit 1 bis 12 C-Atomen, welches optional durch F, Cl oder CN ein- oder mehrfach substituiert ist, und worin auch eine oder mehrere nicht benachbarte $CH_2$-Gruppen jeweils unabhängig voneinander durch -O-, -S-, -NH-, -CH=CH- oder -C=C- ersetzt sein können. Dabei erfolgt die optionale Ersetzung so, dass im Molekül keine zwei O-Atome direkt miteinander verbunden sind.

Typische und besonders bevorzugte Abstandsgruppen $Sp^1$ und $Sp^2$, jeweils unabhängig voneinander, sind beispielsweise $-(CH_2)_{p1}-$, $-(CH_2CH_2O)_{q1}-CH_2CH_2-$, $-CH_2CH_2-S-CH_2CH_2-$ und $-CH_2CH_2-NH-CH_2CH_2-$, worin p1 eine ganze Zahl von 1 bis 12, besonders bevorzugt 2 bis 12, und q1 eine ganze Zahl von 1 bis 3 ist.

Besonders bevorzugte Gruppen sind beispielsweise geradkettiges Ethylen, Propylen, Butylen, Pentylen, Hexylen, Heptylen, Octylen, Nonylen, Decylen, Undecylen, Dodecylen, Ethylenoxyethylen, Methylenoxybutylen, Ethylenthioethylen, Ethylen-N-methyl-iminoethylen, 1-Methylalkylen, Ethenylen, Propenylen und Butenylen.

In einer besonders bevorzugten Ausführungsform bedeuten $Sp^1$ und $Sp^2$, jeweils unabhängig voneinander, Ethylen, Propylen, Butylen, Pentylen, Hexylen, Heptylen, und insbesondere Propylen.

Es ist bevorzugt, dass $Sp^1$ und $Sp^2$ die gleiche Bedeutung haben, d.h. die gleiche Struktur aufweisen.

In einer Ausführungsform bedeutet

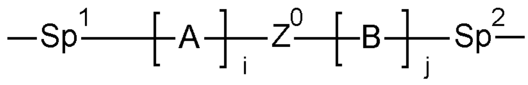

geradkettiges oder verzweigtes Alkylen, vorzugsweise mit 9 bis 16 C-Atomen, welches optional durch F, Cl oder CN ein- oder mehrfach substituiert ist, und worin auch eine oder mehrere nicht benachbarte $CH_2$-Gruppen jeweils unabhängig voneinander durch -O-, -S-, -NH-, -CO-, -COO-, -OCO-, -OCO-O-, -CH=CH- oder -C=C- ersetzt sein können.

Gemäß einer bevorzugten Ausführungsform ist

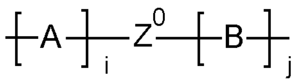

eine Gruppe ausgewählt aus -Cyc-, -Phe-, -Cyc-Cyc-, -Cyc-Phe-, -Phe-Cyc-, -Phe-Phe-, -Cyc-Z-Cyc-, -Cyc-Z-Phe-, -Phe-Z-Cyc- und -Phe-Z-Phe-, wobei

Cyc trans-1,4-Cyclohexylen bedeutet, worin auch eine oder mehrere nicht benachbarte $CH_2$-Gruppen durch -O- und/oder -S- ersetzt sein können und worin auch ein oder mehrere H-Atome durch F ersetzt sein können, Phe 1,3-Phenylen oder 1,4-Phenylen, bevorzugt 1,4-Phenylen, bedeutet, worin auch eine oder zwei nicht benachbarte CH-Gruppen durch N ersetzt sein können und worin auch ein oder mehrere H-Atome durch L ersetzt sein können,

Z $-CH_2-$, $-CF_2-$, -CO-, -O-, -NH-, -NH-(CO)-, $-CH_2CH_2-$, -CH=CH-, $-CF_2O-$, $-OCF_2-$, $-CH_2O-$, $-OCH_2-$, -COO-, -OCO-, $-C_2F_4-$ oder -CF=CF- bedeutet, und L die oben für Formel I angegebene Bedeutung hat.

Bevorzugt weist

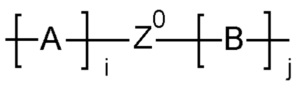

eine der folgenden Strukturen auf

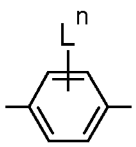

,

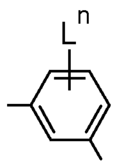

,

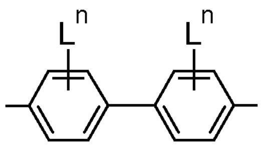

,

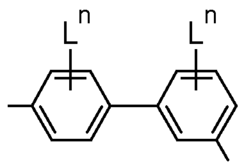

,

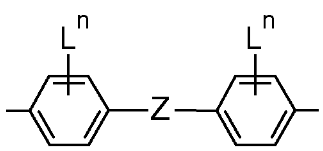

,

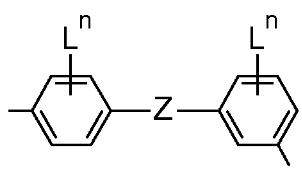

,

wobei

Z $-CH_2-$, $-CF_2-$, -CO-, -O-, -NH-, -NH-(CO)-, $-CH_2CH_2-$, -CH=CH-, $-CF_2O-$, $-OCF_2-$, $-CH_2O-$, $-OCH_2-$, -COO-, -OCO-, $-C_2F_4-$ oder -CF=CF-, vorzugsweise $-CF_2O-$ oder -COO-, bedeutet, und

$L^n$ jeweils unabhängig voneinander 1, 2, 3 oder 4, vorzugsweise 1 oder 2, optionale Substituenten L bedeutet, wobei L bei jedem Auftreten gleich oder verschieden F, Cl, CN oder geradkettiges oder verzweigtes, jeweils optional

fluoriertes, Alkyl, Alkoxy, Arylalkyl oder Alkylarylalkyl mit 1 bis 15 C-Atomen bedeutet, worin auch eine oder mehrere nicht benachbarte CH2-Gruppen durch -O- und/oder -S- ersetzt sein können.

Gemäß einer Ausführungsform umfasst die Gruppe

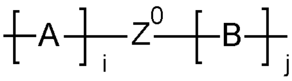

eine der folgenden Strukturen

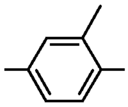 , 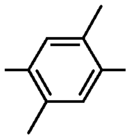 , 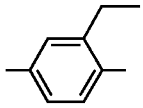 ,

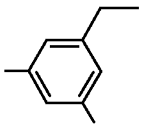 , 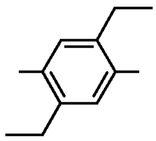 , 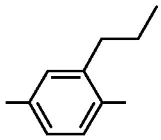 ,

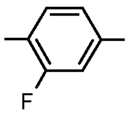 , 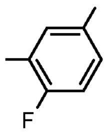 , 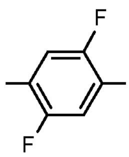 ,

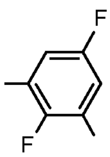 , 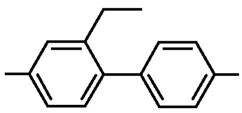 , 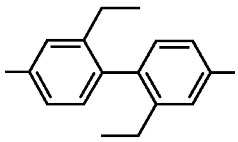 ,

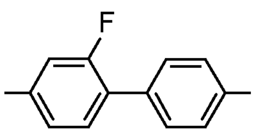 , 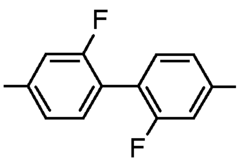 , 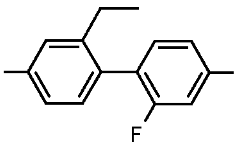 ,

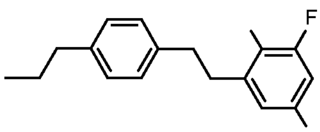 , 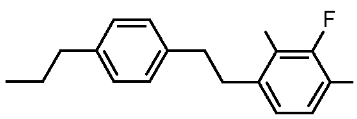 ,

wobei die Gruppe vorzugsweise aus einer dieser Strukturen besteht.

Gemäß einer bevorzugten Ausführungsform werden Verbindungen der Formel I bereitgestellt, wobei i=1 und j=0 sind.

Eine besonders bevorzugte Ausführungsform der Erfindung sieht vor, dass die erfindungsgemäße(n) Verbindung(en) ausgewählt ist (sind) aus Verbindungen der Formel I-A

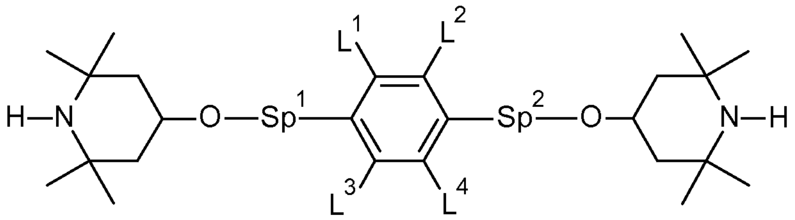

I-A,

wobei $Sp^1$ und $Sp^2$ die oben angegebenen Bedeutungen haben und $L^1$ bis $L^4$ jeweils unabhängig voneinander H, F oder geradkettiges oder verzweigtes, jeweils optional fluoriertes, Alkyl mit 1 bis 7 C-Atomen, worin auch eine oder mehrere nicht benachbarte $CH_2$-Gruppen durch -O- ersetzt sein können, bedeuten.

Insbesondere bedeuten $L^1$ bis $L^4$ besonders bevorzugt jeweils unabhängig voneinander H, F oder geradkettiges Alkyl mit 1 bis 6 C-Atomen. Gemäß einer Ausführungsform sind mindestens zwei von $L^1$ bis $L^4$ H, wobei die restliche(n) Gruppe(n) von $L^1$ bis $L^4$ ausgewählt ist (sind) aus F, Methyl, Ethyl und Propyl.

Für den Fall in dem i=0 und j=0 sind, bilden gemäß einer bevorzugten Ausführungsform die Gruppen $Sp^1$, $Z^0$ und $Sp^2$ zusammen eine unverzweigte Alkylgruppe, vorzugsweise mit mindestens 9 C-Atomen, weiter bevorzugt mit 9 bis 16 C-Atomen.

Besonders bevorzugte Verbindungen der Formel I sind ausgewählt aus den folgenden Verbindungen der Formeln I-1 bis I-12:

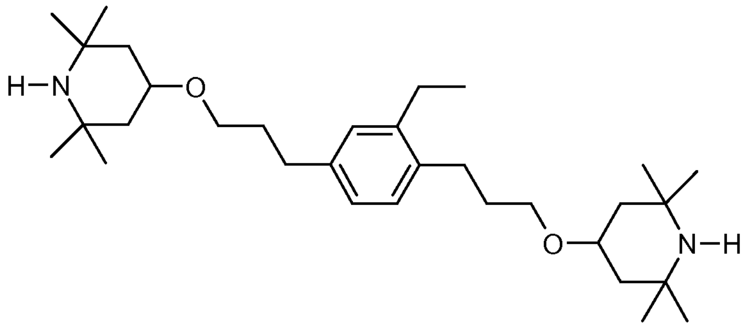

I-1

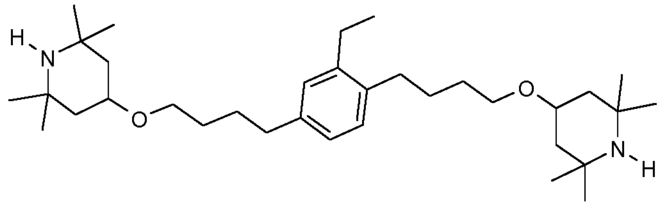

I-2

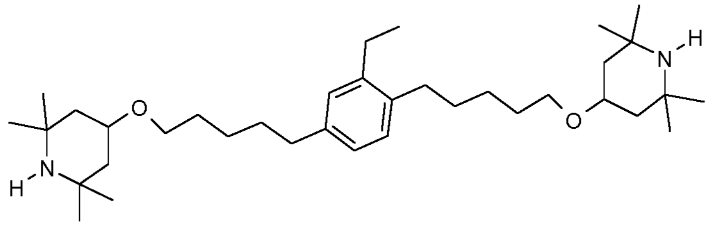

I-3

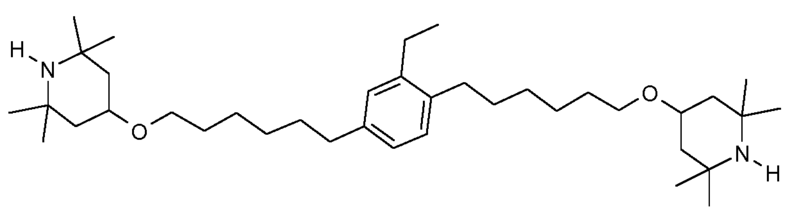

I-4

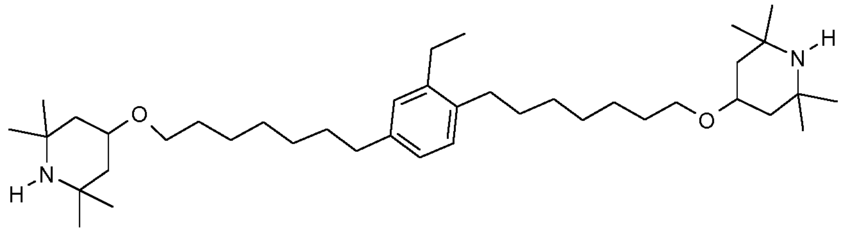

I-5

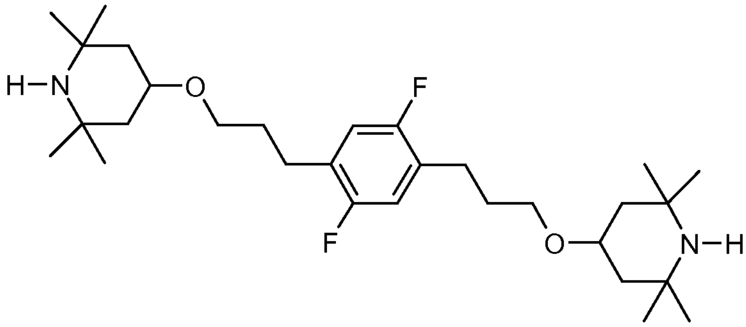

I-6

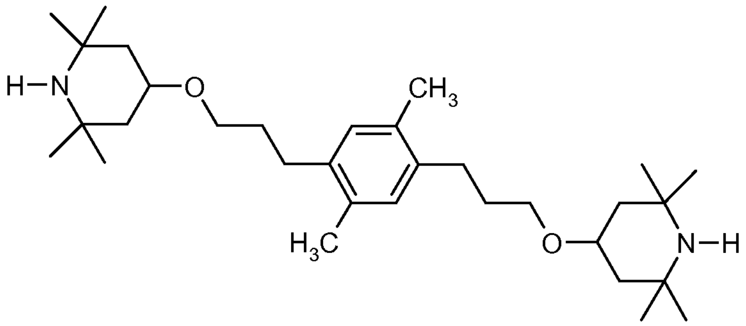

I-7

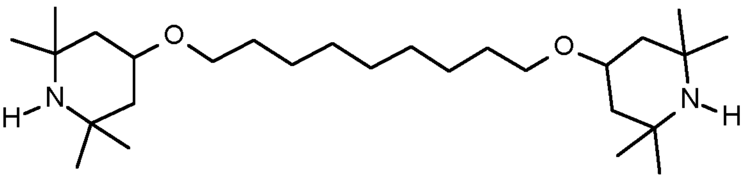

I-8

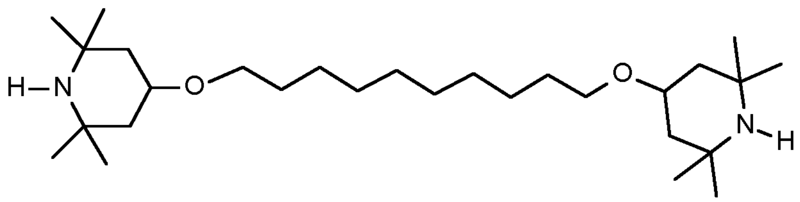

I-9

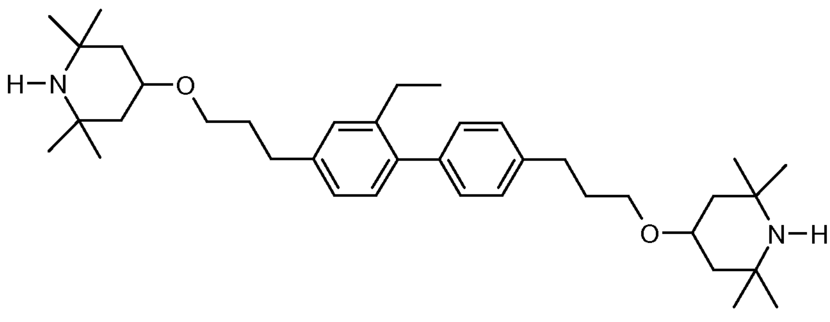

I-10

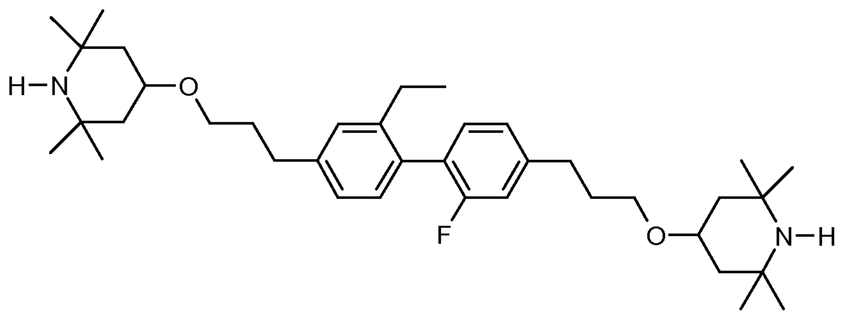

I-11

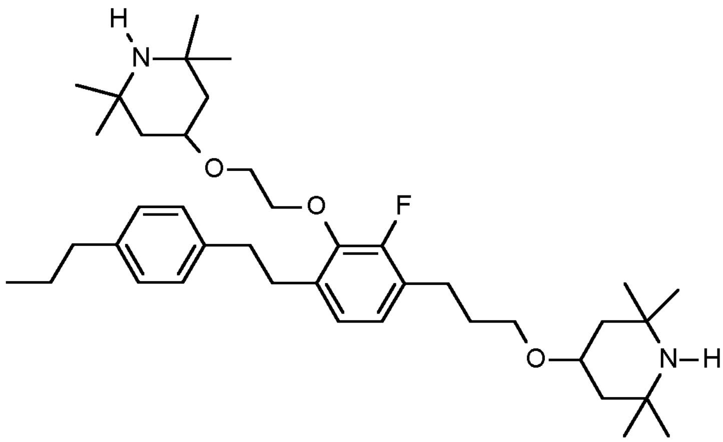

I-12

Gemäß einer besonders bevorzugten Ausführungsform ist die Verbindung der Formel I eine Verbindung der Formel I-1, I-2, I-3 I-4, I-5, I-6 oder I-7, insbesondere I-1.

Diese Verbindungen eignen sich besonders hervorragend als Stabilisatoren in Flüssigkristallmischungen, wobei sie insbesondere die VHR der Mischungen gegen UV-Belastung stabilisieren.

Die erfindungsgemäßen Verbindungen der Formel I können nach üblichen literaturbekannten Verfahren und gegebenenfalls dem Fachmann gängigen Abwandlungen davon hergestellt werden (siehe z.B. Houben Weyl, Methoden der Organischen Chemie, Thieme-Verlag, Stuttgart).

Vorliegend wurde vorteilhaft erkannt, dass ein besonders günstiges und effektives Verfahren zur Herstellung der Verbindungen der Formel I bereitgestellt werden kann, indem TEMPOL als ein Ausgangsstoff verwendet wird.

Ein allgemeines Syntheseschema zur Herstellung von erfindungsgemäßen Verbindungen ist nachfolgend gezeigt.

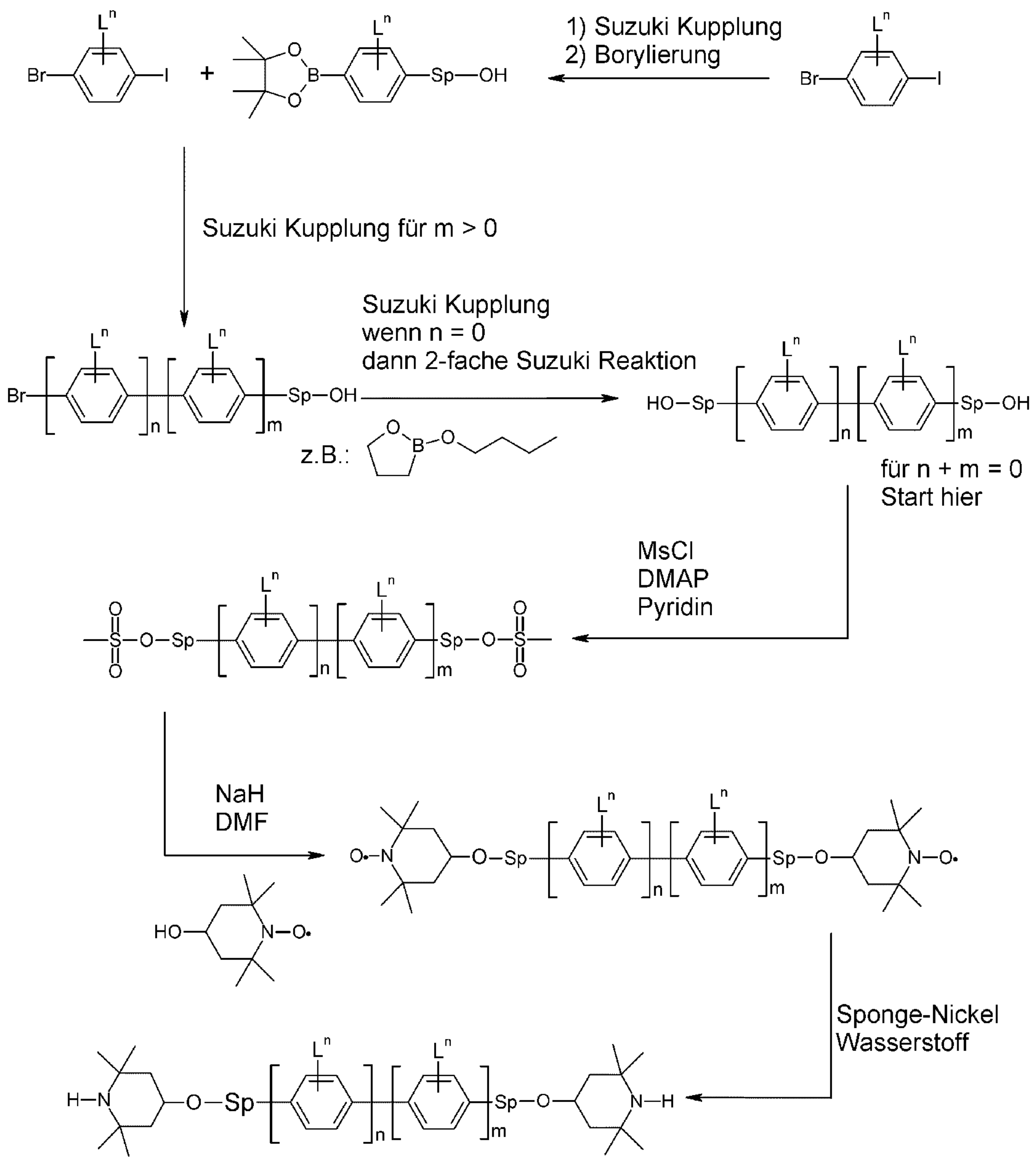


wobei n und m jeweils unabhängig voneinander 0 oder 1 sind und $L^n$ jeweils unabhängig voneinander 1, 2, 3 oder 4 optionale Substituenten L bedeutet, wobei L bei jedem Auftreten gleich oder verschieden F, Cl, CN oder geradkettiges oder verzweigtes, jeweils optional fluoriertes, Alkyl, Alkoxy, Arylalkyl oder Alkylarylalkyl mit 1 bis 15 C-Atomen bedeutet, worin auch eine oder mehrere nicht benachbarte $CH_2$-Gruppen durch -O- und/oder -S- ersetzt sein können.

Erfindungsgemäß können eine oder mehrere Verbindungen der Formel I vorteilhaft zur Stabilisierung von flüssigkristallinen Medien verwendet werden. Insbesondere können die erfindungsgemäßen Verbindungen bei dielektrisch negativen Flüssigkristallmedien die Stabilität günstig beeinflussen, insbesondere in Gegenwart einer oder mehrerer Orientierungsschichten aus z.B. Polyimid. Dies kann in den erfindungsgemäßen Anzeigen vorteilhaft zu besonders niedrigen Schaltzeiten bei gleichzeitig hohem Spannungshaltevermögen (VHR) führen.

Hierfür wird ein Verfahren zur Stabilisierung eines flüssigkristallinen Mediums bereitgestellt, wobei dem Medium eine oder mehrere Verbindungen der Formel I zugesetzt werden.

Gemäß einer bevorzugten Ausführungsform betrifft die Stabilisierung ein flüssigkristallines Medium, welches eine negative dielektrische Anisotropie aufweist. In diesem Fall werden typischerweise allenfalls nennenswerte Mengen an dielektrisch neutralen Flüssigkristallverbindungen und in der Regel nur sehr geringe Mengen an oder gar keine dielektrisch positiven Verbindungen eingesetzt, da generell die Flüssigkristallanzeigen möglichst niedrige Ansteuerspannungen haben sollen.

Vorzugsweise enthalten die erfindungsgemäßen flüssigkristallinen Medien eine oder mehrere Verbindungen ausgewählt aus der Gruppe der Verbindungen der Formeln II-1 bis II-4

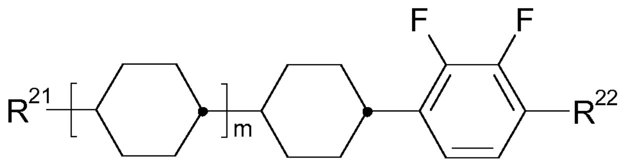

II-1

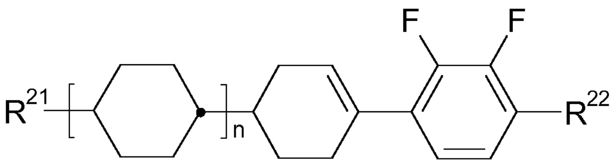

II-2

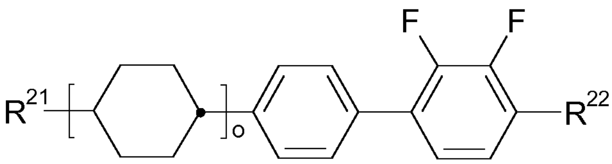

II-3

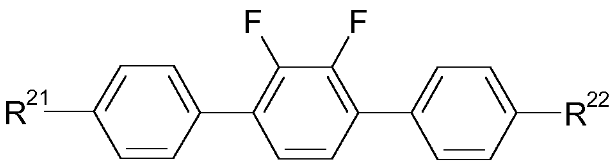

II-4

wobei

$R^{21}$ einen unsubstituierten Alkylrest mit 1 bis 7 C-Atomen,
$R^{22}$ einen unsubstituierten Alkylrest mit 1 bis 7 C-Atomen, oder einen unsubstituierten Alkoxyrest mit 1 bis 6 C-Atomen,

und

m, n und o jeweils unabhängig voneinander 0 oder 1

bedeuten,
enthält.

Überraschend wurde gefunden, dass Flüssigkristallmischungen realisiert werden können, die insbesondere in ECB-Anzeigen sowie für IPS- oder FFS-Anzeigen mit einer Aktivmatrix-Adressierung eine niedrige Schwellenspannung bei geringen Schaltzeiten aufweisen und gleichzeitig eine ausreichend breite nematische Phase, eine günstige, relativ niedrige Doppelbrechung ($\Delta n$), gute Stabilität gegen Zersetzung durch thermische und durch UV-Belastung und eine stabile hohe VHR haben, wenn man nematische Flüssigkristallmischungen verwendet, welche mindestens eine Verbindung der Formel I sowie mindestens eine Verbindung ausgewählt aus der Gruppe der Verbindungen der Formeln II-1 bis II-4, bevorzugt der Formel II-3, enthalten.

Die erfindungsgemäßen Mischungen zeigen bevorzugt sehr breite nematische Phasenbereiche mit Klärpunkten $\geq$ 70°C, sehr günstige Werte für die kapazitive Schwelle, relativ hohe Werte für VHR und gleichzeitig gute Tieftemperaturstabilitäten bei -20°C und -30°C sowie sehr geringe Rotationsviskositäten. Weiterhin zeichnen sich die erfindungsgemäßen Mischungen bevorzugt durch ein gutes Verhältnis von Klärpunkt und Rotationsviskosität und durch eine hohe negative dielektrische Anisotropie aus.

Überraschenderweise wurde ferner gefunden, dass flüssigkristalline Medien mit einem geeignet hohen $\Delta\varepsilon$, einem geeigneten Phasenbereich und einem geeigneten $\Delta n$ verwirklicht werden können, welche die Nachteile der Materialien des Standes der Technik nicht oder zumindest nur in erheblich geringerem Maße aufweisen. Insbesondere wurde hierbei überraschend gefunden, dass die Verbindungen der Formel I, auch wenn sie alleine ohne zusätzliche Temperaturstabilisatoren verwendet werden, zu einer erheblichen, in vielen Fällen ausreichenden, Stabilisierung von Flüssigkristallmischungen sowohl gegen UV-Belastung als auch gegen Temperaturbelastung führen.

Vorzugsweise enthält das erfindungsgemäße Medium eine oder mehrere Verbindungen ausgewählt aus der

Gruppe der Verbindungen der Formeln II-1 bis II-4 in einer Gesamtkonzentration im Bereich von 10 % oder mehr bis 80 % oder weniger, bevorzugt von 15 % oder mehr bis 70 % oder weniger, besonders bevorzugt von 20 % oder mehr bis 60 % oder weniger.

Insbesondere bevorzugt enthält das erfindungsgemäße Medium

eine oder mehrere Verbindungen der Formel II-1 in einer Gesamtkonzentration im Bereich von 5 % oder mehr bis 30 % oder weniger und/oder

eine oder mehrere Verbindungen der Formel II-2 in einer Gesamtkonzentration im Bereich von 3 % oder mehr bis 30 % oder weniger und/oder

eine oder mehrere Verbindungen der Formel II-3 in einer Gesamtkonzentration im Bereich von 5 % oder mehr bis 30 % oder weniger und/oder

eine oder mehrere Verbindungen der Formel II-4 in einer Gesamtkonzentration im Bereich von 1 % oder mehr bis 30 % oder weniger.

In einer bevorzugten Ausführungsform der vorliegenden Erfindung enthalten die erfindungsgemäßen Medien eine oder mehrere Verbindungen der Formel II-1, bevorzugt eine oder mehrere Verbindungen ausgewählt aus der Gruppe der Verbindungen der Formeln II-1-1 und II-1-2

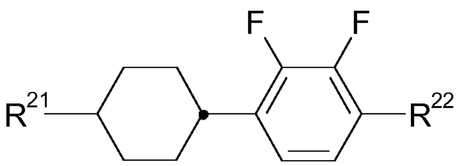

II-1-1

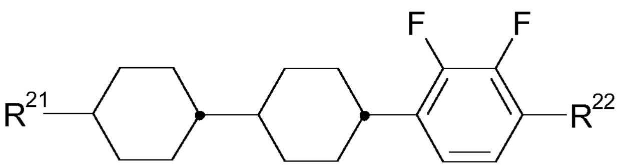

II-1-2

wobei $R^{21}$ und $R^{22}$ die oben für Formel II-1 gegebene Bedeutung haben, und bevorzugt

$R^{21}$ einen Alkylrest mit 2 bis 5 C-Atomen, besonders bevorzugt mit 3 bis 5 C-Atomen, und

$R^{22}$ einen Alkyl- oder Alkoxyrest mit 2 bis 5 C-Atomen, besonders bevorzugt einen Alkoxyrest mit 2 bis 4 C-Atomen, oder ein Alkenyloxyrest mit 2 bis 4 C-Atomen.

bedeuten.

In einer bevorzugten Ausführungsform der vorliegenden Erfindung enthalten die erfindungsgemäßen Medien eine oder mehrere Verbindungen der Formel II-2, besonders bevorzugt eine oder mehrere Verbindungen ausgewählt aus der Gruppe der Verbindungen der Formeln II-2-1 und II-2-2

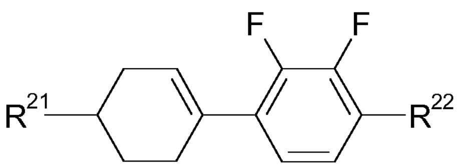

II-2-1

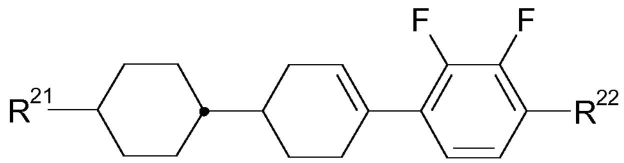

II-2-2

wobei $R^{21}$ und $R^{22}$ die oben für Formel II-2 gegebene Bedeutung haben, und bevorzugt

$R^{21}$ einen Alkylrest mit 2 bis 5 C-Atomen, besonders bevorzugt mit 3 bis 5 C-Atomen, und
$R^{22}$ einen Alkyl- oder Alkoxyrest mit 2 bis 5 C-Atomen, besonders bevorzugt einen Alkoxyrest mit 2 bis 4 C-Atomen oder ein Alkenyloxyrest mit 2 bis 4 C-Atomen,

bedeuten.

In einer bevorzugten Ausführungsform der vorliegenden Erfindung enthalten die erfindungsgemäßen Medien eine oder mehrere Verbindungen der Formel II-3, besonders bevorzugt eine oder mehrere Verbindungen ausgewählt aus der Gruppe der Verbindungen der Formeln II-3-1 und II-3-2, ganz besonders bevorzugt der Formel II-3-2,

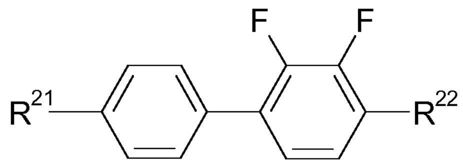

II-3-1

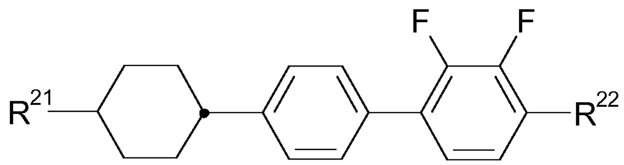

II-3-2

wobei $R^{21}$ und $R^{22}$ die oben für Formel II-3 gegebene Bedeutung haben, und bevorzugt

$R^{21}$ einen Alkylrest mit 2 bis 5 C-Atomen, besonders bevorzugt mit 3 bis 5 C-Atomen, und
$R^{22}$ einen Alkyl- oder Alkoxyrest mit 2 bis 5 C-Atomen, besonders bevorzugt ein Alkoxyrest mit 2 bis 4 C-Atomen oder ein Alkenyloxyrest mit 2 bis 4 C-Atomen,

bedeuten.

In einer weiteren bevorzugten Ausführungsform enthält das Medium eine oder mehrere Verbindungen der Formel II-4, besonders bevorzugt der Formel II-4-a,

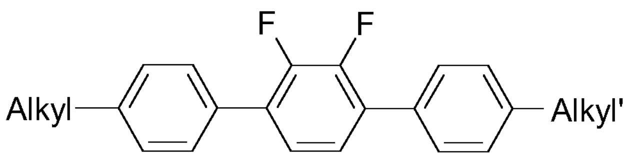

II-4-a

wobei

Alkyl und Alkyl' unabhängig voneinander Alkyl mit 1 bis 7 C-Atomen, bevorzugt mit 2 bis 5 C-Atomen,

bedeuten.

Gemäß einer besonders bevorzugten Ausführungsform können die erfindungsgemäßen Medien zusätzlich eine oder mehrere Verbindungen der Formel III-3 und/oder Formel IV,

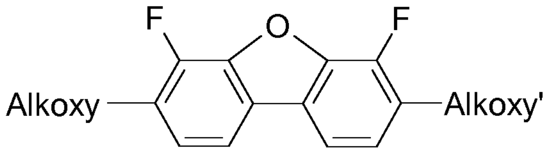

III-3

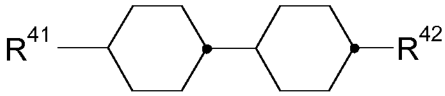

IV

wobei

Alkoxy, Alkoxy' unabhängig voneinander einen Alkoxyrest mit 1 bis 5 C-Atomen,

$R^{41}$ einen unsubstituierten Alkylrest mit 1 bis 7 C-Atomen oder einen unsubstituierten Alkenylrest mit 2 bis 7 C-Atomen,

und

$R^{42}$ einen unsubstituierten Alkylrest mit 1 bis 7 C-Atomen, einen unsubstituierten Alkoxyrest mit 1 bis 6 C-Atomen, oder einen unsubstituierten Alkenylrest mit 2 bis 7 C-Atomen,

bedeuten,
enthalten.

Besonders bevorzugt enthält das erfindungsgemäße Medium zusätzlich zu den Verbindungen ausgewählt aus der Gruppe der Verbindungen der Formeln II-1 bis II-4 eine oder mehrere Verbindungen der Formel III-3 in einer Gesamtkonzentration im Bereich von 1 % oder mehr bis 20 % oder weniger, bevorzugt von 2 % oder mehr bis 15 % oder weniger, besonders bevorzugt von 3 % oder mehr bis 10 % oder weniger.

Des Weiteren kann das erfindungsgemäße Medium eine oder mehrere Verbindungen der Formeln III-1 und III-2

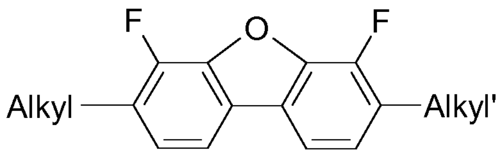

III-1

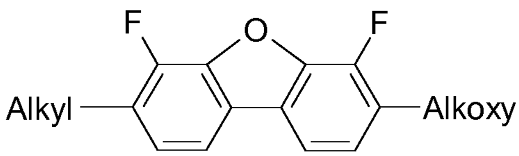

III-2

enthalten, wobei

Alkyl, Alkyl' Alkyl mit 1 bis 7 C-Atomen, bevorzugt mit 2-5 C-Atomen,

und

Alkoxy, Alkoxy' Alkoxy mit 1 bis 7 C-Atomen, bevorzugt mit 2 bis 5 C-Atomen,

bedeuten.

Vorzugsweise enthalten die Medien gemäß der vorliegenden Erfindung eine oder mehrere dielektrisch neutrale Verbindungen der Formel IV in einer Gesamtkonzentration im Bereich von 5 % oder mehr bis 90 % oder weniger, bevorzugt von 10 % oder mehr bis 80 % oder weniger, besonders bevorzugt von 20 % oder mehr bis 70 % oder weniger.

In einer besonders bevorzugten Ausführungsform enthält das erfindungsgemäße Medium eine oder mehrere Verbindungen der Formel IV, ausgewählt aus der Gruppe der Verbindungen der Formeln IV-1 bis IV-4,

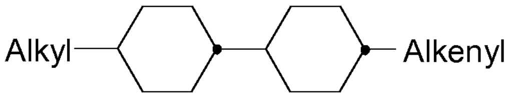

IV-1

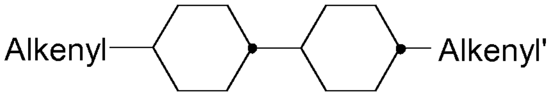

IV-2

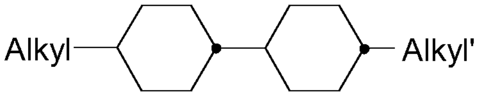

IV-3

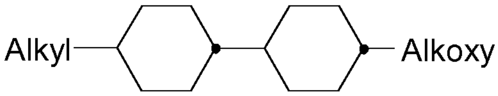

IV-4

wobei

| | |
|---|---|
| Alkyl und Alkyl’ | unabhängig voneinander Alkyl mit 1 bis 7 C-Atomen, bevorzugt mit 2 bis 5 C-Atomen, |
| Alkenyl | einen Alkenylrest mit 2 bis 5 C-Atomen, bevorzugt mit 2 bis 4 C-Atomen, besonders bevorzugt 2 C-Atomen, |
| Alkenyl’ | einen Alkenylrest mit 2 bis 5 C-Atomen, bevorzugt mit 2 bis 4 C-Atomen, besonders bevorzugt mit 2 bis 3 C-Atomen, und |
| Alkoxy | Alkoxy mit 1 bis 5 C-Atomen, bevorzugt mit 2 bis 4 C Atomen, |

bedeuten.

In einer besonders bevorzugten Ausführungsform enthalten die erfindungsgemäßen Medien eine oder mehrere Verbindungen der Formel IV-1 und/oder eine oder mehrere Verbindungen der Formel IV-2.

Des Weiteren können die erfindungsgemäßen flüssigkristallinen Medien optional eine oder mehrere Verbindungen der Formeln V

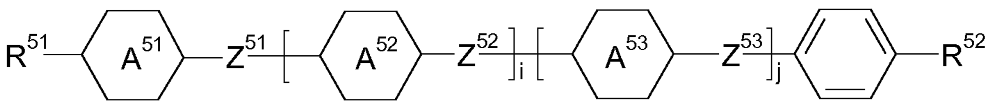

V

enthalten, wobei

| | |
|---|---|
| $R^{51}$ und $R^{52}$ | unabhängig voneinander eine der für $R^{21}$ und $R^{22}$ gegebenen Bedeutungen haben, bevorzugt Alkyl mit 1 bis 7 C-Atomen, Alkoxy mit 1 bis 7 C-Atomen und Alkoxyalkyl, Alkenyl oder Alkenyloxy mit 2 bis 7 C-Atomen, besonders *n*-Alkyl mit 1 bis 5 C-Atomen, *n*-Alkoxy mit 2 bis 5 C-Atomen und Alkenyl oder Alkenyloxy mit 2 bis 4 C-Atomen, und |

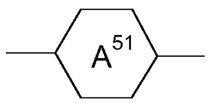

bis

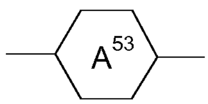

,

soweit vorhanden, jeweils unabhängig voneinander

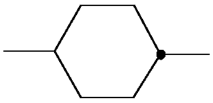 , 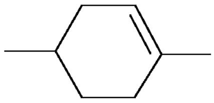 , 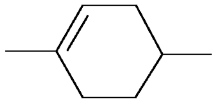 ,

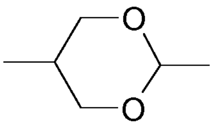 , 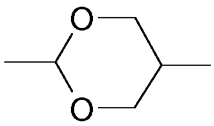 , 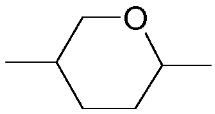 ,

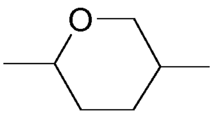 , 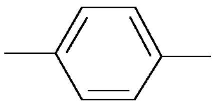 , 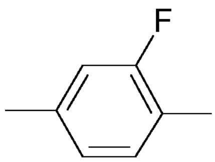 ,

oder

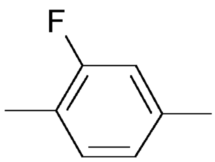 ,

bevorzugt

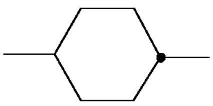 , 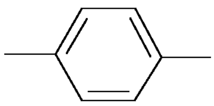 , 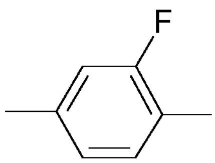 ,

oder

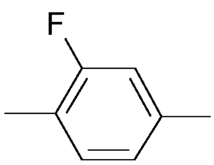 ,

bevorzugt

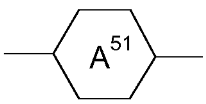 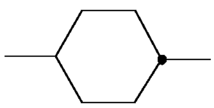 ,

und, wenn vorhanden,

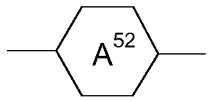

bevorzugt

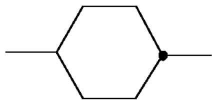

,

Z51 bis Z53 jeweils unabhängig voneinander $-CH_2-CH_2-$, $-CH_2-O-$, -CH=CH-, -C=C-, -COO- oder eine Einfachbindung, bevorzugt $-CH_2-CH_2-$, $-CH_2-O-$ oder eine Einfachbindung, bevorzugt eine Einfachbindung,

i und j jeweils unabhängig voneinander 0 oder 1

und

(i + j) bevorzugt 0 oder 1

bedeuten.

Bevorzugt enthalten die erfindungsgemäßen Medien die folgenden Verbindungen in den angegebenen Gesamtkonzentrationen:

5 - 60 Gew.-% einer oder mehrerer Verbindungen ausgewählt aus der Gruppe der Verbindungen der Formel II-1 bis II-4 und III-1 bis III-3, und/oder

10 - 60 Gew.-% einer oder mehrerer Verbindungen ausgewählt aus der Gruppe der Verbindungen der Formeln II-1 bis II-4 und/oder

10 - 60 Gew.-% einer oder mehrerer Verbindungen der Formeln IV und/oder V,

wobei der Gesamtgehalt aller Verbindungen in dem Medium 100 % beträgt.

In einer weiteren bevorzugten Ausführungsform enthält das Medium eine oder mehrere Verbindungen der Formel V ausgewählt aus der Gruppe der Verbindungen der Formeln V-1 bis V-10, bevorzugt ausgewählt aus der Gruppe der Verbindungen der Formeln V-1 bis V-5,

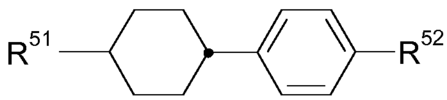

V-1

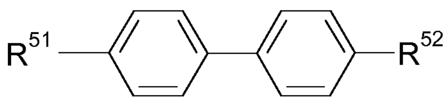

V-2

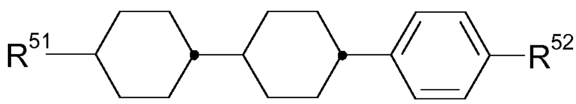

V-3

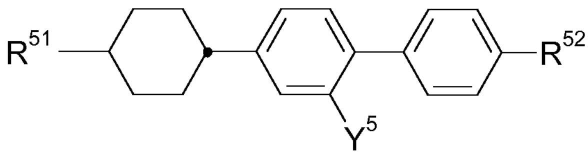

V-4

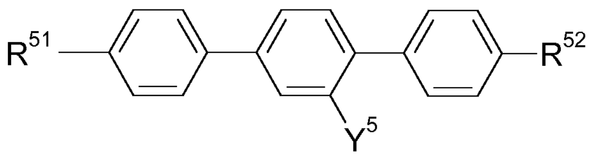

V-5

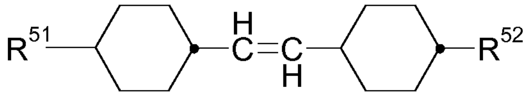

V-6

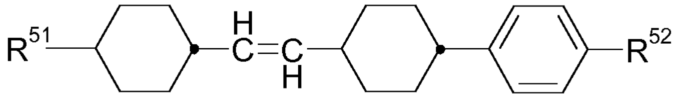

V-7

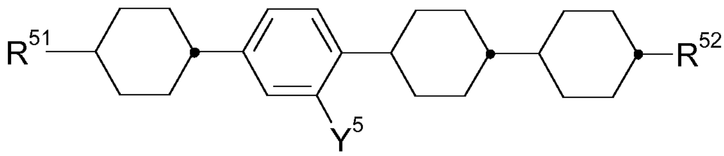

V-8

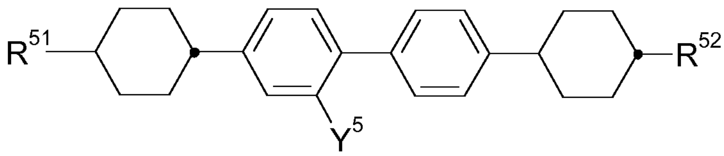

V-9

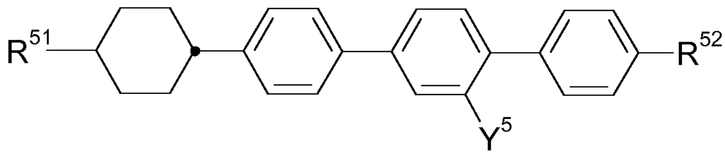

V-10

worin die Reste die oben unter Formel V gegebenen Bedeutungen haben und

$Y^5$ H oder F bedeutet,

und bevorzugt

$R^{51}$ Alkyl mit 1 bis 7 C-Atomen oder Alkenyl mit 2 bis 7 C-Atomen und

$R^{52}$ Alkyl mit 1 bis 7 C-Atomen, Alkenyl mit 2 bis 7 C-Atomen oder Alkoxy mit 1 bis 6 C-Atomen,

bedeuten.

In einer weiteren bevorzugten Ausführungsform enthält das erfindungsgemäße Medium eine oder mehrere Verbindungen der Formel V-1, ausgewählt aus der Gruppe der Verbindungen der Formeln V-1a und V-1b, bevorzugt der Formel V-1b,

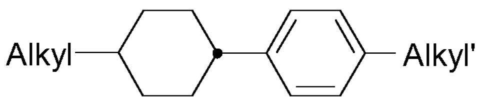

V-1a

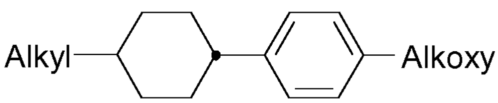

V-1b

worin

| | |
|---|---|
| Alkyl und Alkyl' | unabhängig voneinander Alkyl mit 1 bis 7 C-Atomen, bevorzugt mit 2 bis 5 C-Atomen, |
| Alkoxy | Alkoxy mit 1 bis 5 C-Atomen, bevorzugt mit 2 bis 4 C-Atomen |

bedeuten.

In einer weiteren bevorzugten Ausführungsform enthält das Medium eine oder mehrere Verbindungen der Formel V-3, ausgewählt aus der Gruppe der Verbindungen der Formeln V-3a und V-3b,

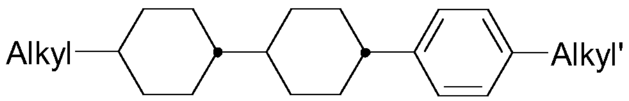

V-3a

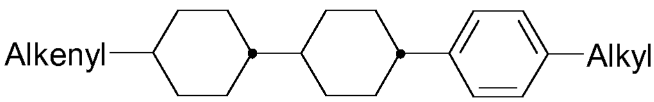

V-3b

worin

| | |
|---|---|
| Alkyl und Alkyl' | unabhängig voneinander Alkyl mit 1 bis7 C-Atomen, bevorzugt mit 2 bis 5 C-Atomen und |
| Alkenyl | Alkenyl mit 2 bis 7 C-Atomen, bevorzugt mit 2 bis 5 C-Atomen |

bedeuten.

In einer weiteren bevorzugten Ausführungsform enthält das Medium eine oder mehrere Verbindungen der Formel V-4 ausgewählt aus der Gruppe der Verbindungen der Formeln V-4a und V-4b,

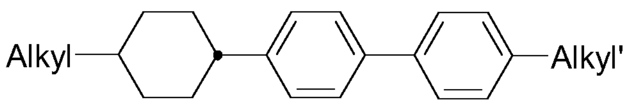

V-4a

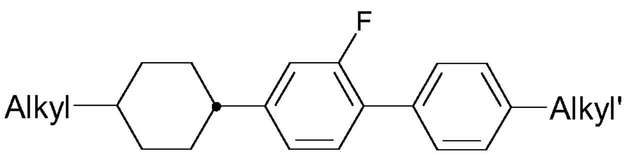

V-4b

worin

| | |
|---|---|
| Alkyl und Alkyl' | unabhängig voneinander Alkyl mit 1 bis 7 C-Atomen, bevorzugt mit 2 bis 5 C-Atomen bedeuten. |

Außerdem betrifft die vorliegende Erfindung ein Verfahren zur Stabilisierung eines flüssigkristallinen Mediums, welches eine oder mehrere Verbindungen ausgewählt aus der Gruppe der Verbindungen der Formeln II-1 bis II-4 enthält und/oder eine oder mehrere Verbindungen der Formel IV und/oder eine oder mehrere Verbindungen der Formel V, wobei dem Medium eine oder mehrere Verbindungen der Formel I zugesetzt werden.

Die Flüssigkristallmedien gemäß der vorliegenden Erfindung können eine oder mehrere chirale Verbindungen enthalten.

In einer besonders bevorzugten Ausführungsform der vorliegenden Erfindung enthalten die flüssigkristallinen Medien eine oder mehrere Verbindungen der Formel

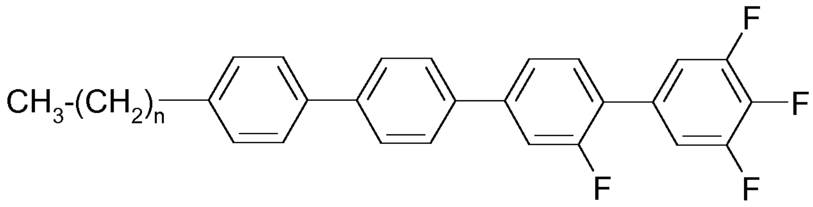

worin n 0, 1, 2, 3, 4, 5 oder 6, bevorzugt 2 oder 4, besonders bevorzugt 2, bedeutet, bevorzugt in einer Konzentration vom 0,1 bis 5 %, besonders bevorzugt von 0,2 bis 1 %.

Besonders bevorzugte Ausführungsformen der vorliegenden Erfindung erfüllen eine oder mehrere der folgenden Bedingungen, wobei die Akronyme (Abkürzungen) in den Tabellen A bis C erläutert und in Tabelle D durch Beispiele illustriert sind.

i. Das flüssigkristalline Medium hat eine Doppelbrechung von 0,06 oder mehr, besonders bevorzugt von 0,07 oder mehr.

ii. Das flüssigkristalline Medium hat eine Doppelbrechung von 0,13 oder weniger, besonders bevorzugt von 0,12 oder weniger.

iii. Das flüssigkristalline Medium hat eine Doppelbrechung im Bereich von 0,09 oder mehr bis 0,12 oder weniger.

iv. Das flüssigkristalline Medium hat eine negative dielektrische Anisotropie mit einem Betrag von 2,0 oder mehr, besonders bevorzugt von 2,5 oder mehr.

v. Das flüssigkristalline Medium hat eine negative dielektrische Anisotropie mit einem Betrag von 5,5 oder weniger, besonders bevorzugt von 5,0 oder weniger.

vi. Das flüssigkristalline Medium hat eine negative dielektrische Anisotropie mit einem Betrag im Bereich von 3,0 oder mehr bis 4,5 oder weniger.

vii. Das flüssigkristalline Medium enthält eine oder mehrere besonders bevorzugte Verbindungen der Formel IV ausgewählt aus den nachfolgend genannten Formeln:

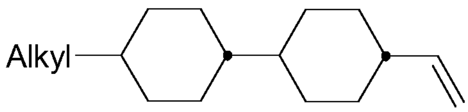

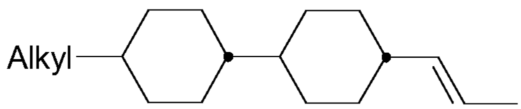

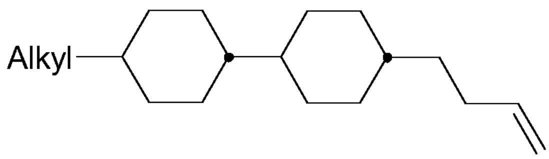

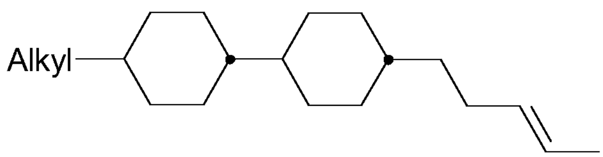

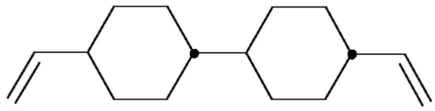

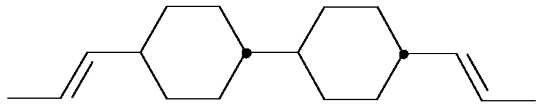

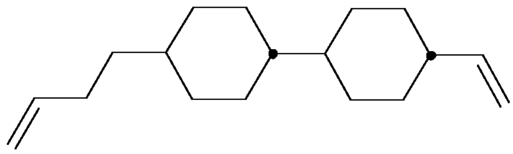

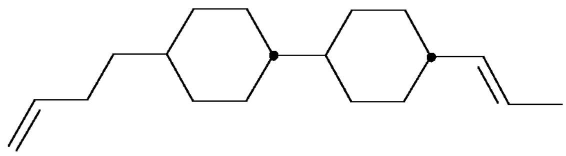

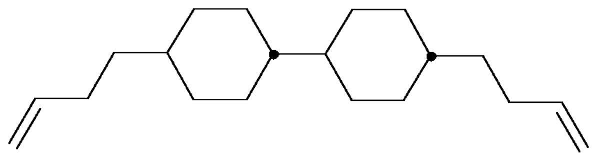

worin Alkyl die oben gegebene Bedeutung besitzt und bevorzugt, jeweils unabhängig voneinander, Alkyl mit 1 bis 6, weiter bevorzugt mit 2 bis 5 C-Atomen und besonders bevorzugt *n*-Alkyl, bedeutet.

viii. Die Gesamtkonzentration der Verbindungen der Formel IV im Gesamtgemisch beträgt 20 % oder mehr, bevorzugt 30 % oder mehr und liegt bevorzugt im Bereich von 20 % oder mehr bis 49 % oder weniger, besonders bevorzugt im Bereich von 29 % oder mehr bis 47 % oder weniger, und ganz besonders bevorzugt im Bereich von 37 % oder mehr bis 44 % oder weniger.

ix. Das flüssigkristalline Medium enthält eine oder mehrere Verbindungen der Formel IV ausgewählt aus der Gruppe der Verbindungen der folgenden Formeln: CC-n-V und/oder CC-n-Vm, insbesondere CC-3-V, bevorzugt in einer Konzentration von bis zu 50 % oder weniger, besonders bevorzugt bis zu 42 % oder weniger, und optional zusätzlich CC-3-V1, bevorzugt in einer Konzentration von bis zu 15 % oder weniger, und/oder CC-4-V, bevorzugt in einer Konzentration von bis zu 20 % oder weniger, besonders bevorzugt bis zu 10 % oder weniger

x. Die Gesamtkonzentration der Verbindungen III-1 bis III-3 im Gesamtgemisch liegt im Bereich von 1 % oder mehr bis 20 % oder weniger, bevorzugt von 2 % oder mehr bis 15 % oder weniger, besonders bevorzugt von 3 % oder mehr bis 10 % oder weniger.

xi. Die Gesamtkonzentration der Verbindungen der Formel CC-3-V im Gesamtgemisch beträgt 18 % oder mehr, bevorzugt 25 % oder mehr.

xii. Der Anteil an Verbindungen der Formeln II-1 bis II-4 und III-1 bis III-3 im Gesamtgemisch beträgt 50 % oder mehr und bevorzugt 75 % oder weniger.

xiii. Das flüssigkristalline Medium besteht im Wesentlichen aus Verbindungen der Formeln I, II-1 bis II-4, III, IV und V, bevorzugt aus Verbindungen der Formeln I, II-1 bis II-4 und IV.

xiv. Das flüssigkristalline Medium enthält eine oder mehrere Verbindungen der Formel IV, bevorzugt der Formeln IV-1 und/oder IV-2, vorzugsweise in einer Gesamtkonzentration von 20 % oder mehr, insbesondere von 25 % oder mehr, und ganz besonders bevorzugt von 30 % oder mehr bis 45 % oder weniger.

Vorzugsweise weist die Flüssigkristallmischung einen nematischen Phasenbereich mit einer Breite von mindestens 80 K und eine Fließviskosität $\nu_{20}$ von maximal 30 $mm^2 \cdot s^{-1}$ bei 20 °C auf.

Die erfindungsgemäße Flüssigkristallmischung weist vorzugsweise ein $\Delta\varepsilon$ von -0,5 bis -8,0 auf, weiter bevorzugt von -1,5 bis -6,0, und ganz besonders bevorzugt von -2,0 bis -5,0, wobei $\Delta\varepsilon$ die dielektrische Anisotropie bedeutet.

Die Rotationsviskosität $\gamma_1$ ist vorzugsweise 200 mPa·s oder weniger, insbesondere 150 mPa·s oder weniger, besonders bevorzugt 120 mPa·s oder weniger.

Die bevorzugten erfindungsgemäßen Mischungen mit negativem $\Delta\varepsilon$ sind für alle VA-TFT-Anwendungen geeignet, wie z.B. VAN, MVA, (S)-PVA und ASV. Weiterhin sind sie für IPS (In plane switching)-, FFS (Fringe field switching)- und PALC- Anwendungen mit negativem $\Delta\varepsilon$ geeignet.

Die nematischen Flüssigkristallmischungen in den erfindungsgemäßen Anzeigen enthalten in der Regel zwei Komponenten A und B, die ihrerseits aus einer oder mehreren Einzelverbindungen bestehen.

Die erfindungsgemäßen flüssigkristallinen Medien enthalten bevorzugt 4 bis 15, insbesondere 5 bis 12, und besonders bevorzugt 10 oder weniger, Verbindungen. Diese sind vorzugsweise ausgewählt aus der Gruppe der Verbindungen der Formeln I, II-1 bis II-4, und/oder IV und/oder V.

Die erfindungsgemäßen flüssigkristallinen Medien können optional auch mehr als 18 Verbindungen enthalten. In diesem Fall enthalten sie vorzugsweise 18 bis 25 Verbindungen.

Neben Verbindungen der Formeln I bis V können auch noch andere Bestandteile zugegen sein, z. B. in einer Menge von bis zu 45 %, vorzugsweise jedoch bis zu 35 %, insbesondere bis zu 10 % der Gesamtmischung.

Optional können die bevorzugten erfindungsgemäßen Medien mit negativem $\Delta\varepsilon$ auch eine dielektrisch positive Komponente enthalten, deren Gesamtkonzentration bevorzugt 10 % oder weniger bezogen auf das gesamte Medium beträgt.

In einer bevorzugten Ausführungsform enthalten die erfindungsgemäßen Flüssigkristallmedien Verbindungen ausgewählt aus der Gruppe der Verbindungen der Formeln I, II-1 bis II-4, III-3, IV und V, bevorzugt ausgewählt aus der Gruppe der Verbindungen der Formeln I und II-1 bis II-4, bevorzugt bestehen sie überwiegend, besonders bevorzugt im wesentlichen und ganz besonders bevorzugt nahezu vollständig aus den Verbindungen der genannten Formeln.

Die erfindungsgemäßen Flüssigkristallmedien weisen bevorzugt eine nematische Phase jeweils mindestens von -20°C oder weniger bis 70°C oder mehr, weiter bevorzugt von -30°C oder weniger bis 80°C oder mehr, besonders bevorzugt von -40°C oder weniger bis 85°C oder mehr und ganz besonders bevorzugt von -40°C oder weniger bis 90°C oder mehr auf.

Hierbei bedeutet der Begriff "eine nematische Phase aufweisen" einerseits, dass bei tiefen Temperaturen bei der entsprechenden Temperatur keine smektische Phase und keine Kristallisation beobachtet wird, und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen einer der elektrooptischen Anwendung entsprechenden Schichtdicke für mindestens 100 Stunden überprüft. Wenn die Lagerstabilität bei einer Temperatur von -20°C in einer entsprechenden Testzelle 1000 h oder mehr beträgt, wird das Medium als bei dieser Temperatur stabil bezeichnet. Bei Temperaturen von -30°C bzw. -40°C betragen die entsprechenden Zeiten 500 h bzw. 250 h. Bei hohen Temperaturen wird der Klärpunkt nach üblichen Methoden in Kapillaren gemessen.

In einer bevorzugten Ausführungsform sind die erfindungsgemäßen Flüssigkristallmedien durch Werte der optischen Anisotropien im mittleren bis niedrigen Bereich gekennzeichnet. Die Werte der Doppelbrechung liegen bevorzugt im Bereich von 0,065 oder mehr bis 0,13 oder weniger, besonders bevorzugt im Bereich von 0,080 oder mehr bis 0,12 oder weniger und ganz besonders bevorzugt im Bereich von 0,085 oder mehr bis 0,110 oder weniger.

In einer bevorzugten Ausführungsform haben die erfindungsgemäßen Flüssigkristallmedien eine negative dielektrische Anisotropie und weisen relativ hohe Werte des Betrags der dielektrischen Anisotropie ($|\Delta\varepsilon|$) auf, die bevorzugt im Bereich von 2,0 oder mehr bis 5,5 oder weniger, bevorzugt bis 5,0 oder weniger, bevorzugt von 2,5 oder mehr bis 4,7 oder weniger, besonders bevorzugt von 3,0 oder mehr bis 4,7 oder weniger und ganz besonders bevorzugt von 3,2 oder mehr bis 4,5 oder weniger, liegen.

Die erfindungsgemäßen Flüssigkristallmedien weisen relativ kleine Werte für die Schwellenspannung ($V_0$) im Bereich von 1,7 V oder mehr bis 2,5 V oder weniger, bevorzugt von 1,8 V oder mehr bis 2,4 V oder weniger, besonders bevorzugt von 1,9 V oder mehr bis 2,3 V oder weniger und ganz besonders bevorzugt von 1,95 V oder mehr bis 2,1 V oder weniger, auf.

In einer weiteren bevorzugten Ausführungsform weisen die erfindungsgemäßen Flüssigkristallmedien bevorzugt relativ niedrige Werte der mittleren dielektrischen Anisotropie ($\varepsilon_{av.} \equiv (\varepsilon\| + 2\varepsilon\perp)/3$) auf, die bevorzugt im Bereich von 5,0 oder mehr bis 8,0 oder weniger, bevorzugt von 5,4 oder mehr bis 7,5 oder weniger, noch mehr bevorzugt von 5,5 oder mehr bis 7,3 oder weniger, besonders bevorzugt von 5,6 oder mehr bis 7,1 oder weniger und ganz besonders bevorzugt von 5,7 oder mehr bis 6,8 oder weniger, liegen.

Außerdem weisen die erfindungsgemäßen Flüssigkristallmedien hohe Werte für die VHR in Flüssigkristallzellen auf.

Diese sind in frisch gefüllten Zellen bei 20°C in den Zellen vorzugsweise größer oder gleich 95 %, bevorzugt größer oder gleich 97 %, besonders bevorzugt größer oder gleich 98 % und ganz besonders bevorzugt größer oder gleich 99 %, und nach 5 Minuten im Ofen bei 100°C in den Zellen vorzugsweise größer oder gleich 80 %, bevorzugt größer oder gleich 85 %, besonders bevorzugt größer oder gleich 90 % und ganz besonders bevorzugt größer oder gleich 95 %.

In der Regel weisen dabei Flüssigkristallmedien mit einer geringen Ansteuerspannung bzw. Schwellenspannung eine geringere VHR auf als solche mit einer größeren Ansteuerspannung bzw. Schwellenspannung und umgekehrt.

Diese bevorzugten Werte für die einzelnen physikalischen Eigenschaften werden von den erfindungsgemäßen Medien bevorzugt auch jeweils miteinander kombiniert eingehalten.

Vorliegendend bedeutet der Begriff "Verbindungen", auch geschrieben als "Verbindung(en)", sofern nicht explizit anders angegeben, sowohl eine als auch mehrere Verbindungen.

Die einzelnen Verbindungen werden, sofern nichts anderes angegeben, in den Mischungen in Konzentrationen generell jeweils von 1 % oder mehr bis 30 % oder weniger, bevorzugt von 2 % oder mehr bis 30 % oder weniger und besonders bevorzugt von 3 % oder mehr bis 16 % oder weniger eingesetzt.

In einer bevorzugten Ausführungsform enthalten die erfindungsgemäßen flüssigkristallinen Medien
die Verbindung der Formel I,
eine oder mehrere Verbindungen der Formel IV, bevorzugt ausgewählt aus der Gruppe der Verbindungen der Formeln CC-n-V und CC-n-Vm, bevorzugt CC-3-V, CC-3-V1, CC-4-V und CC-5-V, besonders bevorzugt ausgewählt aus der Gruppe der Verbindungen CC-3-V, CC-3-V1 und CC-4-V, ganz besonders bevorzugt der Verbindung CC-3-V und gegebenenfalls zusätzlich der Verbindung CC-4-V und/oder CC-3-V1,
eine oder mehrere Verbindungen der Formel II-1-1, bevorzugt der Formel CY-n-Om, besonders bevorzugt ausgewählt aus der Gruppe der Verbindungen der Formeln CY-3-O2, CY-3-O4, CY-5-O2 und CY-5-O4,
eine oder mehrere Verbindungen der Formel II-1-2, bevorzugt ausgewählt aus der Gruppe der Verbindungen der Formel CCY-n-m und CCY-n-Om, bevorzugt der Formel CCY-n-Om, bevorzugt ausgewählt aus der Gruppe der Verbindungen der Formeln CCY-3-O2, CCY-2-O2, CCY-3-O1, CCY-3-O3, CCY-4-O2, CCY-3-O2 und CCY-5-O2,
optional eine oder mehrere Verbindungen der Formel II-2-2, bevorzugt der Formel CLY-n-Om, besonders bevorzugt ausgewählt aus der Gruppe der Verbindungen der Formeln CLY-2-O4, CLY-3-O2 und CLY-3-O3,
eine oder mehrere Verbindungen der Formel II-3-2, bevorzugt der Formel CPY-n-Om, besonders bevorzugt ausgewählt aus der Gruppe der Verbindungen der Formeln CPY-2-O2 und CPY-3-O2, CPY-4-O2 und CPY-5-O2,
eine oder mehrere Verbindungen der Formel II-4, bevorzugt der Formel PYP-n-m, besonders bevorzugt ausgewählt aus der Gruppe der Verbindungen der Formeln PYP-2-3 und PYP-2-4, und
eine oder mehrere der Verbindungen der Formel III-3, bevorzugt die Verbindung der Formel B-2O-O5.

Vorzugsweise beträgt die Gesamtkonzentration der einen oder mehreren Verbindungen der Formel I im erfindungsgemäßen Medium, bezogen auf das Gesamtmedium, 5000 ppm oder weniger, weiter bevorzugt 2000 ppm oder weniger, noch weiter bevorzugt 1000 ppm oder weniger, besonders bevorzugt 500 ppm oder weniger und ganz besonders bevorzugt 250 ppm oder weniger.

Gemäß einer Ausführungsform liegt die Konzentration der einen oder mehreren Verbindungen der Formel I im erfindungsgemäßen Medium, bezogen auf das Gesamtmedium, im Bereich von 1 ppm bis 5000 ppm, bevorzugt im Bereich von 1 ppm bis 2000 ppm, weiter bevorzugt im Bereich von 1 ppm bis 1000 ppm, besonders bevorzugt im Bereich von 1 ppm bis 500 ppm und ganz besonders bevorzugt im Bereich von 1 ppm bis 250 ppm. In einer besonders bevorzugten Ausführungsform liegt die Konzentration der einen oder mehreren Verbindungen der Formel I im erfindungsgemäßen Medium, bezogen auf das Gesamtmedium, im Bereich von 5 ppm bis 2000 ppm, und insbesondere im Bereich von 10 ppm bis 500 ppm.

Gemäß einer Ausführungsform können den erfindungsgemäßen Flüssigkristallmischungen zusätzlich zu der Verbindung der Formel I oder den Verbindungen der Formel I eine oder mehrere weitere Stabilisatoren, bevorzugt phenolische Stabilisatoren, zugegeben werden, um eine ausreichende Stabilisierung gegen UV- und Temperaturbelastung, insbesondere gegen thermische Belastung, weiter günstig zu beeinflussen oder gegebenenfalls auch sicherzustellen.

In einer besonders bevorzugten Ausführungsform enthalten die erfindungsgemäßen Medien eine oder mehrere Verbindungen ausgewählt aus der Gruppe der Verbindungen der Formeln OH-1 bis OH-6

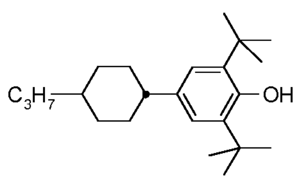

OH-1

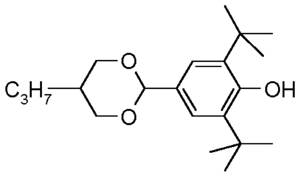

OH-2

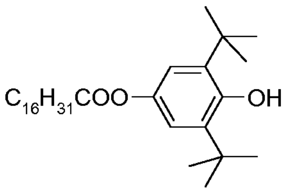

OH-3

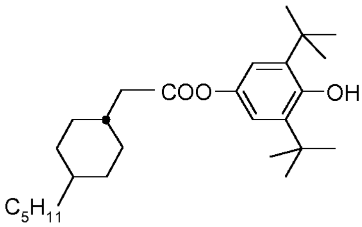

OH-4

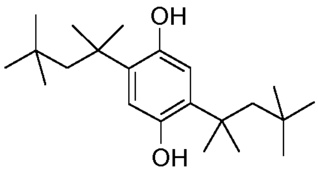

OH-5

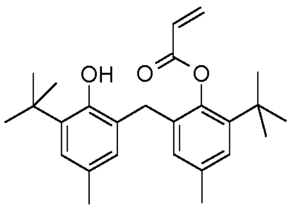

OH-6

Diese Verbindungen eignen sich hervorragend zur Stabilisierung der Medien gegen thermische Belastungen.

In einer anderen bevorzugten Ausführungsform der vorliegenden Erfindung können die erfindungsgemäßen Medien auch ausreichende Stabilität aufweisen, wenn sie keine Phenolverbindung, insbesondere ausgewählt aus der Gruppe der Verbindungen der Formeln OH-1 bis OH-6, enthalten.

Vorliegend wird ferner ein Verfahren zur Herstellung eines flüssigkristallinen Mediums bereitgestellt, worin eine oder mehrere Verbindungen der Formel I mit einer oder mehreren Verbindungen der Formeln II-1 bis II-4 und gegebenenfalls einer oder mehreren Verbindungen der Formel III-3 und/oder gegebenenfalls mit einer oder mehreren Verbindungen der Formel IV gemischt werden.

In einer bevorzugten Ausführungsform enthalten die erfindungsgemäßen Flüssigkristallmedien zusätzlich eine oder mehrere polymerisierbare Verbindungen, insbesondere einen oder mehrere Polymervorläufer, die eine oder mehrere reaktive Verbindungen, bevorzugt reaktive Mesogene und bei Bedarf auch weitere Zusatzstoffe wie z. B. Polymerisationsinitiatoren und/oder Polymerisationsmoderatoren in den üblichen Mengen enthalten. Die eingesetzte Menge dieser Zusatzstoffe beträgt insgesamt 0 % oder mehr bis 10 % oder weniger bezogen auf die Menge der gesamten Mischung, bevorzugt 0,1 % oder mehr bis 2 % oder weniger.

Die Konzentration dieser polymerisierbaren Verbindungen und Polymervorläufer wird bei der Angabe der Konzentrationen sowie der Konzentrationsbereiche der Flüssigkristallverbindungen in den Flüssigkristallmedien nicht berücksichtigt.

Die erfindungsgemäßen Flüssigkristallmedien können bei Bedarf auch weitere Zusatzstoffe wie z.B. zusätzliche Stabilisatoren und/oder pleochroitische Farbstoffe und/oder chirale Dotierstoffe in den üblichen Mengen enthalten. Die

eingesetzte Menge dieser Zusatzstoffe beträgt bevorzugt insgesamt 0 % oder mehr bis 10 % oder weniger bezogen auf die Menge der gesamten Mischung, besonders bevorzugt 0,1 % oder mehr bis 6 % oder weniger. Die Konzentration der einzelnen eingesetzten Verbindungen beträgt bevorzugt 0,1 % oder mehr bis 3 % oder weniger. Die Konzentration dieser und ähnlicher Zusatzstoffe wird bei der Angabe der Konzentrationen sowie der Konzentrationsbereiche der Flüssigkristallverbindungen in den Flüssigkristallmedien in der Regel nicht berücksichtigt.

Ein weiterer Aspekt der vorliegenden Erfindung ist die Verwendung des erfindungsgemäßen Mediums in einer elektrooptischen Anzeige oder in einer elektrooptischen Komponente.

Gegenstand der vorliegenden Erfindung sind auch elektrooptische Anzeigen oder elektrooptische Komponenten, die erfindungsgemäße flüssigkristalline Medien enthalten.

Bevorzugt sind elektrooptische Anzeigen, die mittels einer Aktivmatrix-Adressierungsvorrichtung angesteuert werden, und insbesondere solche, die auf dem VA- oder dem ECB-Effekt oder dem IPS- oder FFS-Effekt basieren. Besonders bevorzugt sind elektrooptische Anzeigen, insbesondere mit Aktivmatrixadressierung, die auf dem IPS- oder FFS-Effekt basieren.

Gemäß der vorliegenden Erfindung umfassen die Elemente alle ihre jeweiligen Isotope. Insbesondere können in den Verbindungen ein oder mehrere H durch D ersetzt sein, und dieses ist in einigen Ausführungsformen auch besonders bevorzugt. Ein entsprechend hoher Deuterierungsgrad der entsprechenden Verbindungen ermöglicht z.B. eine Detektion und Wiedererkennung der Verbindungen. Dieses ist insbesondere bei den Verbindungen der Formel I in einigen Fällen sehr hilfreich.

Gemäß der vorliegenden Erfindung bedeuten

Alkyl besonders bevorzugt geradkettiges Alkyl, insbesondere $CH_3$-, $C_2H_5$-, $n$-$C_3H_7$, $n$-$C_4H_9$- oder $n$-$C_5H_{11}$-,

Alkenyl besonders bevorzugt $CH_2$=CH-, $E$-$CH_3$-CH=CH-, $CH_2$=CH-$CH_2$-$CH_2$-, $E$-$CH_3$-CH=CH-$CH_2$-$CH_2$- oder $E$-($n$-$C_3H_7$)-CH=CH-, und

Alkoxy besonders bevorzugt geradkettiges Alkoxy, insbesondere $CH_3O$-, $C_2H_5O$-, $n$-$C_3H_7O$-, $n$-$C_4H_9O$- oder $n$-$C_5H_{11}O$-.

Für die vorliegende Erfindung bedeutet im Zusammenhang mit der Angabe der Bestandteile der Zusammensetzungen, wenn nicht im Einzelfall anders angegeben:

- "enthalten": die Konzentration der betreffenden Bestandteile in der Zusammensetzung beträgt bevorzugt 5 % oder mehr, besonders bevorzugt 10 % oder mehr und ganz besonders bevorzugt 20 % oder mehr,

- "überwiegend bestehen aus": die Konzentration der betreffenden Bestandteile in der Zusammensetzung beträgt bevorzugt 50 % oder mehr, besonders bevorzugt 55 % oder mehr und ganz besonders bevorzugt 60 % oder mehr,

- "im Wesentlichen bestehen aus": die Konzentration der betreffenden Bestandteile in der Zusammensetzung beträgt bevorzugt 80 % oder mehr, besonders bevorzugt 90 % oder mehr und ganz besonders bevorzugt 95 % oder mehr und

- "nahezu vollständig bestehen aus": die Konzentration der betreffenden Bestandteile in der Zusammensetzung beträgt bevorzugt 98 % oder mehr, besonders bevorzugt 99 % oder mehr und ganz besonders bevorzugt 100,0 %.

Dies gilt sowohl für die Medien als Zusammensetzungen mit ihren Bestandteilen, die Komponenten und Verbindungen sein können, als auch für die Komponenten mit ihren Bestandteilen, den Verbindungen. Lediglich in Bezug auf die Konzentration einer einzelnen Verbindung im Verhältnis zum gesamten Medium bedeutet der Begriff enthalten: die Konzentration der betreffenden Verbindung beträgt bevorzugt 1 % oder mehr, besonders bevorzugt 2 % oder mehr, ganz besonders bevorzugt 4 % oder mehr.

Für die vorliegende Erfindung bedeutet "≤" kleiner oder gleich, bevorzugt kleiner, und "≥" größer oder gleich, bevorzugt größer.

Für die vorliegende Erfindung bedeuten

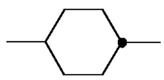 , 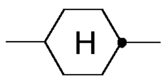 und 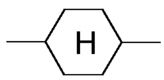

trans-1,4-Cyclohexylen und

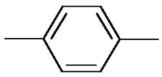

und

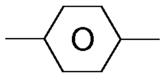

1,4-Phenylen.

Für die vorliegende Erfindung bedeuten die Begriffe "dielektrisch positive Verbindungen" solche Verbindungen mit einem $\Delta\varepsilon > 1,5$, "dielektrisch neutrale Verbindungen" solche mit $-1,5 \leq \Delta\varepsilon \leq 1,5$ und "dielektrisch negative" Verbindungen solche mit $\Delta\varepsilon < -1,5$. Hierbei wird die dielektrische Anisotropie der Verbindungen bestimmt, indem 10 % der Verbindungen in einem flüssigkristallinen Host gelöst werden und von der resultierenden Mischung die Kapazität in mindestens jeweils einer Testzelle mit 20 $\mu$m Schichtdicke mit homöotroper und mit homogener Oberflächenorientierung bei 1 kHz bestimmt wird. Die Meßspannung beträgt typischerweise 0,5 V bis 1,0 V, sie ist jedoch stets niedriger als die kapazitive Schwelle der jeweiligen untersuchten Flüssigkristallmischung.

Als Hostmischung für dielektrisch positive und dielektrisch neutrale Verbindungen wird ZLI-4792 und für dielektrisch negative Verbindungen ZLI-2857, beide von Merck KGaA, Deutschland, verwendet. Aus der Änderung der Dielektrizitätskonstante der Hostmischung nach Zugabe der zu untersuchenden Verbindung und Extrapolation auf 100 % der eingesetzten Verbindung werden die Werte für die jeweiligen zu untersuchenden Verbindungen erhalten. Die zu untersuchende Verbindung wird zu 10 % in der Hostmischung gelöst. Wenn die Löslichkeit der Substanz hierzu zu gering ist, wird die Konzentration schrittweise solange halbiert, bis die Untersuchung bei der gewünschten Temperatur erfolgen kann.

Die Zusammensetzungen bestehen aus mehreren Verbindungen, bevorzugt aus 3 oder mehr bis 30 oder weniger, besonders bevorzugt aus 6 oder mehr bis 20 oder weniger und ganz besonders bevorzugt aus 10 oder mehr bis 16 oder weniger Verbindungen, die auf herkömmliche Weise gemischt werden. In der Regel wird die gewünschte Menge der in geringerer Menge verwendeten Komponenten in den Komponenten gelöst, die den Hauptbestandteil der Mischung ausmachen. Dies erfolgt zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus einem sogenannten "Multi Bottle System" herzustellen.

Die erfindungsgemäßen Mischungen zeigen sehr breite nematische Phasenbereiche, bevorzugt mit Klärpunkten von 65°C oder mehr, sehr günstige Werte für die kapazitive Schwelle, relativ hohe Werte für die Holding Ratio und gleichzeitig sehr gute Tieftemperaturstabilitäten bei -30°C und -40°C. Weiterhin zeichnen sich die erfindungsgemäßen Mischungen durch niedrige Rotationsviskositäten $\gamma_1$ aus.

Es versteht sich für den Fachmann von selbst, dass die erfindungsgemäßen Medien für die Verwendung in VA-, IPS-, FFS- oder PALC-Anzeigen auch Verbindungen enthalten können, worin beispielsweise H, N, O, Cl, F durch die entsprechenden Isotope ersetzt sind.

Der Aufbau der erfindungsgemäßen Flüssigkristallanzeigen entspricht den üblichen Geometrien, wie z. B. in EP 0 240 379 A1 beschrieben.

Mittels geeigneter Zusatzstoffe können die erfindungsgemäßen Flüssigkristallphasen derart modifiziert werden, dass sie in jeder bisher bekannt gewordenen Art von z. B. ECB-, VAN-, IPS-, FFS-, GH- oder ASM-VA-LCD-Anzeige einsetzbar sind.

In der nachfolgenden Tabelle E werden mögliche Dotierstoffe angegeben, die den erfindungsgemäßen Mischungen zugesetzt werden können. Sofern die Mischungen einen oder mehrere Dotierstoffe enthalten, wird er in Mengen von 0,01 % bis 4 %, vorzugsweise 0,1 % bis 1,0 %, eingesetzt.

Zusätzliche Stabilisatoren, die beispielsweise den erfindungsgemäßen Mischungen zugesetzt werden können, vorzugsweise in Mengen von 0,01 % bis 6 %, insbesondere 0,1 % bis 3 %, werden nachfolgend in Tabelle F genannt.

Alle Konzentrationen sind für die Zwecke der vorliegenden Erfindung, soweit nicht explizit anders vermerkt, in Massenprozent angegeben und beziehen sich auf die entsprechende Mischung oder Mischungskomponente, soweit nicht explizit anders angegeben.

Gemäß der vorliegenden Erfindung sind alle angegebenen Werte für Temperaturen, wie z. B. der Schmelzpunkt T(C,N), der Übergang von der smektischen (S) zur nematischen (N) Phase T(S,N) und der Klärpunkt T(N,I), in Grad Celsius (°C) und alle Temperaturdifferenzen entsprechend als Differenzgrad (° oder Grad) angegeben, sofern nicht explizit anders angegeben.

Der Begriff "Schwellenspannung" bezieht sich für die vorliegende Erfindung auf die kapazitive Schwelle ($V_0$), auch Freedericksz-Schwelle genannt, sofern nicht explizit anders angegeben.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C, und $\Delta n$ wird bei 589 nm und $\Delta\varepsilon$ bei 1 kHz bestimmt, sofern nicht jeweils explizit anders angegeben.

Die elektrooptischen Eigenschaften, z. B. die Schwellenspannung ($V_0$) (kapazitive Messung) werden, ebenso

wie das Schaltverhalten, in bei der Firma Merck Japan hergestellten Testzellen bestimmt. Dabei haben die Meßzellen Substrate aus Natriumglas (Sodalime Glas) und sind in einer ECB- bzw. VA-Konfiguration mit Polyimidorientierungsschichten (SE-1211 mit Verdünner **26 (Mischungsverhältnis 1:1), beide der Firma Nissan Chemicals, Japan), die senkrecht zueinander gerieben sind und die eine homöotrope Orientierung der Flüssigkristalle bewirken, ausgeführt. Die Fläche der durchsichtigen, nahezu quadratischen Elektroden aus ITO beträgt 1 $cm^2$.

Die verwendeten Flüssigkristallmischungen sind, wenn nicht anders angegeben, nicht mit einem chiralen Dotierstoff versetzt, sie eignen sich aber auch besonders für Anwendungen, in denen eine solche Dotierung erforderlich ist.

Die VHR wird in bei der Firma Merck Japan hergestellten Testzellen bestimmt. Die Testzellen haben Substrate aus Natriumglas und sind mit Polyimidorientierungsschichten mit einer Schichtdicke von 50 nm versehen. Der Zellspalt beträgt einheitlich 6,5 $\mu$m. Die Fläche der durchsichtigen Elektroden aus ITO beträgt 1 $cm^2$.

Die VHR wird, soweit nicht anders angegeben, bei 20°C ($VHR_{20}$) und nach 5 Minuten im Ofen bei 100°C ($VHR_{100}$) in einem kommerziell erhältlichen Gerät der Firma Autronic Melchers, Deutschland bestimmt. Die verwendete Spannung hat eine Frequenz in einem Bereich von 1 Hz bis 60 Hz, falls nicht genauer angegeben.

Die Genauigkeit der Meßwerte der VHR hängt vom jeweiligen Wert der VHR ab. Dabei nimmt die Genauigkeit mit geringer werdenden Werten ab. Die bei Werten in den verschiedenen Größenbereichen in der Regel beobachteten Abweichungen sind in ihrer Größenordnung in der folgenden Tabelle zusammengestellt.

| VHR-Bereich | | Abweichung (relativ) |
|---|---|---|
| VHR-Werte | | $\Delta_G$VHR/VHR /% |
| von | bis | ca. |
| 99,6 % | 100 % | +/- 0,1 |
| 99,0 % | 99,6 % | +/-0,2 |
| 98 % | 99 % | +/- 0,3 |
| 95 % | 98 % | +/- 0,5 |
| 90 % | 95 % | +/-1 |
| 80 % | 90 % | +/- 2 |
| 60 % | 80 % | +/- 4 |
| 40 % | 60 % | +/- 8 |
| 20 % | 40 % | +/- 10 |
| 10 % | 20 % | +/- 20 |

Die Stabilität gegen Bestrahlung mit UV wird in einem kommerziellen "Suntest CPS" Gerät der Firma Heraeus, Deutschland, untersucht. Dabei werden die versiegelten Testzellen zwischen 30 min und 2,0 Stunden, falls nicht explizit angegeben, ohne zusätzliche thermische Belastung bestrahlt. Die Bestrahlungsleistung im Wellenlängenbereich von 300 nm bis 800 nm beträgt 765 $W/m^2$, falls nicht anders angegeben. Es wird ein UV "cut-off" Filter mit einer Kantenwellenlänge von 310 nm verwendet, um den sogenannten Fensterglasmodus (Englisch: "window glass mode") zu simulieren. Bei jeder Versuchsserie werden für jede Bedingung mindestens vier Testzellen untersucht und die jeweiligen Ergebnisse werden als Mittelwerte der entsprechenden einzelnen Messungen angegeben.

Die üblicherweise durch die Belastung, z.B. durch Bestrahlung mit UV durch eine LCD-Hintergrundbeleuchtung, verursachte Abnahme der Voltage Holding Ratio ($\Delta$VHR) wird nach der folgenden Gleichung (1) bestimmt:

$$\Delta VHR(t) = VHR(t) - VHR(t=0) \qquad (1).$$

Die Rotationsviskosität wird mit der Methode des rotierenden Permanentmagneten und die Fließviskosität in einem modifizierten Ubbelohde-Viskosimeter bestimmt. Für die Flüssigkristallmischungen ZLI-2293, ZLI-4792 und MLC-6608, alle Produkte der Firma Merck KGaA, Darmstadt, Deutschland, betragen die bei 20°C bestimmten Werte der Rotationsviskosität 161 mPa·s, 133 mPa·s bzw. 186 mPa·s und die der Fließviskosität (v) 21 $mm^2 \cdot s^{-1}$, 14 $mm^2 \cdot s^{-1}$ bzw. 27 $mm^2 \cdot s^{-1}$.

Es werden, wenn nicht explizit anders angegeben, die folgenden Symbole verwendet:

| | |
|---|---|
| $V_o$ | Schwellenspannung, kapazitiv [V] bei 20°C, |
| $n_e$ | außerordentlicher Brechungsindex gemessen bei 20°C und 589 nm, |
| $n_o$ | ordentlicher Brechungsindex gemessen bei 20°C und 589 nm, |
| $\Delta n$ | optische Anisotropie gemessen bei 20°C und 589 nm, |
| $\varepsilon_\perp$ | dielektrische Suszeptibilität senkrecht zum Direktor bei 20°C und 1 kHz, |
| $\varepsilon_\parallel$ | dielektrische Suszeptibilität parallel zum Direktor bei 20°C und 1 kHz, |
| $\Delta\varepsilon$ | dielektrische Anisotropie bei 20°C und 1 kHz, |
| cp. bzw. T(N,I) | Klärpunkt [°C], |
| $\nu$ | Fließviskosität gemessen bei 20°C [$mm^2 \cdot s^{-1}$], |
| $\gamma_1$ | Rotationsviskosität gemessen bei 20°C [mPa·s], |
| $K_1$ | elastische Konstante, "splay"-Deformation bei 20°C [pN], |
| $K_2$ | elastische Konstante, "twist"-Deformation bei 20°C [pN], |
| $K_3$ | elastische Konstante, "bend"-Deformation bei 20°C [pN], |
| LTS | Tieftemperaturstabilität der Phase ("low temperature stability"), bestimmt in Testzellen, |
| VHR | Spannungshaltevermögen ("voltage holding ratio"), |
| $\Delta$VHR | Abnahme der Voltage Holding Ratio und |
| $S_{rel}$ | relative Stabilität der VHR. |

Die folgenden Beispiele erläutern die vorliegende Erfindung ohne sie zu begrenzen. Sie zeigen dem Fachmann jedoch bevorzugte Mischungskonzepte mit bevorzugt einsetzbaren Verbindungen und deren jeweiligen Konzentrationen sowie deren Kombinationen miteinander. Außerdem illustrieren die Beispiele, welche Eigenschaften und Eigenschaftskombinationen zugänglich sind.

Für die vorliegende Erfindung und in den folgenden Beispielen sind die Strukturen der Flüssigkristallverbindungen durch Akronyme angegeben, wobei die Transformation in chemische Formeln gemäß folgender Tabellen A bis C erfolgt. Alle Reste $C_nH_{2n+1}$, $C_mH_{2m+1}$ und $C_lH_{2l+1}$ bzw. $C_nH_{2n}$, $C_mH_{2m}$ und $C_lH_{2l}$ sind geradkettige Alkylreste bzw. Alkylenreste jeweils mit n, m bzw. l C-Atomen, wobei n, m und l unabhängig voneinander jeweils eine ganze Zahl sind, bevorzugt 1 bis 6. In Tabelle A sind die Ringelemente der Kerne der Verbindung codiert, in Tabelle B sind die Brückenglieder aufgelistet und in Tabelle C sind die Bedeutungen der Symbole für die linken bzw. rechten Endgruppen der Moleküle aufgelistet. Die Akronyme werden aus den Codes für die Ringelemente mit optionalen Verknüpfungsgruppen, gefolgt von einem ersten Bindestrich und den Codes für die linke Endgruppe, sowie einem zweiten Bindestrich und den Codes für die rechte Endgruppe, zusammengesetzt. In Tabelle D sind Beispielstrukturen von Verbindungen mit ihren jeweiligen Abkürzungen zusammengestellt.

**Tabelle A: Ringelemente**

| | | | |
|---|---|---|---|
| **C** | 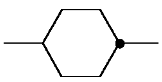 | | |
| **D** | 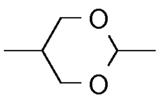 | **DI** | 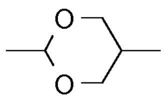 |
| **A** | 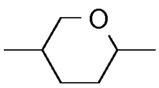 | **AI** | 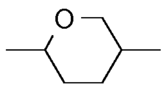 |
| **P** | 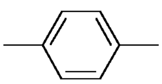 | | |
| **G** | 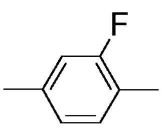 | **GI** | 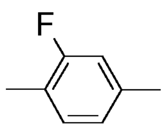 |
| **U** | 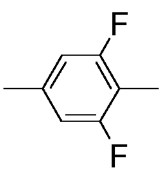 | **UI** | 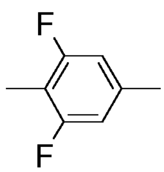 |

(fortgesetzt)

| | | | |
|---|---|---|---|
| **Y** | 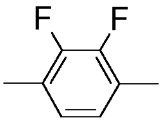 | | |
| **P(F, Cl)Y** | 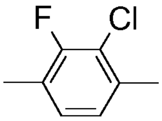 | **P(Cl,F)Y** | 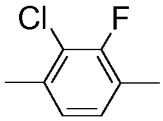 |
| **np** | 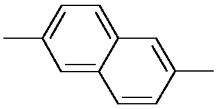 | | |
| **n3f** | 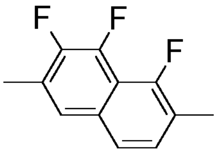 | **nN3fl** | 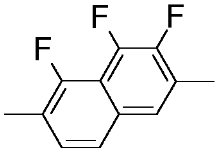 |
| **th** | 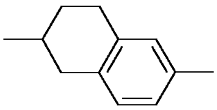 | **thl** | 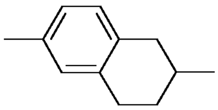 |
| **tH2f** | 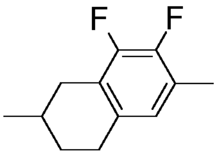 | **tH2fl** | 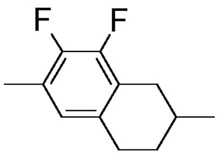 |
| **o2f** | 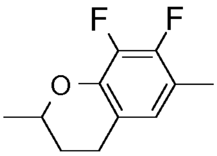 | **o2fl** | 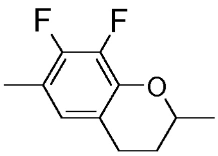 |
| **dh** | 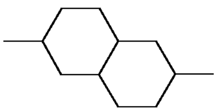 | **B** | 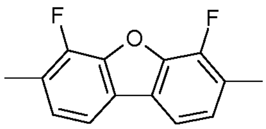 |
| | | **B(S)** | 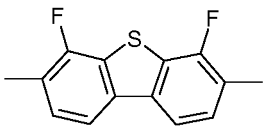 |
| **K** | 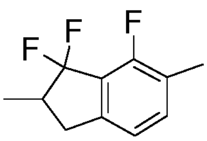 | **Kl** | 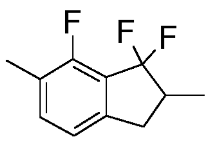 |
| **L** | 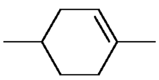 | **Ll** | 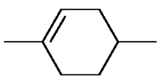 |
| **F** | 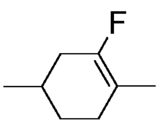 | **Fl** | 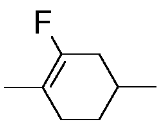 |

**Tabelle B: Brückenglieder**

| | | | |
|---|---|---|---|
| **E** | $-CH_2-CH_2-$ | | |
| **V** | -CH=CH- | | |
| **T** | -C≡C- | | |
| **W** | $-CF_2-CF_2-$ | | |
| **B** | -CF=CF- | | |
| **Z** | -CO-O- | **ZI** | -O-CO- |
| **X** | -CF=CH- | **XI** | -CH=CF- |
| **O** | $-CH_2-O-$ | **OI** | $-O-CH_2-$ |
| **Q** | $-CF_2-O-$ | **QI** | $-O-CF_2-$ |

**Tabelle C: Endgruppen**

| **Links einzelstehend oder in Kombination** | | **Rechts einzelstehend oder in Kombination** | |
|---|---|---|---|
| **-n-** | $C_nH_{2n+1}-$ | **-n** | $-C_nH_{2n+1}$ |
| **-nO-** | $C_nH_{2n+1}-O-$ | **-nO** | $-O- C_nH_{2n+1}$ |
| **-V-** | $CH_2=CH-$ | **-V** | $-CH=CH_2$ |
| **-nV-** | $C_nH_{2n+1}-CH=CH-$ | **-nV** | $-C_nH_{2n}-CH=CH_2$ |
| **-Vn-** | $CH_2=CH- C_nH_{2n}-$ | **-Vn** | $-CH=CH-C_nH_{2n+1}$ |
| **-nVm-** | $C_nH_{2n+1}-CH=CH-C_mH_{2m}-$ | **-nVm** | $-C_nH_{2n}-CH=CH-C_mH_{2m+1}$ |
| **-N-** | N≡C- | **-N** | -C≡N |
| **-S-** | S=C=N- | **-S** | -N=C=S |
| **-F-** | F- | **-F** | -F |
| **-Cl-** | Cl- | **-Cl** | -Cl |
| **-M-** | $CFH_2-$ | **-M** | $-CFH_2$ |
| **-D-** | $CF_2H-$ | **-D** | $-CF_2H$ |
| **-T-** | $CF_3-$ | **-T** | $-CF_3$ |
| **-MO-** | $CFH_2O$ - | **-OM** | $-OCFH_2$ |
| **-DO-** | $CF_2HO$ - | **-OD** | $-OCF_2H$ |
| **-TO-** | $CF_3O$ - | **-OT** | $-OCF_3$ |
| **-A-** | H-C≡C- | **-A** | -C≡C-H |
| **-nA-** | $C_nH_{2n+1}$-C≡C- | **-An** | -C≡C-$C_nH_{2n+1}$ |
| **-NA-** | N≡C-C≡C- | **-AN** | -C≡C-C≡N |

| **Links nur in Kombination** | | **Rechts nur in Kombination** | |
|---|---|---|---|
| **-...n...-** | $-C_nH_{2n}-$ | **-...n...** | $-C_nH_{2n}-$ |
| **-...M...-** | -CFH- | **-...M...** | -CFH- |
| **-...D...-** | $-CF_2-$ | **-...D...** | $-CF_2-$ |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

worin n und m jeweils ganze Zahlen und die drei Punkte "..." Platzhalter für andere Abkürzungen aus dieser Tabelle sind.

Vorzugsweise enthalten die erfindungsgemäßen Mischungen neben den Verbindungen der Formel I eine oder mehrere Verbindungen der nachfolgend genannten Verbindungen.

Hierbei sind n, m und l unabhängig voneinander jeweils eine ganze Zahl, bevorzugt 1 bis 6.

**Tabelle D**

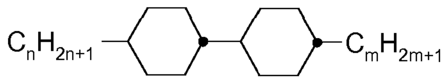

**CC-n-m**

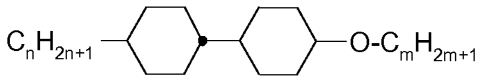

**CC-n-Om**

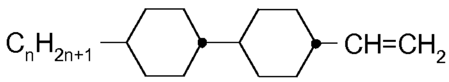

**CC-n-V**

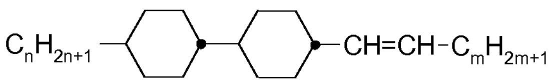

**CC-n-Vm**

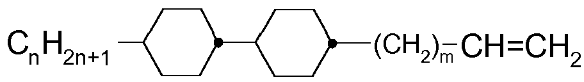

**CC-n-mV**

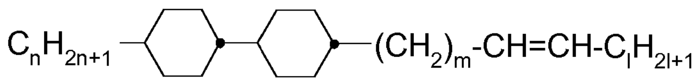

**CC-n-mVl**

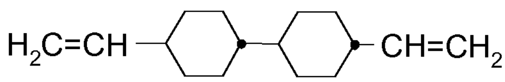

**CC-V-V**

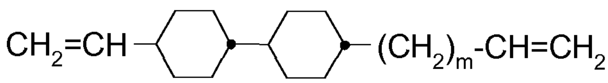

**CC-V-mV**

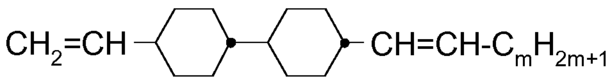

**CC-V-Vm**

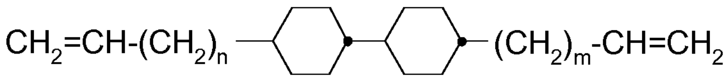

**CC-Vn-mV**

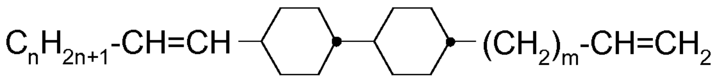

**CC-nV-mV**

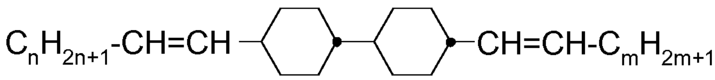

**CC-nV-Vm**

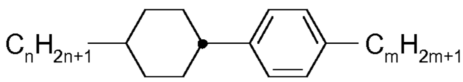

**CP-n-m**

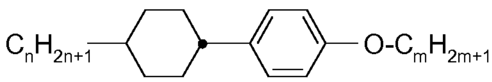

**CP-n-Om**

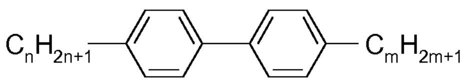

**PP-n-m**

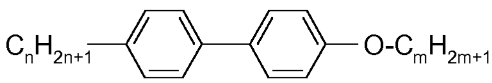

**PP-n-Om**

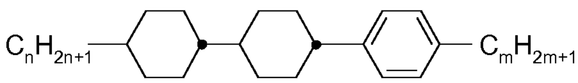

**CCP-n-m**

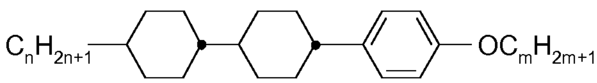

**CCP-n-Om**

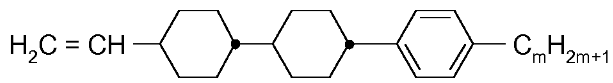

**CCP-V-m**

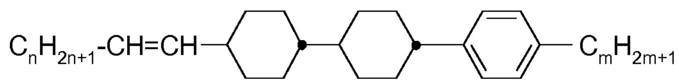

**CCP-nV-m**

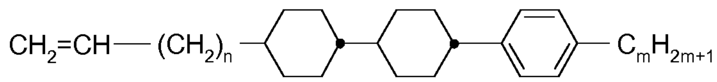

**CCP-Vn-m**

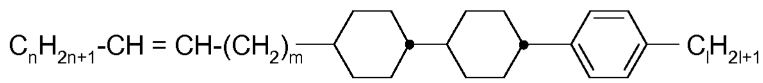

**CCP-nVm-l**

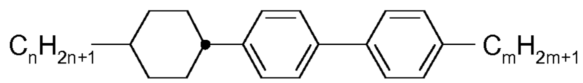

**CPP-n-m**

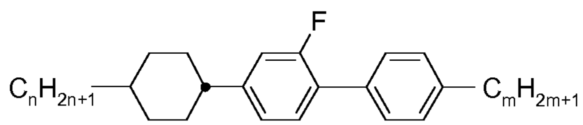

**CGP-n-m**

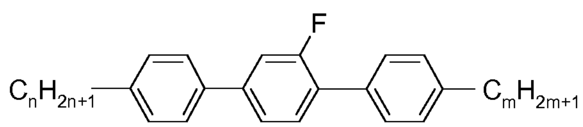

**PGP-n-m**

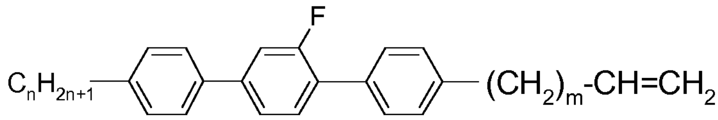

**PGP-n-mV**

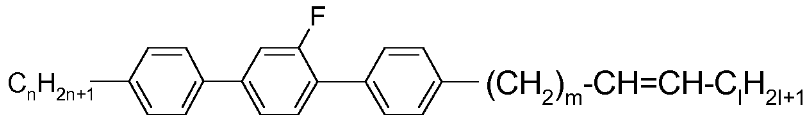

**PGP-n-mVI**

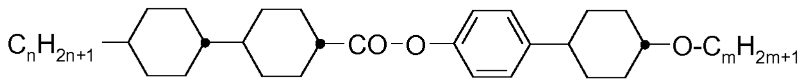

**CCZPC-n-m**

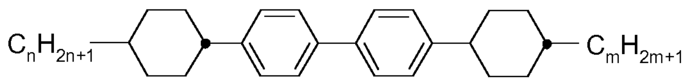

**CPPC-n-m**

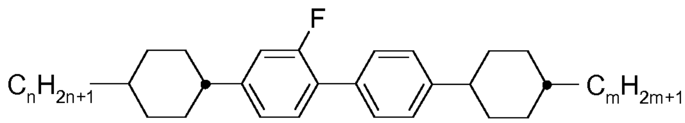

**CGPC-n-m**

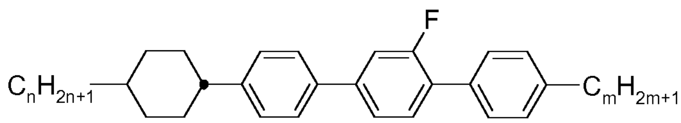

**CPGP-n-m**

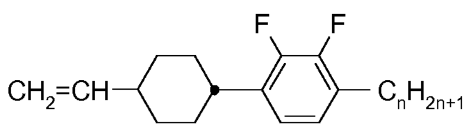

**CY-V-n**

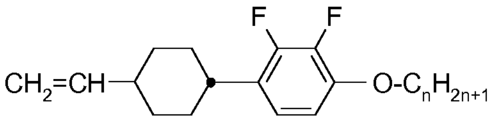

**CY-V-On**

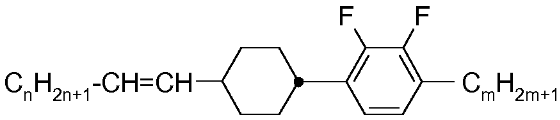

**CY-nV-m**

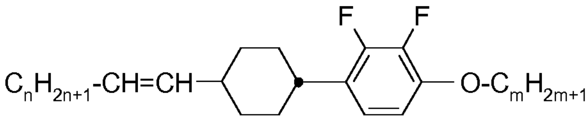

**CY-nV-Om**

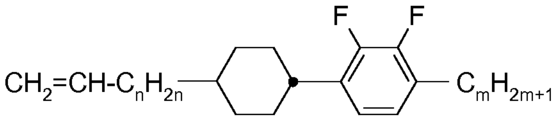

**CY-Vn-m**

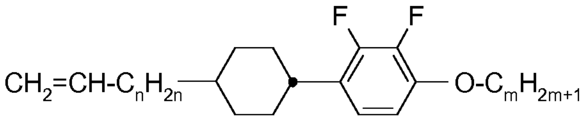

**CY-Vn-Om**

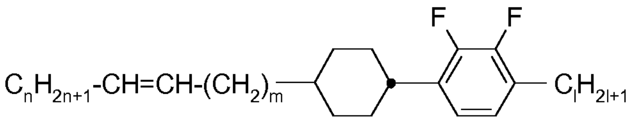

**CY-nVm-l**

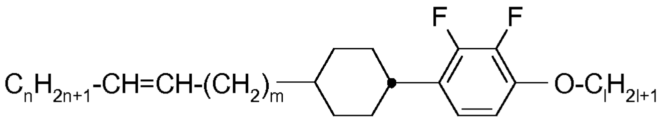

**CY-nVm-Ol**

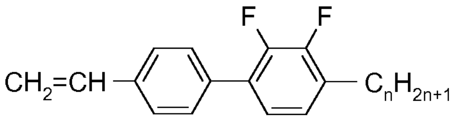

**PY-V-n**

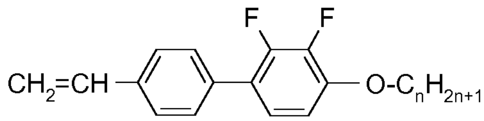

**PY-V-On**

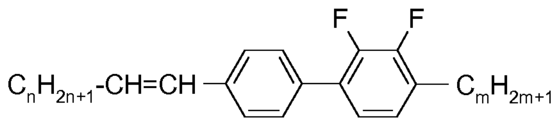

**PY-nV-m**

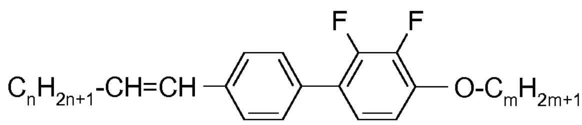

**PY-nV-Om**

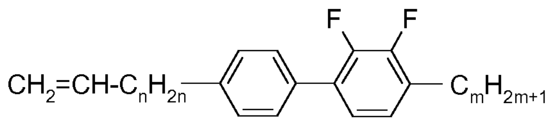

**PY-Vn-m**

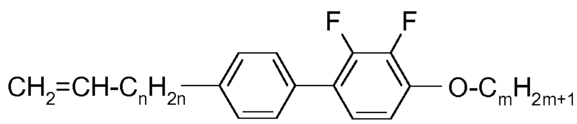

**PY-Vn-Om**

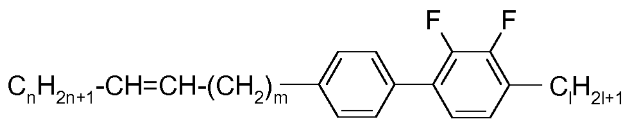

**PY-nVm-l**

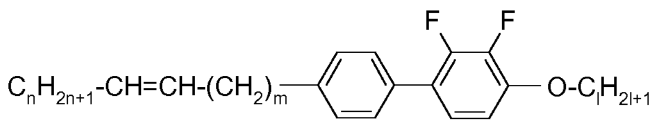

**PY-nVm-Ol**

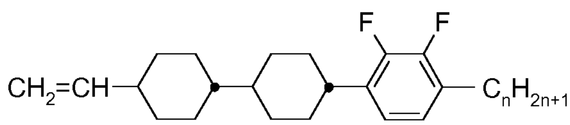

**CCY-V-n**

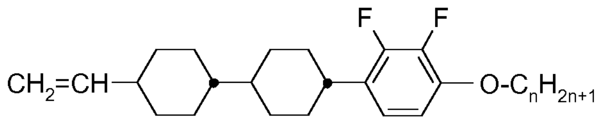

**CCY-V-On**

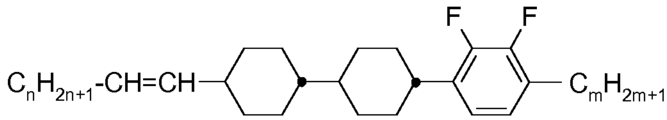

**CCY-nV-m**

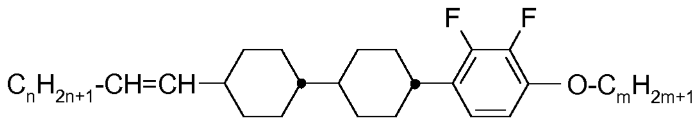

**CCY-nV-Om**

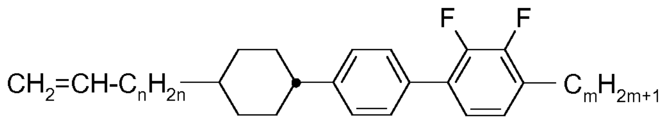

**CPY-Vn-m**

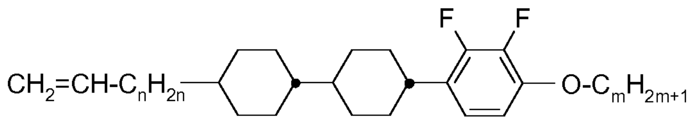

**CCY-Vn-Om**

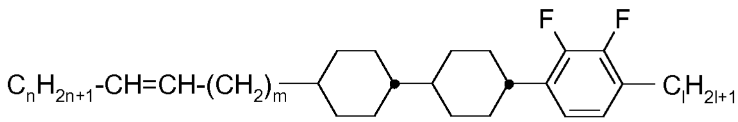

**CCY-nVm-l**

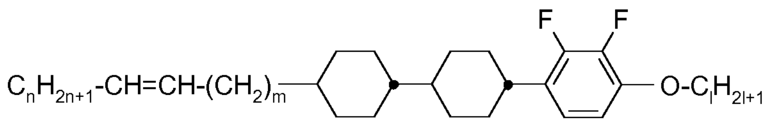

**CCY-nVm-Ol**

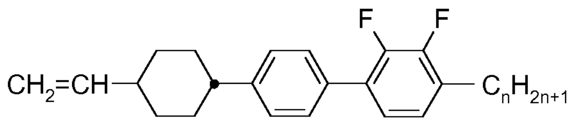

**CPY-V-n**

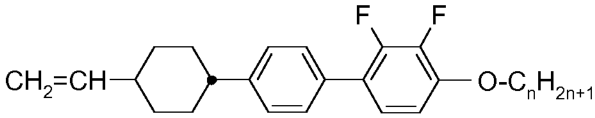

**CPY-V-On**

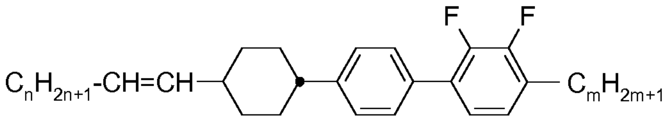

**CPY-nV-m**

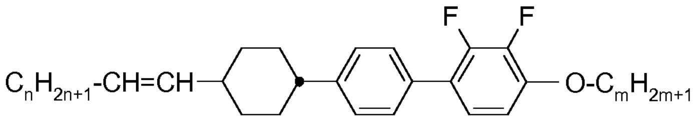

**CPY-nV-Om**

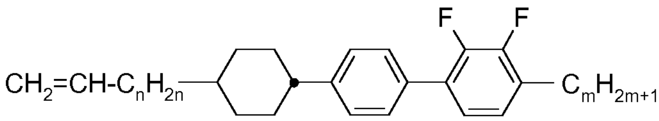

**CPY-Vn-m**

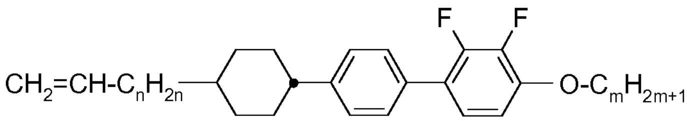

**CPY-Vn-Om**

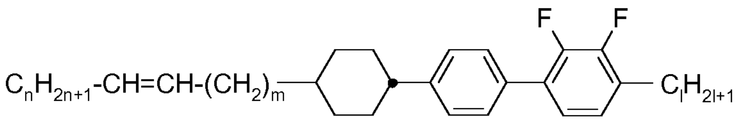

**CPY-nVm-l**

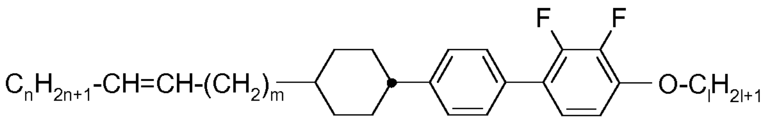

**CPY-nVm-Ol**

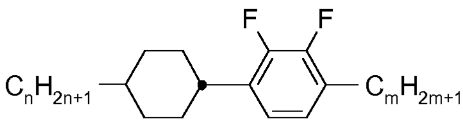

**CY-n-m**

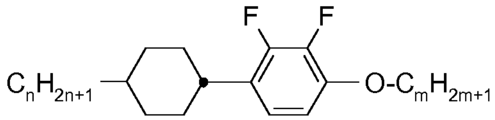

**CY-n-Om**

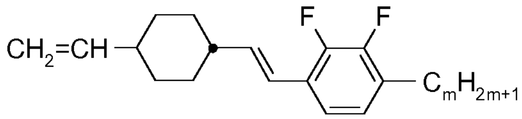

**CVY-n-m**

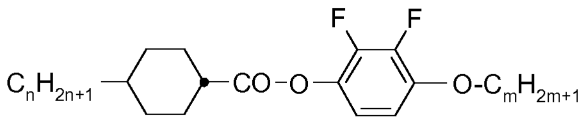

**CZY-n-Om**

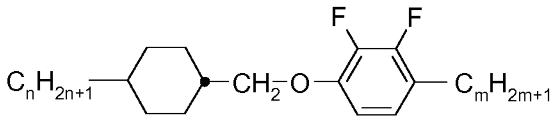

**COY-n-m**

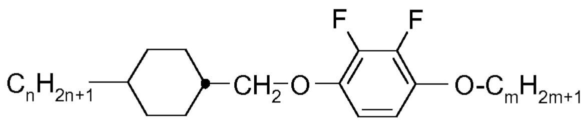

**COY-n-Om**

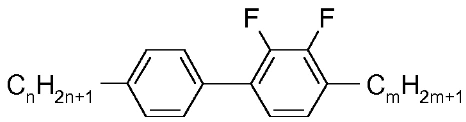

**PY-n-m**

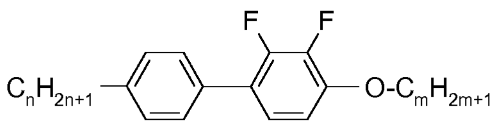

**PY-n-Om**

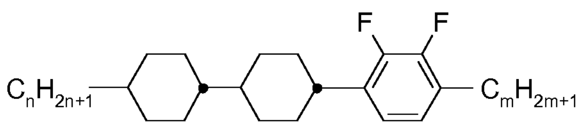

**CCY-n-m**

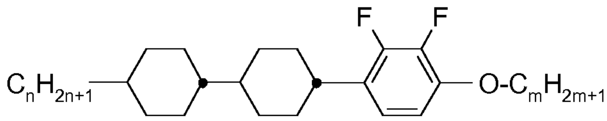

**CCY-n-Om**

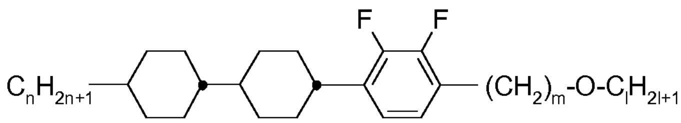

**CCY-n-mOl**

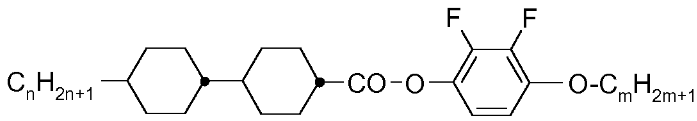

**CCZY-n-Om**

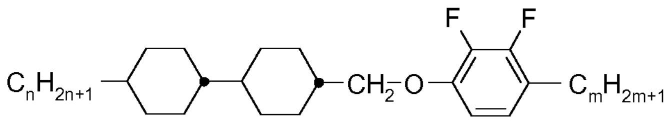

**CCOY-n-m**

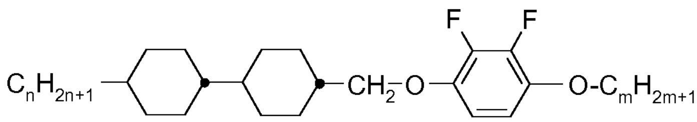

**CCOY-n-Om**

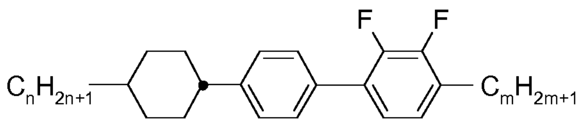

**CPY-n-m**

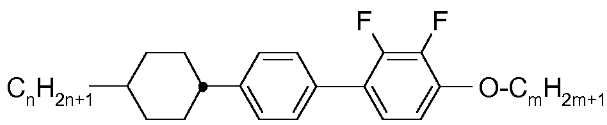

**CPY-n-Om**

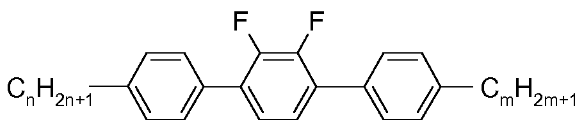

**PYP-n-m**

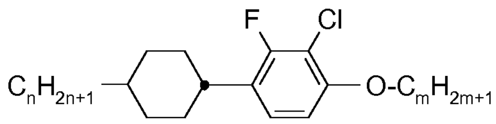

**CP(F,Cl)-n-Om**

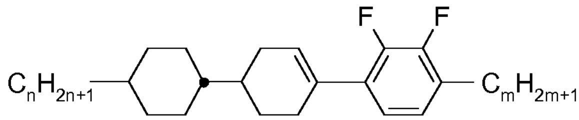

**CLY-n-m**

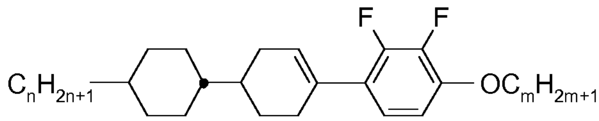

**CLY-n-Om**

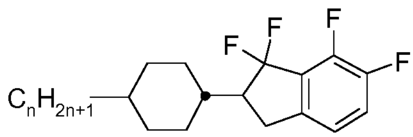

**CK-n-F**

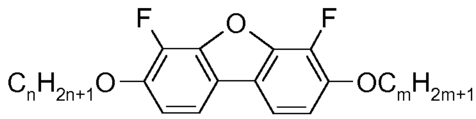

**B-nO-Om**

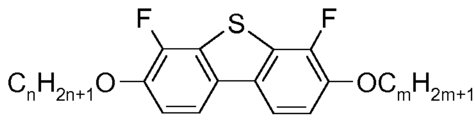

**B(S)-nO-Om**

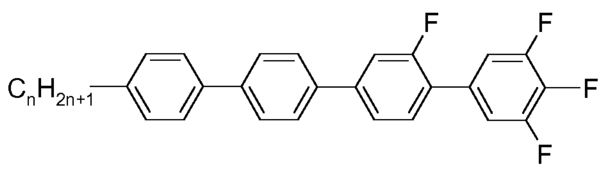

**PPGU-n-F**

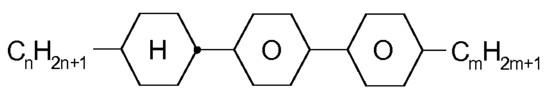

**BCH-nm**

In der Tabelle E werden chirale Dotierstoffe genannt, die bevorzugt in den erfindungsgemäßen Mischungen eingesetzt werden.

**Tabelle E**

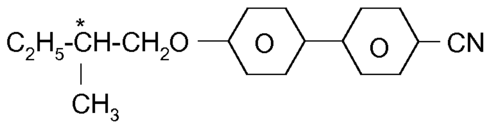

**C 15**

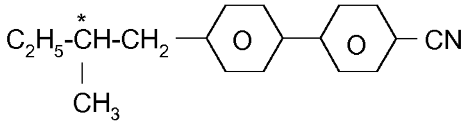

**CB 15**

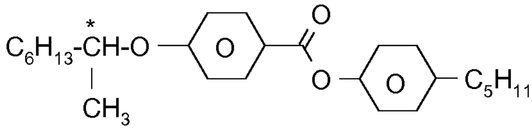

**CM 21**

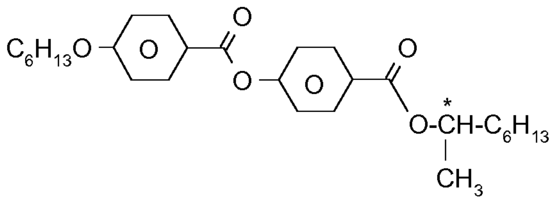

**R S-811 / S-811**

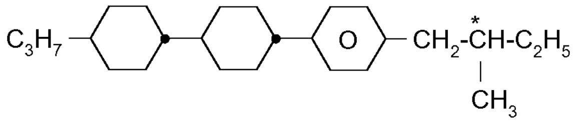

**CM 44**

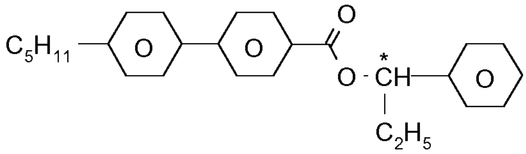

**CM 45**

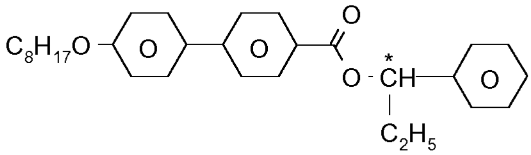

**CM 47**

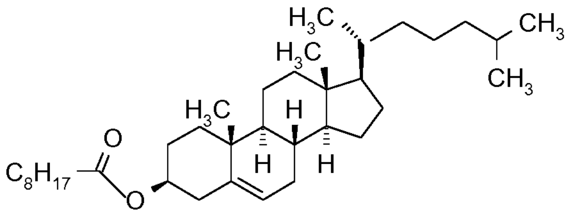

**CN**

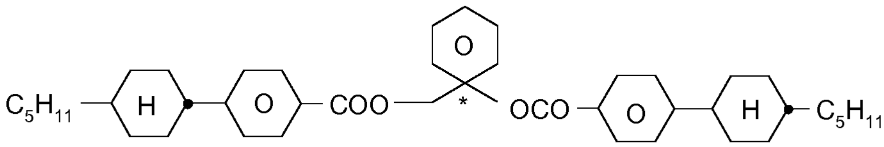

**R-1011 / S-1011**

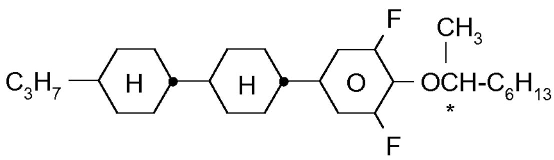

**R-2011 / S-2011**

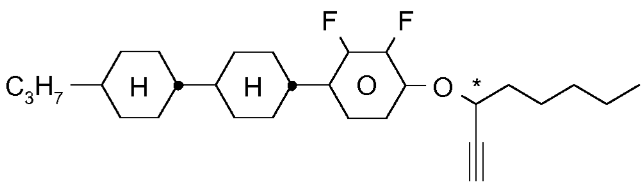

**R-3011 / S-3011**

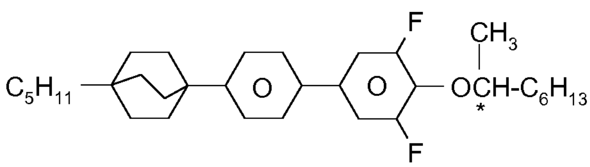

**R-4011 / S-4011**

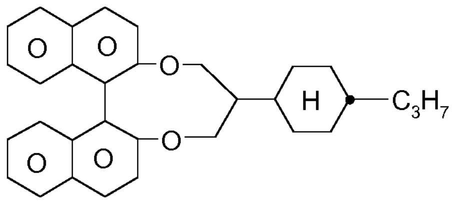

**R-5011 / S-5011**

In einer bevorzugten Ausführungsform der vorliegenden Erfindung enthalten die erfindungsgemäßen Medien eine oder mehrere Verbindungen ausgewählt aus der Gruppe der Verbindungen der Tabelle E.

In der nachfolgenden Tabelle F werden Stabilisatoren genannt, die zusätzlich zu den Verbindungen der Formel I in den erfindungsgemäßen Mischungen eingesetzt werden können. Hierbei bedeutet n eine ganze Zahl im Bereich von 1 bis 12. Insbesondere die gezeigten Phenolderivate sind als zusätzliche Stabilisatoren besonders vorteilhaft einsetzbar, insbesondere als Antioxidantien.

**Tabelle F**

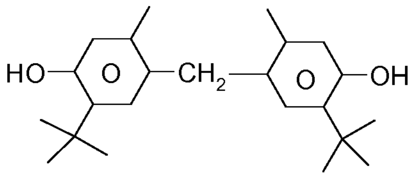

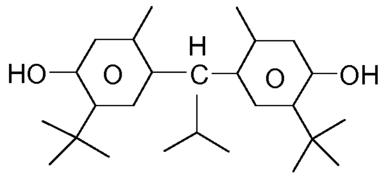

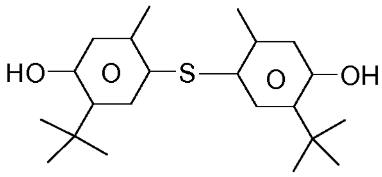

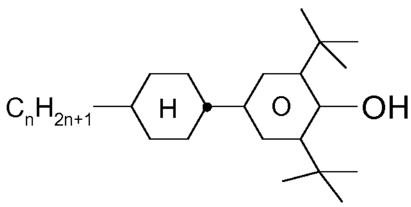

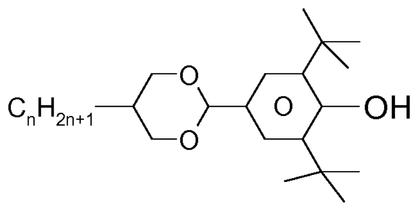

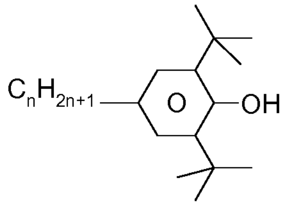

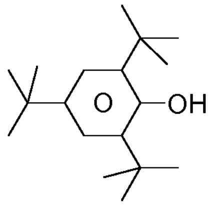

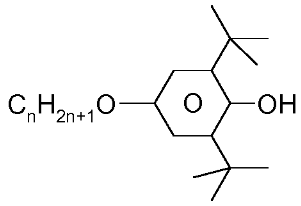

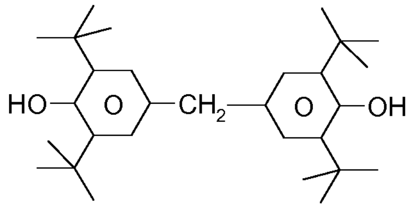

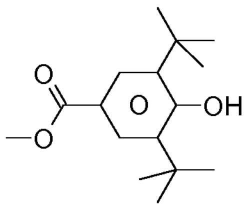

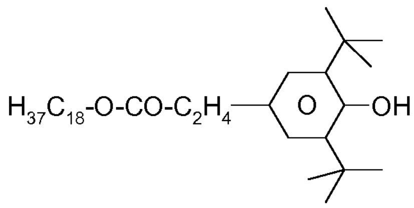

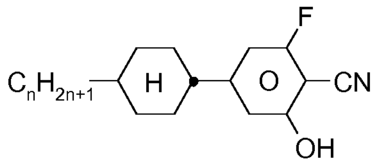

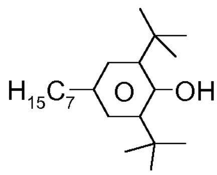

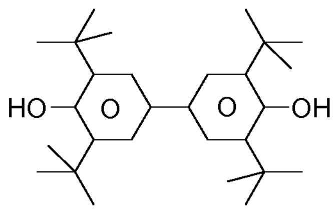

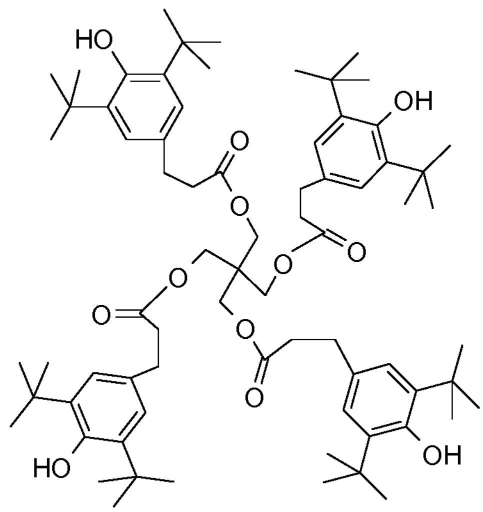

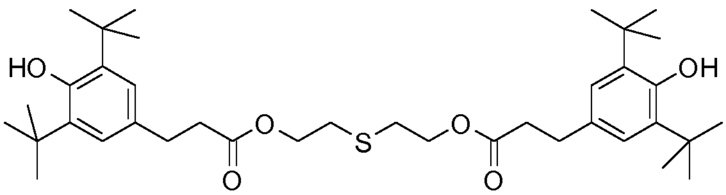

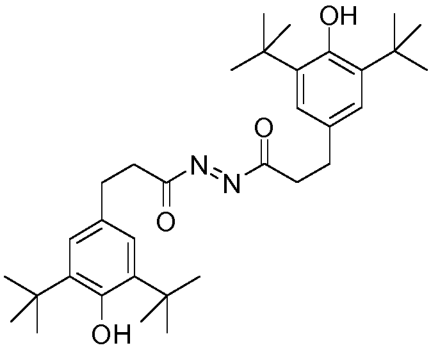

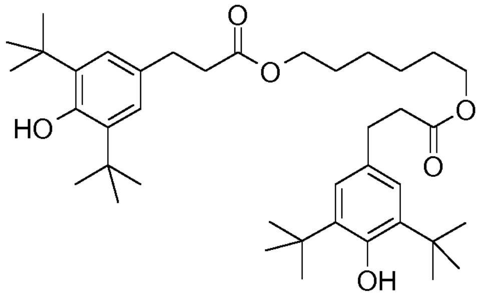

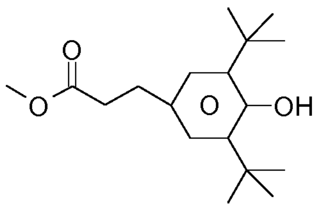

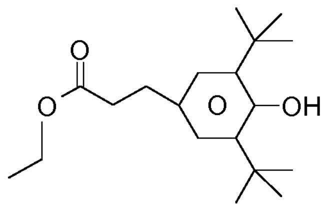

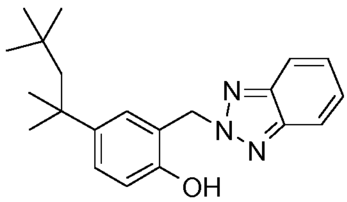

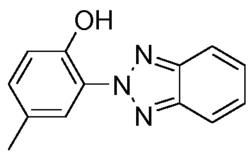

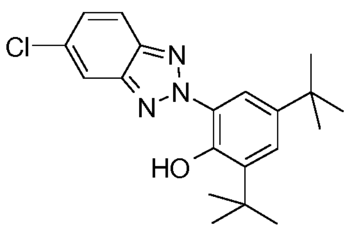

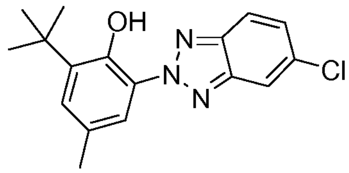

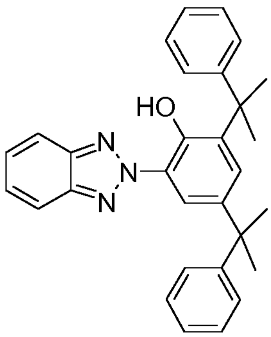

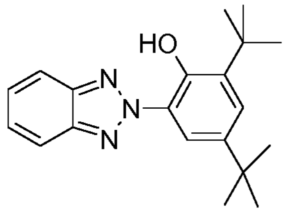

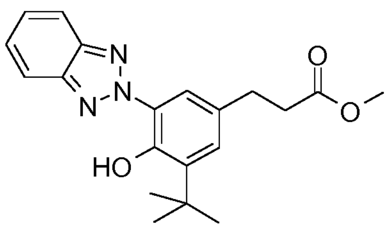

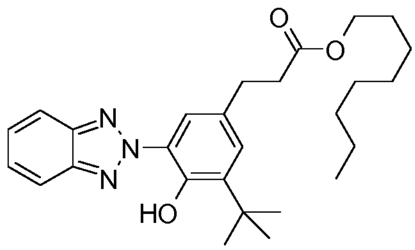

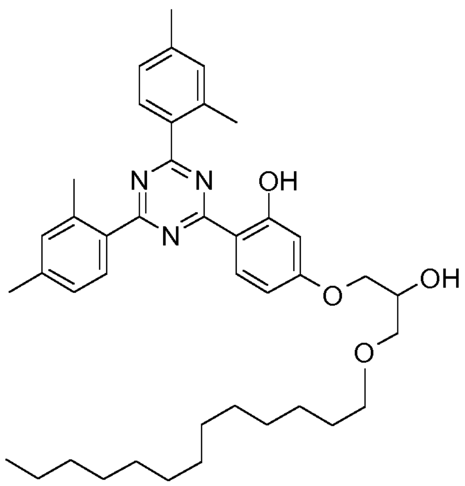

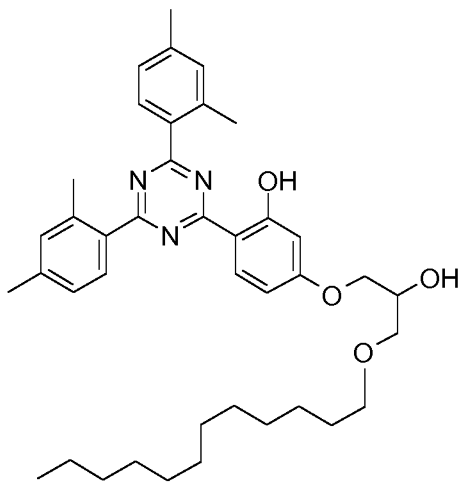

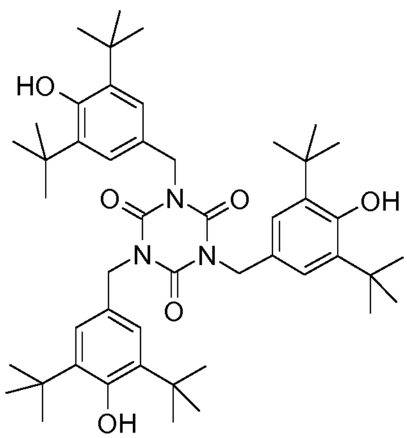

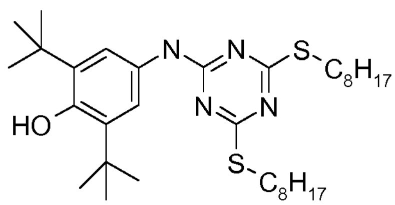

In einer bevorzugten Ausführungsform der vorliegenden Erfindung enthalten die erfindungsgemäßen Medien eine oder mehrere Verbindungen ausgewählt aus der Gruppe der Verbindungen der Tabelle F, insbesondere eine oder mehrere Verbindungen ausgewählt aus der Gruppe der Verbindungen der beiden Formeln

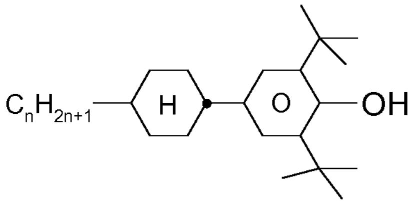

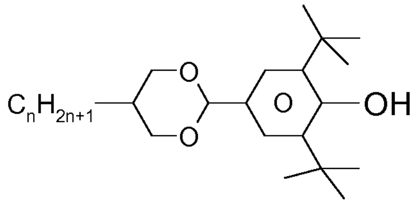

.

Beispiele und Vergleichsbeispiele

Die folgenden Beispiele erläutern die vorliegende Erfindung, ohne sie in irgendeiner Weise zu beschränken. Jedoch wird dem Fachmann deutlich, welche Eigenschaften erzielbar sind und in welchen Bereichen sie modifizierbar sind. Insbesondere werden also die verschiedenen Eigenschaften und ihre Kombinationen, die vorzugsweise erreicht werden können, veranschaulicht.

Synthesebeispiele

Synthesebeispiel I-1

Synthese von 4-[3-(2-Ethyl-4-{3-[(2,2,6,6-tetramethylpiperidin-4-yl)oxy]propyl}phenyl)propoxy]-2,2,6,6-tetramethylpiperidin

(Verbindung der Formel I-1)

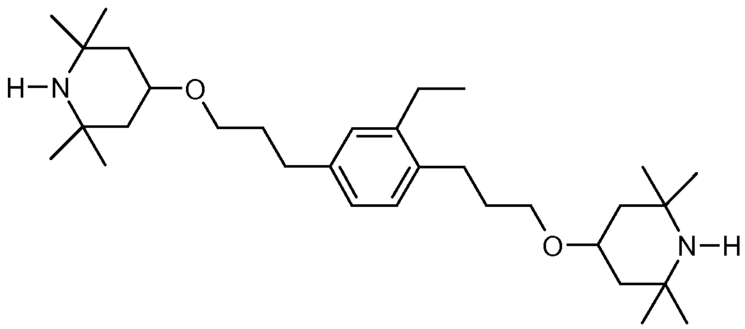

I-1

i) Synthese von 3-[3-Ethyl-4-(3-hydroxypropyl)phenyl]propan-1-ol (**2**)

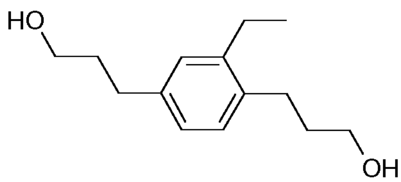

**2**

Es werden 11,0 g (103,8 mmol) Natriumcarbonat in 50 mL Wasser vorgelegt. 10,0 g (32,2 mmol) 4-Brom-2-ethyl-1-iodbenzol, 15,0 g (105,6 mmol) 2-Butoxy-[1,2]oxaborolan und 0,50 mL (3,67 mmol) Triethylamin werden in 250 mL Tetrahydrofuran gelöst und zur Reaktionslösung hinzugegeben und für 40 min unter Argonstrom entgast. Es wird nun mit 170 mg (0,96 mmol) Palladium(II)Chlorid (59% Pd, wasserfrei) und 0,90 g (1,92 mmol) 2-Dicyclohexylphoshino-2',6'-di-isopropoxy-1-1'-biphenyl versetzt und für 18h unter Rückfluss gerührt. Nach vollständiger Umsetzung wird das Reaktionsgemisch auf Raumtemperatur abgekühlt, mit Wasser und Methyltertiärbutylether (MtBE) versetzt, und die

Phasen werden getrennt. Die wässrige Phase wird mit MtBE extrahiert und die vereinigten organischen Phasen werden mit gesättigter Natriumchloridlösung gewaschen, über Natriumsulfat getrocknet, filtriert und unter Vakuum eingeengt. Das Rohprodukt wird mit Dichlormethan / Methanol über Kieselgel (300 g, 50 µm Combiflash-Anlage) filtriert und die Produktfraktionen unter Vakuum eingeengt.

Man erhält das gewünschte Produkt als gelbliches Öl.

ii) Synthese von 3-{3-Ethyl-4-[3-(methansulfonyloxy)propyl]phenyl}propyl Methansulfonat (**3**)

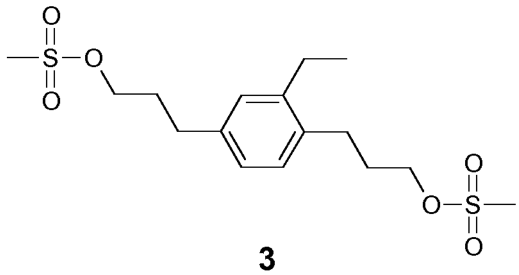

**3**

Es werden 18,2 g (81,9 mmol) an Alkohol **2** und 1,0 g (8,2 mmol) 4-(Dimethylamino)-pyridin in 100 mL Dichlormethan (DCM) vorgelegt und mit 25 mL (309,7 mmol) Pyridin versetzt. Das Reaktionsgemisch wird auf 3-4°C gekühlt und tropfenweise mit 15,0 mL (193,4 mmol) Methansufonylchlorid versetzt. 5 min nach Zugabe wird das Kühlbad entfernt und anschließend für 2 Stunden bei Raumtemperatur gerührt. Nach vollständiger Umsetzung wird das Reaktionsgemisch vorsichtig auf Wasser gegossen und die Phasen werden getrennt. Die Wasserphase wird mit DCM extrahiert und die vereinigten organischen Phasen werden mit 2N Salzsäure und Wasser gewaschen, über Natriumsulfat getrocknet, filtriert und unter Vakuum eingeengt. Das ölige Rohprodukt wird über 1 Liter (L) Kieselgel mit (DCM:MTB-Ether 98:2 nach 95:5 nach 9:1) filtriert und die Produktfraktionen unter Vakuum eingeengt.

Man erhält das Produkt als farbloses Öl.

iii)Synthese von 4-[3-(3-Ethyl-4-{3-[(1-oxyl-2,2,6,6-tetramethylpiperidin-4-yl)oxy]propyl}phenyl)propoxy]-2,2,6,6-tetramethylpiperidin-1-oxyl (freies Radikal) (**4**)

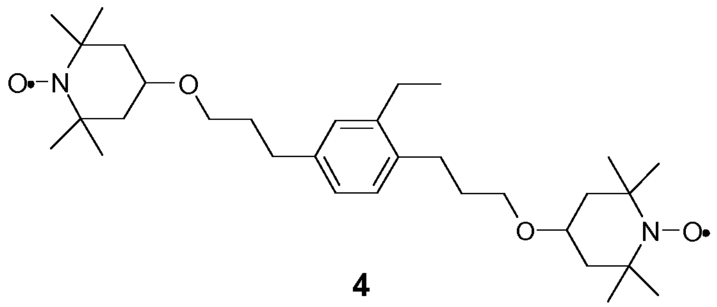

**4**

6,20 g (155 mmol) Natriumhydrid (60%ig in Paraffinöl) werden vorsichtig bei Raumtemperatur (RT) mit 50 mL N,N-Dimethylformamid (DMF) versetzt und auf 40°C erwärmt. Bei dieser Temperatur wird mit einer Lösung von 27,0 g (157 mmol) 4-Hydroxy-TEMPO (freies Radikal) tropfenweise versetzt (leichte Wasserstoffgasentwicklung). Nach vollständiger Zugabe und beendeter Gasentwicklung wird noch 2 Stunden bei 40°C gerührt und dann auf Raumtemperatur abgekühlt. Es wird nun mit einer Lösung von 23,6 g (62,4 mmol) an Mesylat **3** in 100 mL DMF innerhalb von 10 min versetzt (die Temperatur steigt hierbei um 5°C auf 30°C an) und anschließend für 48 h bei Raumtemperatur nachgerührt. Die Reaktionslösung wir vorsichtig auf Eiswasser gegossen und mit MTB-Ether extrahiert. Die Phasen werden getrennt und die Wasserphase mit MTB-Ether extrahiert. Die organischen Phasen werden vereinigt, über Natriumsulfat getrocknet, filtriert und unter Vakuum eingeengt. Das Rohprodukt fällt als rotes Öl an und wird über 1,3 L Kieselgel mit einem Gemisch aus Heptan (H) / Essigsäureethylester (EE) (2:1) filtriert. Die Produktfraktionen werden unter Vakuum eingeengt und man erhält das Produkt als roten Feststoff. Es wird nochmals über 1 L Kieselgel mit H: EE (3:1 nach 2:1) filtriert und das erhaltene Produkt aus 250 mL Heptan und 10 mL Ethanol bei -20°C kristallisiert.

Das Produkt fällt als orangener Feststoff an.

iv)Synthese von 4-[3-(3-Ethyl-4-{3-[(2,2,6,6-tetramethylpiperidin-4-yl)oxy]propyl}phenyl)propoxy]-2,2,6,6-tetramethylpiperidin (Verbindung der Formel I-1)

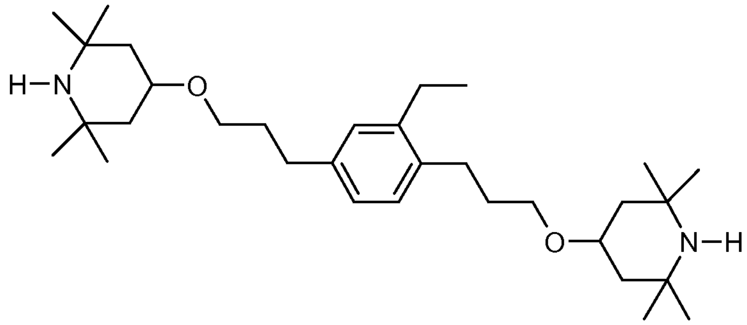

I-1

21,4 g (40,3 mmol) an freiem Radikal **4** werden in 200 mL Tetrahydrofuran gelöst (THF) und mit 10g Sponge-Nickel-Catalyst (watery) (Johnson Matthey GmbH A-7000) unter 5 bar Wasserstoffatmosphäre und 50°C für 16 Stunden hydriert. Es wird zwei Mal mit 10 g Katalysator nachgesetzt (nach Filtration) und für weitere 34 Stunden unter 5 bar Wasserstoffatmosphäre und 50°C reduziert. Die Reaktionslösung wird vom Katalysator abfiltriert und eingeengt und mit MTB-Ether über 500 mL Alox (basisch) filtriert und unter Vakuum eingeengt. Das Rohprodukt wird vorsichtig in 100 mL 2N Salzsäure gelöst und 2 Mal mit MTB-Ether extrahiert. Anschließend wird die wässrige Phase (enthaltenes Hydrochlorid-Produkt) vorsichtig mit 32%iger NaOH-Lösung auf pH 9-10 eingestellt und die Wasserphase mehrfach mit MTB-Ether extrahiert. Die vereinigten organischen Phasen werden mit Wasser gewaschen, über Natriumsulfat getrocknet, filtriert und unter Vakuum eingeengt. Das erhaltene farblose Öl wird unter 0,4 mbar 4 Stunden getrocknet.

Phasen: $T_g$ -43°C Isotrop

$^1H$ NMR (500 MHz, $CDCl_3$)

$\delta$ = 0,68 ppm s (breit) (2 H, 2 X NH), 1,03 (dt, 3,78, 11,74 Hz, 4 H), 1,14, 1,15, 1,18, 1,18 (4 S, 24 H, $CH_3$), 1,24 (t, 7,54 Hz, 3 H, $CH_3$), 1,89 ($m_c$, 4 H), 1,96 (dd, 3,97, 12,6 Hz, 4 H), 2,61 - 2,71 (m, 6H), 3,52 (2 t $_{(überlagert)}$, 7,53 Hz, 4 H), 3,66 ($m_c$, 2 H, CH), 6,96 (dd, 1,59, 7,71 Hz, 1 H, arom.-H), 7,03 (s (breit), 1 H, arom.-H), 7,09, (d, 7,72 Hz, 1 H, arom.-H).

Synthesebeispiel I-6

Synthese von 4-[3-(2,5-Difluor-4-{3-[(2,2,6,6-tetramethylpiperidin-4-yl)oxy]propyl}phenyl)propoxy]-2,2,6,6-tetramethylpiperidin

(Verbindung der Formel I-6)

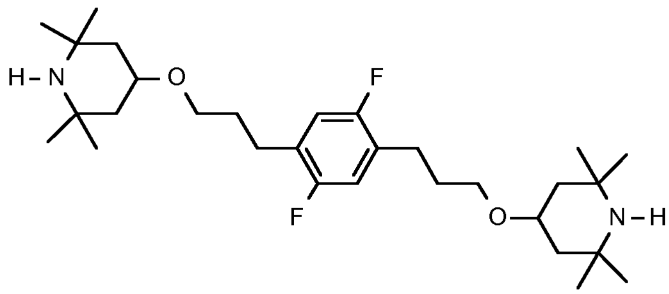

I-6

Verbindung I-6 wird analog zum Synthesebeispiel I-1 der Verbindung I-1, ausgehend von kommerziell erhältlichem 1,4-Dibrom-2,5-difluorbenzol, hergestellt.

Phasen: Tm (Schmelzpunkt) = 79°C Isotrop

$^1H$ NMR (500 MHz, $CDCl_3$)

δ = 0,69 ppm ($S_{(breit)}$, 2 H, NH), 1,01 (dd, 12,3, 12,3 Hz, 4 H, $CH_2$), 1,14, 1,18 (2 X s (überlagert mit 2 X NH) 26 H, $CH_3$, NH), 1,86 (q, 7,24 Hz, 4 H, $CH_2$), 1,94 (dd, J = 3,97, 12,61 Hz, 4 H, $CH_2$), 2,67 (t, J = 7,49 Hz, 4 H, $CH_2$), 3,49 (t, J = 6,32 Hz, 4H, $CH_2$), 3,61- 3,69 (m, 2 H, CH), 6,84 (t, J = 7,74 Hz, 2 H, arom-H).

Synthesebeispiel I-9

Synthese von 2,2,6,6-Tetramethyl-4-({10-[(2,2,6,6-tetramethylpiperidin-4-yl)oxy]decyl}oxy)piperidin

(Verbindung der Formel I-9)

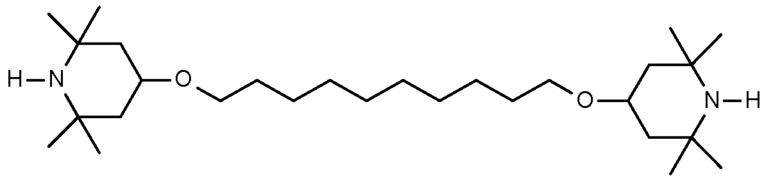

I-9

41,9 g (266 mmol) 2,2,6,6-Tetramethylpiperidin-4-ol werden in 700 mL Toluol gelöst und portionsweise mit 28,7 g (1,2 mol) NaH versetzt. Es wird für 15 min bei Raumtemperatur gerührt und anschließend mit 40,0 g (133 mmol) 1,10-Dibromdekan versetzt. Das Reaktionsgemisch wird für 16 Stunden unter Rückfluss gerührt, auf Raumtemperatur abgekühlt und vorsichtig mit Wasser gequencht (Wasserstoffentwicklung). Die organische Phase wird abgetrennt, mit Wasser gewaschen, über Natriumsulfat getrocknet und unter Vakuum eingeengt. Der erhaltene ölige Rückstand wird an Kieselgel mit Heptan und dann mit MethylTertiärbutylether (MTB-E) säulen-chromatographisch aufgereinigt.

Phasen: Tm (Schmelzpunkt) = 36°C Isotrop

$^1$H NMR (500 MHz, $CDCl_3$)

δ = 0,76 ppm ($s_{(breit)}$, 2 H, NH), 1,00 (t, J = 11,77 Hz, 4 H, $CH_2$), 1,14 (s, 12 H, $CH_3$), 1,18 (s, 12H, $CH_3$), 1,23 -1,37 (m, 12 H, $CH_2$), 1,56 (quint, 7,23 Hz, 4 H, $CH_2$), 1,95 (dd, J = 3,98, 12,65 Hz, 4 H, $CH_2$), 3.46 (t, 6,76 Hz, 4 H, $CH_2$), 3,65 ($m_c$, 2 H, CH).

Synthesebeispiel I-12

Synthese von 4-{2-[2-Fluor-6-(2-phenylethyl)-3-{3-[(2,2,6,6-tetramethylpiperidin-4-yl)oxy]propyl}phenoxy]ethoxy}-2,2,6,6-tetramethylpiperidin

(Verbindung der Formel I-12)

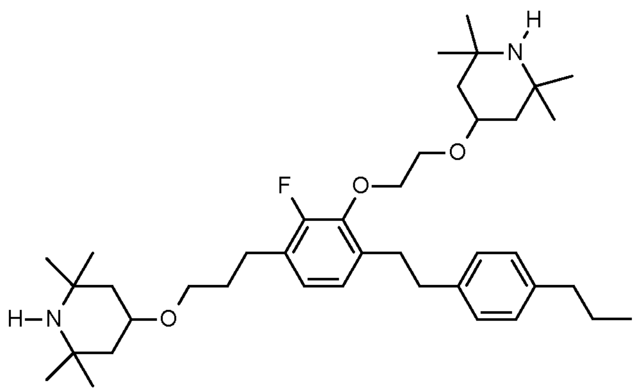

I-12

i) Synthese von [2-(6-Brom-2-fluor-3-iodphenoxy)ethoxy](tert-butyl)dimethylsilan (**5**)

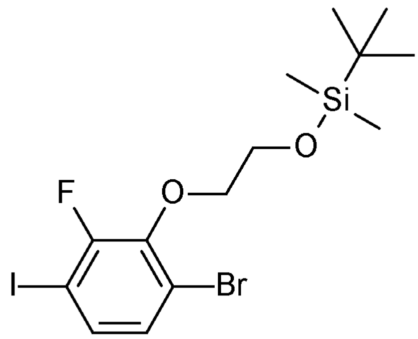

**5**

43,3 g (135,6 mmol) kommerziell erhältliches 6-Brom-3-fluor-3-iod-phenol, 38,10 g (159,3 mmol) (2-Bromethoxy)-tert-butyl-dimethylsilan werden in 130 mL N,N-Dimethylformamid (DMF) gelöst. Das Reaktionsgemisch wird mit 66,95 g (203,4 mmol) Cäsiumcarbonat versetzt und anschließend bei 50°C für 16 Stunden gerührt. Das Reaktionsgemisch wird auf Eiswasser gegossen, mehrfach mit Essigsäureethylester (EE) extrahiert, die organischen Phasen werden vereinigt, über Natriumsulfat getrocknet, filtriert und unter Vakuum eingeengt. Das Rohprodukt fällt als braune Flüssigkeit an und wird über Kieselgel mit Heptan und Heptan / Toluol (10:1) filtriert. Die Produktfraktionen werden unter Vakuum eingeengt und das erhaltene farblose Öl wird bei 160°C und 6 mbar Vakuum von Resten DMF abgetrennt.

ii) Synthese von [2-(6-Brom-3-{3-[(tert-butyldimethylsilyl)oxy]prop-1-yn-1-yl}-2-fluorophenoxy)ethoxy](tert-butyl)dimethylsilan (**6**)

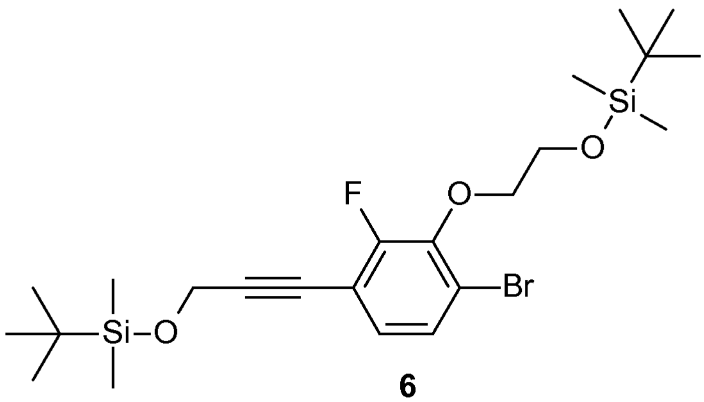

**6**

28,0 g (58,9 mmol) Bromid **5** werden in 300 mL Triethylamin bei Raumtempratur (RT) vorgelegt und entgast. Es werden 1,30 g (1,85 mmol) Bis(triphenylphosphin)-palladium(II)-chlorid (15,2% Pd) und 350 mg (1,84 mmol)Kupfer(I)-Iodid zugestetzt und bei Raumtemperatur mit einer Lösung von 20,0 g (117 mmol) tert-Butyl-dimethyl-prop-2-ynyloxy-silan in 150mL entgasten Triethylamin zugetropft. Das Reaktionsgemisch erwärmt sich beim Zutropfen auf 31°C und es entsteht eine dicke Suspension, die 2 Stunden bei Raumtemperatur nachgerührt wird. Das Reaktionsgemisch wird abgesaugt, das Filtrat unter Vakuum eingeengt und mit Methyl-tert-butylether (MTB-E) gelöst. Der Filterrückstand wird mit MTB-E extrahiert. Die vereinigten organischen Phasen werden mit Ammoniumchlorid- und Natriumchloridlösung gewaschen, über Natriumsulfat getrocknet, filtriert und unter Vakuum eingeengt. Man erhält das Rohprodukt als dunkles Öl, welches an Kieselgel mit Heptan / Toluol (9:1 bis 1:1) aufgereinigt wird. Das erhaltene Produkt fällt als orangenes Öl an.

iii)Synthese von tert-Butyl[2-(3-{3-[(tert-butyldimethylsilyl)oxy]prop-1-yn-1-yl}-2-fluor-6-(2-phenylethynyl)phenoxy)ethoxy]dimethylsilan (**7**)

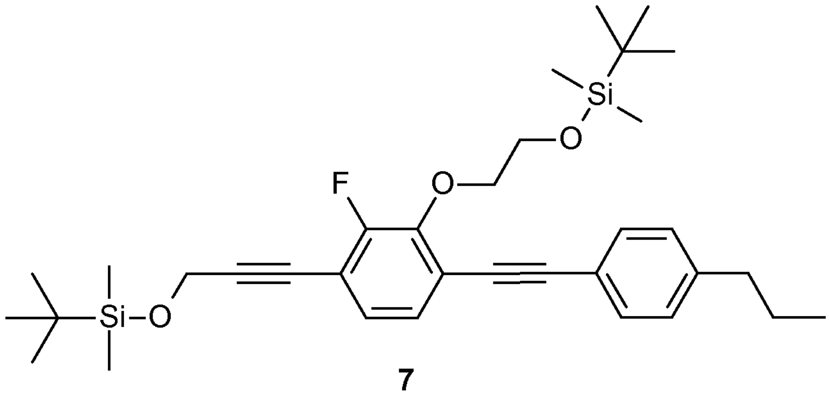

**7**

24,1 g (46,6 mmol) Bromid **6** und 8,00 g (55,5 mmol) 1-Ethinyl-4-propylbenzol werden in 300 mL Diisopropylamin gelöst und für 30 min entgast. 500 mg (2,23 mmol) Palladium(II)acetat, 700 mg (2,41 mmol) Tritert-butylphosphonium-tetrafluoroborat und 350 mg (1,84 mmol) Kupfer(I)-Iodid werden zum Reaktionsgemisch zugesetzt und eine Stunde bei 80°C gerührt. Das Reaktionsgemisch wird dunkel und es fällt ein Niedererschlag aus. Es wird auf Raumtemperatur abgekühlt, filtriert und unter Vakuum eingeengt. Das Filtrat wird in MTB-Ether aufgenommen und die abgesaugten Ammoniumsalze mit MTB-Ether gespült bis das Filtrat farblos ist. Die organischen Phasen werden vereinigt, mit Ammoniumchlorid- und Natriumchloridlösung gewaschen, über Natriumsulfat getrocknet, filtriert und unter Vakuum eingeengt. Das Rohprodukt fällt als schwarzes Öl an, welches über Kieselgel mit Heptan / Toluol (7:3 bis 1:1) filtriert wird. Die Produktfraktionen werden vereinigt und unter Vakuum eingeengt. Man erhält das Produkt als orangen, teilkristallinen Feststoff.

iv)Synthese von tert-Butyl[2-(3-{3-[(tert-butyldimethylsilyl)oxy]propyl}-2-fluor-6-(2-phenylethyl)phenoxy)ethoxy]dimethylsilan (**8**)

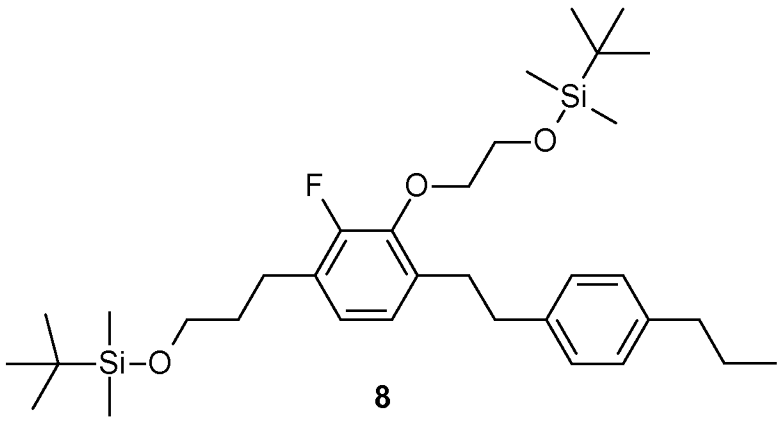

**8**

22,2 g (38,2 mmol) der Verbindung **7** und 2,00 g Sponge-NickelKatalysator (Johnson Matthey, wässrig, A-700) werden in 220 mL Tetrahydrofuran (THF) gelöst und bei Raumtemperatur und Normaldruck für 20,5 Stunden unter Wasserstoffatmosphäre gerührt. Das Reaktionsgemisch wird filtriert und die Lösunug unter Vakkum eingeengt. Das Rohprodukt wird über Kieselgel mit Heptan / Chlorbutan (1:1 bis 1:2) aufgereinigt. Die Produktfraktionen werden unter Vakuum eingeengt und man erhält das Reaktionsprodukt als orangenes Öl.

v) Synthese von 3-[2-Fluor-3-(2-hydroxyethoxy)-4-(2-phenylethyl)phenyl]-propan-1-ol (**9**)

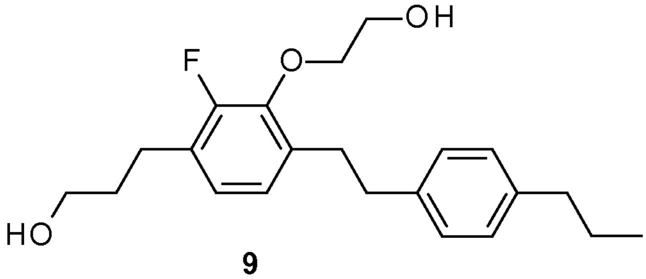

9

17,3 g (27,7 mmol) der Verbindung **8** werden in 200 mL Tetrahydrofuran gelöst und auf 2°C eingekühlt. Es wird mit 80,0 mL (80 mmol) einer 1 molaren Lösung von Tetrabutylammoniumfluorid tropfenweise versetzt und 3 Stunden bei Raumtemperatur nachgerührt. Die Reaktionslösung wird auf eine eiskalte Natriumhydrogencabonat / MTB-Ether Mischung gegossen, kurz nachgerührt, und die Phasen werden getrennt. Die Wasserphase wird mit MTB-Ether extrahiert und die vereinigten organischen Phasen werden mit Natriumchloridlösung gewaschen, über Natriumsulfat getrocknet, filtriert und unter Vakuum eingeengt. Man erhält das Reaktionsprodukt als orangenes Öl, welches über Kieselgel mit Dichlormethan / Methanol (95:5 bis 9:1) aufgereinigt wird. Das erhaltene Rohprodukt wird aus Heptan bei -25°C kristallisiert und man erhält Verbindung **9** als beige Kristalle.

$^1H$ NMR (500 MHz, $CDCl_3$)

$\delta$ = 0,96 ppm (t, J = 7,35 Hz, 3 H, $CH_3$), 1,32 ($s_{(breit)}$, 1 H, OH), 1,66 (sext., J = 7,41 Hz, 2 H, $CH_2CH_3$), 1,86 -1,95 ($m_c$ 2 H, $CH_2$), 2,07 ($s_{(breit)}$, 1 H, OH), 2,58 (dd, J = 7,54 Hz, 2 H, $CH_2$), 2,75 ($t_{(breit)}$, J = 8,29 Hz, 2 H, $CH_2$), 2,91 ($m_c$, 4 H), 3,71 (t, J = 6,37 Hz, 2 H, $CH_2$), 3,92 ($dd_{(breit)}$, 4,61 Hz, 2 H, $CH_2$), 4,08 ($dd_{(breit)}$, 4,08 Hz, 2 H, $CH_2$), 6,84 - 6,92 (m, 2 H), 7,12 (s, 4 H).

vi)Synthese von 4-{2-[2-Fluor-6-(2-phenylethyl)-3-{3-[(2,2,6,6-tetramethylpiperidin-4-yl)oxy]propyl}phenoxy]ethoxy}-2,2,6,6-tetramethylpiperidin

(Verbindung der Formel I-12)

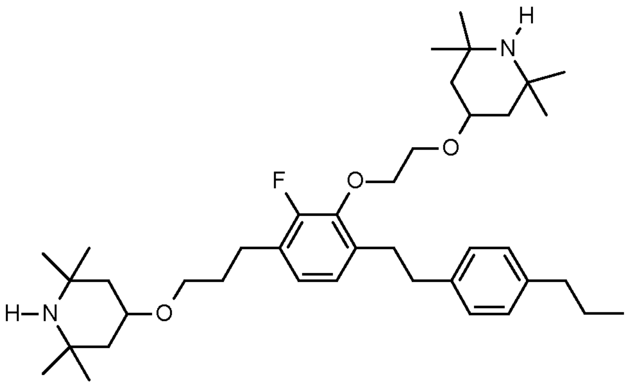

I-12

Ausgehend von Verbindung **9** wird Verbindung I-12 analog dem gezeigten Synthesebeispiel I-1 hergestellt. Man erhält das Produkt als farbloses Öl.

Phasen: $T_g$ -31°C Isotrop

$^1H$ NMR (500 MHz, $CDCl_3$)

$\delta$ = 0,61-0,78 ppm (2 $s_{(breit,\ überlappend)}$, 2 H, OH), 0,86 (t, J = 7,36 Hz, 3 H, $CH_3$), 0,92 (d, J = 11,3 Hz, 2 H, $CH_2$), 0,96 (d, J = 11,22 Hz, 2 H, $CH_2$), 1,04 (s, 6 H, $CH_3$), 1,08 (s, 6 H, $CH_3$), 1.09 (s, 6 H, $CH_3$), 1,12 (s, 6 H, $CH_3$), 1,55 (sext, J = 7,57 Hz, 2 H, $CH_2CH_3$), 1,79 (quint, J = 6,93 Hz, 2 H, $CH_2$), 1,89 ($m_c$, 4 H), 2,48 (t, J = 7,8 Hz, 2 H, $CH_2$), 2,62 (t, J = 7,51 Hz, 2 H, $CH_2$), 2,82 ($m_c$, 4 H, $CH_2$), 3,43 (t, J = 6,42 Hz, 2 H, $CH_2$), 3,58 ($m_c$, 1 H, CH), 3,61- 3,78 (m, 3 H, $CH_2$, CH), 4,08 (t, J = 5,1 Hz, 2 H, $CH_2$), 6,69 - 7,78 (m, 2 H), 7,01 (d, J = 8 Hz, 2 H), 7,07 (d, J = 7,99 Hz).

Es werden Flüssigkristallmischungen mit den Zusammensetzungen und den Eigenschaften wie in den folgenden Tabellen angegeben hergestellt und untersucht. Die verbesserte bzw. günstige Stabilität der Mischungen enthaltend Verbindungen der Formel I wird durch entsprechende Vergleiche mit unstabilisierten Basismischungen oder anders stabilisierten Mischungen gezeigt.

Referenzbeispiel 1, Beispiele 1.1-1.12 und Vergleichsbeispiele 1.a-1.c

Die folgende Mischung (M-1) wird hergestellt und untersucht.

| Mischung M-1 | | | | | |
|---|---|---|---|---|---|
| Zusammensetzung | | | Physikalische Eigenschaften | | |
| Verbindung | | Konzentration | T(N, I) [°C)] | = | 85,0 |
| Nr. | Abkürzung | /Massen-% | $n_e$(20°C,589 nm) | = | 1,587 |
| 1 | CC-3-V | 32,00 | $\Delta$n(20°C,589 nm) | = | 0,109 |
| 2 | CC-3-V1 | 11,00 | $\varepsilon_\perp$(20°, 1 kHz) | = | 3,7 |
| 3 | CC-3-2V1 | 4,50 | $\Delta\varepsilon$(20°, 1 kHz) | = | 15,3 |
| 4 | PP-1-2V1 | 2,00 | $k_{11}$(20°C) [pN] | = | 14,4 |
| 5 | CCP-3-OT | 7,50 | $k_{33}$(20°C) [pN] | = | 15,1 |
| 6 | CCP-5-OT | 1,50 | $V_0$(20°C) [V] | = | 1,01 |
| 7 | PUQU-3-F | 1,50 | | | |
| 8 | APUQU-2-F | 7,00 | | | |
| 9 | APUQU-3-F | 7,00 | | | |
| 10 | PGUQU-3-F | 3,00 | | | |
| 11 | PGUQU-4-F | 8,00 | | | |
| 12 | PGUQU-5-F | 2,00 | | | |
| 13 | DPGU-4-F | 5,00 | | | |
| 14 | DGUQU-4-F | 8,00 | | | |
| $\Sigma$ | | 100,0 | | | |

Die Mischung M-1 wird in 16 Teile geteilt und wie im weiteren beschrieben untersucht.

Der erste Teil der Mischung M-1 wird wie bereitgestellt im Referenzbeispiel 1 (Ref.) verwendet.

Drei der verbleibenden Teile wird jeweils TINUVIN®770, im Folgenden T770 genannt, d.h. eine Verbindung der Formel

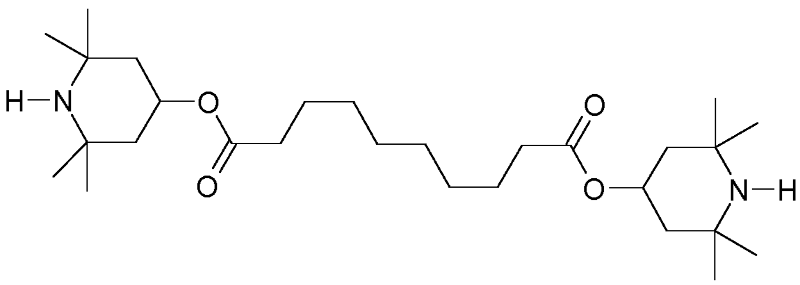
,

hinzugefügt, jeweils in einer Konzentration wie nachfolgend in Tabelle 1 aufgeführt. Die so hergestellten Mischungen M-1.a, M-1.b und M-1.c werden in den Vergleichsbeispielen 1.a bis 1.c (Vgl.) verwendet.

Den restlichen 12 Teilen wird erfindungsgemäß jeweils eine Verbindung der wie oben gezeigten Formeln I-1, I-6-, I-9 bzw. I-12, jeweils in einer Konzentration wie nachfolgend in Tabelle 1 aufgeführt, hinzugefügt. Die so hergestellten Mischungen M-1.1 bis M-1.12 werden in den erfindungsgemäßen Beispielen 1.1 bis 1.12 verwendet.

Tabelle 1

| | Mischung | Stabilisator | Konzentration des Stabilisators [ppm] |
|---|---|---|---|
| 1 (Ref.) | M-1 | - | - |
| 1.a (Vgl.) | M-1.a | T770 | 100 |
| 1.b (Vgl.) | M-1.b | T770 | 500 |

(fortgesetzt)

| | Mischung | Stabilisator | Konzentration des Stabilisators [ppm] |
|---|---|---|---|
| 1.c (Vgl.) | M-1.c | T770 | 1000 |
| 1.1 | M-1.1 | I-1 | 100 |
| 1.2 | M-1.2 | I-1 | 500 |
| 1.3 | M-1.3 | I-1 | 1000 |
| 1.4 | M-1.4 | I-6 | 100 |
| 1.5 | M-1.5 | I-6 | 500 |
| 1.6 | M-1.6 | I-6 | 1000 |
| 1.7 | M-1.7 | I-9 | 100 |
| 1.8 | M-1.8 | I-9 | 500 |
| 1.9 | M-1.9 | I-9 | 1000 |
| 1.10 | M-1.10 | I-12 | 100 |
| 1.11 | M-1.11 | I-12 | 500 |
| 1.12 | M-1.12 | I-12 | 1000 |

Diese Mischungen werden wie nachfolgend beschrieben auf ihre Stabilität untersucht.

Zunächst wird das sogenannte initiale Spannungshaltevermögen (VHR (initial), Englisch "voltage holding ratio", kurz VHR) untersucht. Hierfür wird die VHR in bei der Firma Merck Japan hergestellten Testzellen bestimmt. Die Messzellen haben Substrate aus Natriumglas (Sodalime Glas) und sind mit Polyimidorientierungsschichten (AL-16301 der Firma Japan Synthetic Rubber, Japan) mit einer Schichtdicke von 50 nm, die senkrecht zueinander gerieben sind, ausgeführt. Die Schichtdicke beträgt einheitlich 6,5 μm. Die Fläche der durchsichtigen Elektroden aus ITO beträgt 1 $cm^2$.

Die VHR wird bei 20°C ($VHR_{20}$) und nach 5 Minuten im Ofen bei 100°C ($VHR_{100}$) in einem kommerziell erhältlichen Gerät der Firma Autronic Melchers, Deutschland, bestimmt, bei 1V und 60 Hz.

Die so ermittelten Werte von VHR (initial) sind in Tabelle 2 aufgeführt.

Des Weiteren wird die Lichtbeständigkeit gegen Sonnenlicht, d.h. die Stabilität gegen Bestrahlung mit UV/Sonnenlicht in einem kommerziellen "Suntest CPS" Gerät der Firma Heraeus, Deutschland, untersucht.

Zur Bestimmung der VHR in bezug auf die Sonnenlichtbeständigkeit wird eine Lampe verwendet, die das Wellenlängen-Spektrum des Sonnenlichts emittiert. Der Test wird bei 20°C durchgeführt und die Bestrahlungsdauer entspricht 30 min. Dabei werden die versiegelten Testzellen 0,5 Stunden ohne zusätzliche thermische Belastung bei 20°C mit einer Lampe (Hoya, 340 nm cut Filter, T=50% bei 340 nm) bestrahlt, wobei die mit einem 365 nm Sensor gemessene Intensität 3 $J/cm^2$ beträgt. Danach wird jeweils die Voltage Holding Ratio nach 5 Minuten bei einer Temperatur von 100°C bestimmt.

Die so für den Suntest ermittelten VHR-Werte (VHR (suntest)) sind ebenfalls in Tabelle 2 aufgeführt.

Tabelle 2

| | Mischung | VHR (initial) [%] | VHR (suntest) [%] |
|---|---|---|---|
| 1 (Ref.) | M-1 | 96,4 | 72,4 |
| 1.a (Vgl.) | M-1.a | 98,0 | 89,3 |
| 1.b (Vgl.) | M-1.b | 98,0 | 85,7 |
| 1.c (Vgl.) | M-1.c | 97,8 | 85,9 |
| 1.1 | M-1.1 | 98,6 | 90,7 |
| 1.2 | M-1.2 | 98,4 | 86,5 |
| 1.3 | M-1.3 | 98,5 | 82,8 |
| 1.4 | M-1.4 | 98,7 | 86,0 |
| 1.5 | M-1.5 | 98,9 | 78,4 |

(fortgesetzt)

| | Mischung | VHR (initial) [%] | VHR (suntest) [%] |
|---|---|---|---|
| 1.6 | M-1.6 | 99,0 | 71,9 |
| 1.7 | M-1.7 | 98,3 | 85,3 |
| 1.8 | M-1.8 | 98,4 | 72,4 |
| 1.9 | M-1.9 | 98,0 | 61,5 |
| 1.10 | M-1.10 | 98,4 | 90,2 |
| 1.11 | M-1.11 | 98,0 | 81,8 |
| 1.12 | M-1.12 | 97,9 | 75,6 |

Es zeigt sich, dass die erfindungsgemäßen Mischungen deutlich stabilisiert werden können, auch bereits wenn sie die entsprechenden Verbindungen der Formel I in relativ niedrigen Konzentrationen enthalten.

Des Weiteren wird die Lichtbeständigkeit gegen Hintergrundbeleuchtung (Englisch: "backlight") untersucht, wobei die Mischungen in einer Testzelle mit einem Orientierungsmaterial für planare Ausrichtung und flächigen ITO-Elektroden auf ihre Lichtbeständigkeit (Hintergrundbeleuchtung) untersucht werden. Zur Bestimmung der VHR bezüglich der Lichtbeständigkeit gegenüber einer Hintergrundbeleuchtung werden versiegelte Testzellen mit einer kommerziell erhältlichen Hintergrundbeleuchtungseinheit getestet, ohne zusätzliche thermische Belastung. Die Bestrahlungsdauer entspricht max. 1000 h. Die Ergebnisse (VHR (BL)) sind in Tabelle 3 aufgeführt.

Tabelle 3

| | | VHR (initial) [%] | VHR (BL) [%] | | | | | |
|---|---|---|---|---|---|---|---|---|
| | | | 48h | 168h | 336h | 504h | 744h | 1000h |
| 1 (Ref.) | M-1 | 95,4 | 81,5 | 69,0 | 65,1 | 62,7 | 61,4 | 59,7 |
| 1.a (Vgl.) | M-1.a | 97,8 | 95,9 | 90,8 | 86,5 | 82,9 | 79,3 | 76,8 |
| 1.b (Vgl.) | M-1.b | 97,5 | 95,7 | 92,6 | 89,9 | 87,4 | 85,0 | 82,5 |
| 1.c (Vgl.) | M-1.c | 97,3 | 96,0 | 93,9 | 90,9 | 88,5 | 86,5 | 84,8 |
| 1.1 | M-1.1 | 98,1 | 96,8 | 93,9 | 90,9 | 88,5 | 87,6 | 83,7 |
| 1.2 | M-1.2 | 98,2 | 97,4 | 95,2 | 93,0 | 91,1 | 89,8 | 87,9 |
| 1.3 | M-1.3 | 98,2 | 97,1 | 95,1 | 92,8 | 90,8 | 89,2 | 87,5 |
| 1.4 | M-1.4 | 98,7 | 97,3 | 94,2 | 91,7 | 88,8 | 86,2 | 84,0 |
| 1.5 | M-1.5 | 98,4 | 97,2 | 94,4 | 91,4 | 87,8 | 84,9 | 85,8 |
| 1.6 | M-1.6 | 98,2 | 97,1 | 93,5 | 91,6 | 89,5 | 87,6 | 86,0 |
| 1.7 | M-1.7 | 97,2 | 96,6 | 94,4 | 92,1 | 89,5 | 86,6 | 84,7 |
| 1.8 | M-1.8 | 97,4 | 95,5 | 92,3 | 89,5 | 87,3 | 85,3 | 83,9 |
| 1.9 | M-1.9 | 97,0 | 95,1 | 91,9 | 89,0 | 86,9 | 84,8 | 83,3 |
| 1.10 | M-1.10 | 98,5 | 97,5 | 93,6 | 91,7 | 89,3 | 85,4 | 82,0 |
| 1.11 | M-1.11 | 98,2 | 97,4 | 95,1 | 92,8 | 90,9 | 88,2 | 85,6 |
| 1.12 | M-1.12 | 98,1 | 96,8 | 94,2 | 91,1 | 88,5 | 85,9 | 84,1 |

Es zeigt sich, dass die erfindungsgemäßen Mischungen auch hinsichtlich der Hintergrundbeleuchtung deutlich stabilisiert werden können, auch bereits wenn sie die entsprechenden Verbindungen der Formel I in relativ niedrigen Konzentrationen enthalten.

Zum Vergleich und als Referenz werden die Mischungen M-1, M-1.a, M-1.b und M-1.c ferner hinsichtlich ihrer Hitzebeständigkeit untersucht. Dabei werden diese Mischungen in einer Testzelle mit einem Orientierungsmaterial für

planare Ausrichtung und flächigen ITO-Elektroden auf ihre Hitzebeständigkeit untersucht. Zur Bestimmung der VHR in Abhängigkeit der Hitzebeständigkeit werden versiegelte Testzellen für 120h in einem herkömmlichen Laborheizschrank bei 100 °C aufbewahrt. Die Ergebnisse (VHR (heat)) werden in Tabelle 4 gezeigt.

Tabelle 4

| | Mischung | VHR (initial) [%] | VHR (heat) [%] |
|---|---|---|---|
| 1 (Ref.) | M-1 | 96,3 | 91,1 |
| 1.a (Vgl.) | M-1.a | 97,7 | 90,2 |
| 1.b (Vgl.) | M-1.b | 98,5 | 94,0 |
| 1.c (Vgl.) | M-1.c | 97,8 | 84,9 |

Referenzbeispiel 2, Beispiele 2.1-2.9 und Vergleichsbeispiele 2.a-2.c

Die folgende Mischung (M-2) wird hergestellt und untersucht.

| Mischung M-2 | | | | | |
|---|---|---|---|---|---|
| Zusammensetzung | | | Physikalische Eigenschaften | | |
| Verbindung | | Konzentration | $T(N, I)$ [°C)] | = | 85,5 |
| Nr. | Abkürzung | /Massen-% | $n_e$(20°C,589 nm) | = | 1,588 |
| 1 | CCY-3-O1 | 3,50 | $\Delta n$(20°C,589 nm) | = | 0,105 |
| 2 | CLY-3-O2 | 10,00 | $\varepsilon_\perp$(20°, 1 kHz) | = | 6,9 |
| 3 | CLY-3-O3 | 2,50 | $\Delta\varepsilon$(20°, 1 kHz) | = | -3,4 |
| 4 | CPY-2-O2 | 11,00 | $k_{11}$(20°C) [pN] | = | 14,7 |
| 5 | CPY-3-O2 | 12,50 | $k_{33}$(20°C) [pN] | = | 17,7 |
| 6 | PYP-2-3 | 4,00 | $V_0$(20°C) [V] | = | 2,41 |
| 7 | CC-3-V | 28,00 | | | |
| 8 | CC-3-V1 | 12,00 | | | |
| 9 | CY-3-O2 | 13,50 | | | |
| 10 | CY-5-O2 | 3,00 | | | |
| $\Sigma$ | | 100.0 | | | |

Die Mischung M-2 wird in 13 Teile geteilt und wie im weiteren beschrieben untersucht.

Der erste Teil der Mischung M-2 wird wie bereitgestellt im Referenzbeispiel 2 (Ref.) verwendet.

Drei der verbleibenden Teile wird jeweils TINUVIN®770 (T770) hinzugefügt, jeweils in einer Konzentration wie nachfolgend in Tabelle 5 aufgeführt. Die so hergestellten Mischungen M-2.a, M-2.b und M-2.c werden in den Vergleichsbeispielen 2.a bis 2.c (Vgl.) verwendet.

Den restlichen 9 Teilen wird erfindungsgemäß jeweils eine Verbindung der wie oben gezeigten Formeln I-1, I-6 bzw. I-9, jeweils in einer Konzentration wie nachfolgend in Tabelle 5 aufgeführt, hinzugefügt. Die so hergestellten Mischungen M-2.1 bis M-2.9 werden in den erfindungsgemäßen Beispielen 2.1 bis 2.9 verwendet.

Tabelle 5

| | Mischung | Stabilisator | Konzentration des Stabilisators [ppm] |
|---|---|---|---|
| 2 (Ref.) | M-2 | - | - |
| 2.a (Vgl.) | M-2.a | T770 | 100 |
| 2.b (Vgl.) | M-2.b | T770 | 500 |
| 2.c (Vgl.) | M-2.c | T770 | 1000 |
| 2.1 | M-2.1 | I-1 | 100 |

(fortgesetzt)

| | Mischung | Stabilisator | Konzentration des Stabilisators [ppm] |
|---|---|---|---|
| 2.2 | M-2.2 | I-1 | 500 |
| 2.3 | M-2.3 | I-1 | 1000 |
| 2.4 | M-2.4 | I-6 | 100 |
| 2.5 | M-2.5 | I-6 | 500 |
| 2.6 | M-2.6 | I-6 | 1000 |
| 2.7 | M-2.7 | I-9 | 100 |
| 2.8 | M-2.8 | I-9 | 500 |
| 2.9 | M-2.9 | I-9 | 1000 |

Diese Mischungen werden wie nachfolgend beschrieben auf ihre Stabilität untersucht.

Zunächst wird das sogenannte initiale Spannungshaltevermögen (VHR (initial)) wie oben für die Mischungen M-1 bis M-1.16 beschrieben untersucht.

Die ermittelten Werte von VHR (initial) sind in Tabelle 6 aufgeführt.

Des Weiteren wird die Lichtbeständigkeit gegen Sonnenlicht wie oben für die Mischungen M-1 bis M-1.16 beschrieben untersucht.

Die für den Suntest ermittelten VHR-Werte (VHR (suntest)) sind ebenfalls in Tabelle 6 aufgeführt.

Tabelle 6

| | Mischung | VHR (initial) [%] | VHR (suntest) [%] |
|---|---|---|---|
| 2 (Ref.) | M-2 | 72,8 | 66,0 |
| 2.a (Vgl.) | M-2.a | 82,4 | 81,3 |
| 2.b (Vgl.) | M-2.b | 84,4 | 84,3 |
| 2.c (Vgl.) | M-2.c | 83,7 | 84,6 |
| 2.1 | M-2.1 | 89,8 | 89,9 |
| 2.2 | M-2.2 | 89,9 | 89,6 |
| 2.3 | M-2.3 | 90,1 | 90,0 |
| 2.4 | M-2.4 | 89,5 | 87,3 |
| 2.5 | M-2.5 | 89,7 | 86,6 |
| 2.6 | M-2.6 | 88,3 | 84,5 |
| 2.7 | M-2.7 | 89,2 | 88,8 |
| 2.8 | M-2.8 | 89,4 | 87,6 |
| 2.9 | M-2.9 | 89,6 | 87,5 |

Überraschenderweise zeigen die Mischungen mit negativer dielektrischer Anisotropie eine besonders hervorragende Stabilisierung, wenn sie erfindungsgemäß die entsprechenden Verbindungen der Formel I enthalten, wobei vorteilhaft eine relativ geringe Menge ausreichend sein kann.

Referenzbeispiel 3, Beispiele 3.1-3.6 und Vergleichsbeispiele 3.a-3.c

Die folgende Mischung (M-3) wird hergestellt und untersucht.

| Mischung M-3 | | | | | |
|---|---|---|---|---|---|
| Zusammensetzung | | | Physikalische Eigenschaften | | |
| Verbindung | | Konzentration | T(N, I) [°C)] | = | 85,0 |
| Nr. | Abkürzung | /Massen-% | $n_e$(20°C,589 nm) | = | 1,587 |
| 1 | CCY-3-O2 | 10,00 | $\Delta$n(20°C,589 nm) | = | 0,105 |
| 2 | CCY-5-O2 | 7,00 | $\varepsilon_\perp$(20°, 1 kHz) | = | 6,9 |
| 3 | CPY-2-O2 | 10,00 | $\Delta\varepsilon$(20°, 1 kHz) | = | -3,4 |
| 4 | CPY-3-O2 | 10,00 | $k_{11}$(20°C) [pN] | = | 14,6 |
| 5 | PYP-2-3 | 5,50 | $k_{33}$(20°C) [pN] | = | 17,4 |
| 6 | B-2O-O5 | 4,00 | $V_0$(20°C) [V] | = | 2,37 |
| 7 | CC-3-V | 32,00 | | | |
| 8 | CC-3-V1 | 10,00 | | | |
| 9 | CY-3-O2 | 10,00 | | | |
| 10 | CY-5-O2 | 1,50 | | | |
| $\Sigma$ | | 100,0 | | | |

Die Mischung M-3 wird in 10 Teile geteilt und wie im weiteren beschrieben untersucht.

Der erste Teil der Mischung M-3 wird wie bereitgestellt im Referenzbeispiel 3 (Ref.) verwendet.

Drei der verbleibenden Teile wird jeweils TINUVIN®770 (T770) hinzugefügt, jeweils in einer Konzentration wie nachfolgend in Tabelle 7 aufgeführt. Die so hergestellten Mischungen M-3.a, M-3.b und M-3.c werden in den Vergleichsbeispielen 3.a bis 3.c (Vgl.) verwendet.

Den restlichen 6 Teilen wird erfindungsgemäß jeweils eine Verbindung der wie oben gezeigten Formeln I-1 bzw. I-12, jeweils in einer Konzentration wie nachfolgend in Tabelle 7 aufgeführt, hinzugefügt. Die so hergestellten Mischungen M-3.1 bis M-3.6 werden in den erfindungsgemäßen Beispielen 3.1 bis 3.6 verwendet.

Tabelle 7

| | Mischung | Stabilisator | Konzentration des Stabilisators [ppm] |
|---|---|---|---|
| 3 (Ref.) | M-3 | - | - |
| 3.a (Vgl.) | M-3.a | T770 | 100 |
| 3.b (Vgl.) | M-3.b | T770 | 500 |
| 3.c (Vgl.) | M-3.c | T770 | 1000 |
| 3.1 | M-3.1 | I-1 | 100 |
| 3.2 | M-3.2 | I-1 | 500 |
| 3.3 | M-3.3 | I-1 | 1000 |
| 3.4 | M-3.4 | I-12 | 100 |
| 3.5 | M-3.5 | I-12 | 500 |
| 3.6 | M-3.6 | I-12 | 1000 |

Diese Mischungen werden wie nachfolgend beschrieben auf ihre Stabilität untersucht.

Zunächst wird das sogenannte initiale Spannungshaltevermögen (VHR (initial)) wie oben für die Mischungen M-1 bis M-1.16 beschrieben untersucht.

Die ermittelten Werte von VHR (initial) sind in Tabelle 8 aufgeführt.

Des Weiteren wird die Lichtbeständigkeit gegen Sonnenlicht wie oben für die Mischungen M-1 bis M-1.16 beschrieben untersucht.

Die für den Suntest ermittelten VHR-Werte (VHR (suntest)) sind ebenfalls in Tabelle 8 aufgeführt.

Tabelle 8

| | Mischung | VHR (initial) [%] | VHR (suntest) [%] |
|---|---|---|---|
| 3 (Ref.) | M-3 | 68,9 | 52,0 |
| 3.a (Vgl.) | M-3.a | 76,5 | 70,0 |
| 3.b (Vgl.) | M-3.b | 81,0 | 75,5 |
| 3.c (Vgl.) | M-3.c | 79,5 | 74,8 |
| 3.1 | M-3.1 | 85,2 | 79,0 |
| 3.2 | M-3.2 | 84,3 | 80,0 |
| 3.3 | M-3.3 | 84,7 | 80,2 |
| 3.4 | M-3.4 | 85,6 | 79,4 |
| 3.5 | M-3.5 | 86,1 | 82,0 |
| 3.6 | M-3.6 | 86,3 | 81,2 |

Überraschenderweise zeigen die Mischungen mit negativer dielektrischer Anisotropie eine besonders hervorragende Stabilisierung, wenn sie erfindungsgemäß die entsprechenden Verbindungen der Formel I enthalten, wobei vorteilhaft eine relativ geringe Menge ausreichend sein kann.

Des Weiteren werden die Mischungen M-3, M-3.a bis M-3.c und M-3.1 bis M-3.3 hinsichtlich ihrer Hitzebeständigkeit untersucht. Diese Untersuchungen werden wie bereits oben für die Mischungen M-1, M-1.a, M-1.b und M-1.c beschrieben ausgeführt.

Die Ergebnisse (VHR (heat)) werden in Tabelle 9 gezeigt.

Tabelle 9

| | Mischung | VHR (initial) [%] | VHR (heat) [%] |
|---|---|---|---|
| 3 (Ref.) | M-3 | 74,0 | 56,2 |
| 3.a (Vgl.) | M-3.a | 84,4 | 79,3 |
| 3.b (Vgl.) | M-3.b | 84,1 | 80,1 |
| 3.c (Vgl.) | M-3.c | 84,7 | 79,0 |
| 3.1 | M-3.1 | 88,1 | 84,8 |
| 3.2 | M-3.2 | 88,2 | 81,5 |
| 3.3 | M-3.3 | 89,1 | 77,3 |

Es zeigt sich, dass die erfindungsgemäßen Mischungen auch hinsichtlich der Hitzebeständigkeit deutlich stabilisiert werden können, auch bereits wenn sie die entsprechenden Verbindungen der Formel I in relativ niedrigen Konzentrationen enthalten.

Referenzbeispiel 4, Beispiele 4.1-4.3 und Vergleichsbeispiele 4.a-4.c

Die folgende Mischung (M-4) wird hergestellt und untersucht.

| Mischung M-4 | | | | | |
|---|---|---|---|---|---|
| Zusammensetzung | | | Physikalische Eigenschaften | | |
| Verbindung | | Konzentration | T(N, I) [°C)] | = | 86,5 |
| Nr. | Abkürzung | /Massen-% | $n_e$(20°C,589 nm) | = | 1,592 |
| 1 | CY-3-O2 | 12,00 | $\Delta$n(20°C,589 nm) | = | 0,109 |
| 2 | CY-3-O4 | 2,00 | $\varepsilon_\perp$(20°, 1 kHz) | = | 7,9 |

(fortgesetzt)

| Mischung M-4 | | | | | |
|---|---|---|---|---|---|
| Zusammensetzung | | | Physikalische Eigenschaften | | |
| 3 | CY-5-O2 | 12,00 | $\Delta\varepsilon$(20°, 1 kHz) | = | -4,2 |
| 4 | CCY-3-O1 | 6,00 | $k_{11}$(20°C) [pN] | = | 14,6 |
| 5 | CCY-3-O2 | 8,00 | $k_{33}$(20°C) [pN] | = | 16,6 |
| 6 | CCY-4-O2 | 8,00 | $V_0$(20°C) [V] | = | 2,08 |
| 7 | CPY-2-O2 | 9,00 | | | |
| 8 | CPY-3-O2 | 9,00 | | | |
| 9 | PYP-2-3 | 5,00 | | | |
| 10 | CC-3-V1 | 5,00 | | | |
| 11 | CC-3-V | 19,00 | | | |
| 12 | BCH-32 | 5,00 | | | |
| $\Sigma$ | | 100.0 | | | |

Die Mischung M-4 wird in 7 Teile geteilt und wie im weiteren beschrieben untersucht.

Der erste Teil der Mischung M-4 wird wie bereitgestellt im Referenzbeispiel 4 (Ref.) verwendet.

Drei der verbleibenden Teile wird jeweils TINUVIN®770 (T770) hinzugefügt, jeweils in einer Konzentration wie nachfolgend in Tabelle 10 aufgeführt. Die so hergestellten Mischungen M-4.a, M-4.b und M-4.c werden in den Vergleichsbeispielen 4.a bis 4.c (Vgl.) verwendet.

Den restlichen 3 Teilen wird erfindungsgemäß die Verbindung der wie oben gezeigten Formel I-1, jeweils in einer Konzentration wie nachfolgend in Tabelle 10 aufgeführt, hinzugefügt. Die so hergestellten Mischungen M-4.1 bis M-4.3 werden in den erfindungsgemäßen Beispielen 4.1 bis 4.3 verwendet.

Tabelle 10

| | Mischung | Stabilisator | Konzentration des Stabilisators [ppm] |
|---|---|---|---|
| 4 (Ref.) | M-4 | - | - |
| 4.a (Vgl.) | M-4.a | T770 | 100 |
| 4.b (Vgl.) | M-4.b | T770 | 500 |
| 4.c (Vgl.) | M-4.c | T770 | 1000 |
| 4.1 | M-4.1 | I-1 | 100 |
| 4.2 | M-4.2 | I-1 | 500 |
| 4.3 | M-4.3 | I-1 | 1000 |

Diese Mischungen werden wie nachfolgend beschrieben auf ihre Stabilität untersucht.

Zunächst wird das sogenannte initiale Spannungshaltevermögen (VHR (initial)) wie oben für die Mischungen M-1 bis M-1.16 beschrieben untersucht.

Die ermittelten Werte von VHR (initial) sind in Tabelle 11 aufgeführt.

Des Weiteren wird die Lichtbeständigkeit gegen Sonnenlicht wie oben für die Mischungen M-1 bis M-1.16 beschrieben untersucht.

Die für den Suntest ermittelten VHR-Werte (VHR (suntest)) sind ebenfalls in Tabelle 11 aufgeführt.

Tabelle 11

| | Mischung | VHR (initial) [%] | VHR (suntest) [%] |
|---|---|---|---|
| 4 (Ref.) | M-4 | 65,8 | 54,5 |
| 4.a (Vgl.) | M-4.a | 70,0 | 69,0 |
| 4.b (Vgl.) | M-4.b | 69,5 | 70,7 |
| 4.c (Vgl.) | M-4.c | 72,2 | 70,7 |

(fortgesetzt)

| | Mischung | VHR (initial) [%] | VHR (suntest) [%] |
|---|---|---|---|
| 4.1 | M-4.1 | 80,2 | 78,2 |
| 4.2 | M-4.2 | 79,1 | 80,1 |
| 4.3 | M-4.3 | 79,5 | 77,6 |

Überraschenderweise zeigen die Mischungen mit negativer dielektrischer Anisotropie eine besonders hervorragende Stabilisierung, wenn sie erfindungsgemäß die entsprechenden Verbindungen der Formel I enthalten, wobei vorteilhaft eine relativ geringe Menge ausreichend sein kann.

## Patentansprüche

**1.** Verbindung der Formel I

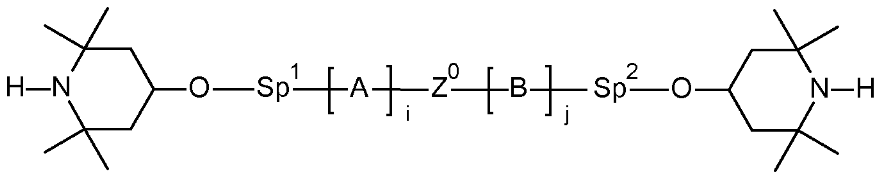 I

wobei

A und B jeweils unabhängig voneinander einen Rest ausgewählt aus den folgenden Gruppen

a) der Gruppe bestehend aus trans-1,4-Cyclohexylen, 1,4-Cyclohexenylen und 1,4'-Bicyclohexylen, worin auch eine oder zwei nicht benachbarte $CH_2$-Gruppen durch -O- und/oder -S- ersetzt sein können und worin auch ein oder mehrere H-Atome durch F ersetzt sein können,
b) der Gruppe bestehend aus 1,4-Phenylen und 1,3-Phenylen, worin auch eine oder zwei nicht benachbarte CH-Gruppen durch N ersetzt sein können und worin auch ein oder mehrere H-Atome durch L ersetzt sein können, und
c) der Gruppe bestehend aus

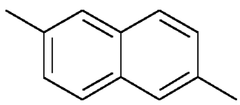 , 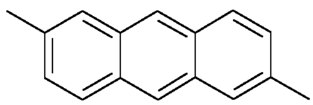 ,

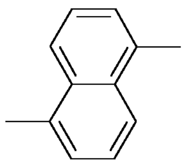 , 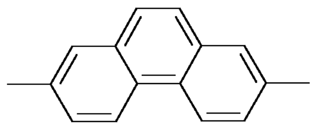 ,

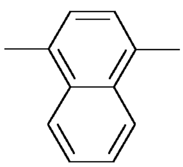 , 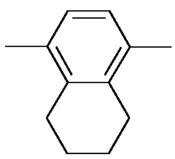 ,

wobei in diesen Resten auch ein oder mehrere H-Atome durch L ersetzt sein können, und/oder eine oder mehrere Doppelbindungen durch Einfachbindungen ersetzt sein können, und/oder eine oder mehrere CH-Gruppen durch N ersetzt sein können,
i und j jeweils unabhängig voneinander 0 oder 1,
$Z^0$ eine Einfachbindung, $-CH_2-$, $-CF_2-$, -CO-, -O-, -NH-, -NH-(CO)-, $-CH_2CH_2-$, -CH=CH-, $-CF_2O-$, $-OCF_2-$, $-CH_2O-$, $-OCH_2-$, -COO-, -OCO-, $-C_2F_4-$ oder -CF=CF-,

und

L bei jedem Auftreten gleich oder verschieden F, Cl, CN oder geradkettiges oder verzweigtes, jeweils optional fluoriertes, Alkyl, Alkoxy, Arylalkyl oder Alkylarylalkyl mit 1 bis 15 C-Atomen, worin auch eine oder mehrere nicht benachbarte $CH_2$-Gruppen durch -O- und/oder -S- ersetzt sein können,

bedeuten,
und wobei

$Sp^1$ und $Sp^2$ jeweils unabhängig voneinander Alkylen mit 1 bis 12 C-Atomen bedeuten, welches optional durch F, Cl oder CN ein- oder mehrfach substituiert ist, und worin auch eine oder mehrere nicht benachbarte $CH_2$-Gruppen jeweils unabhängig voneinander durch -O-, -S-, -NH-, -CH=CH- oder -C≡C-ersetzt sein können, wobei in der Verbindung keine zwei O-Atome direkt miteinander verbunden sind.

**2.** Verbindung nach Anspruch 1, welche ausgewählt ist aus Verbindungen der Formel I-A

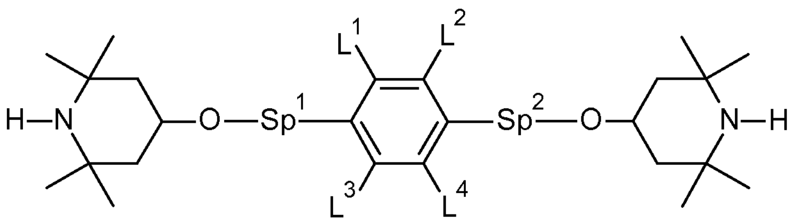
I-A

wobei

$Sp^1$ und $Sp^2$ die in Anspruch 1 angegebene Bedeutung haben

und

$L^1$ bis $L^4$ jeweils unabhängig voneinander H, F oder geradkettiges oder verzweigtes, jeweils optional fluoriertes, Alkyl mit 1 bis 7 C-Atomen, worin auch eine oder mehrere nicht benachbarte $CH_2$-Gruppen durch -O- ersetzt sein können,

bedeuten.

**3.** Verwendung der Verbindung nach Anspruch 1 oder 2 zur Stabilisierung eines flüssigkristallinen Mediums.

**4.** Verfahren zur Herstellung der Verbindung nach Anspruch 1 oder 2, wobei 4-Hydroxy-2,2,6,6-tetramethylpiperidinyloxyl als ein Ausgangsstoff verwendet wird.

**5.** Flüssigkristallines Medium enthaltend

a) eine oder mehrere Verbindungen nach Anspruch 1 oder 2 und
b) eine oder mehrere mesogene Verbindungen.

**6.** Flüssigkristallines Medium nach Anspruch 5, wobei der Bestandteil b) eine oder mehrere Verbindungen ausgewählt aus der Gruppe der Verbindungen der Formeln II-1 bis II-4

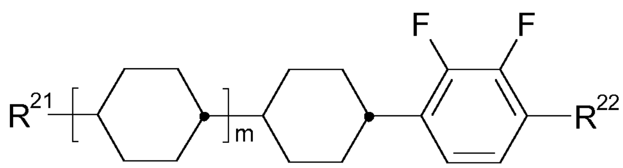

II-1

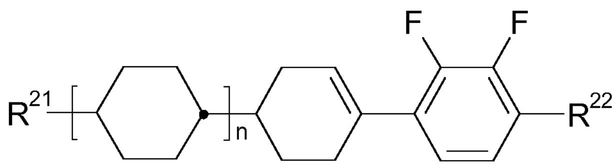

II-2

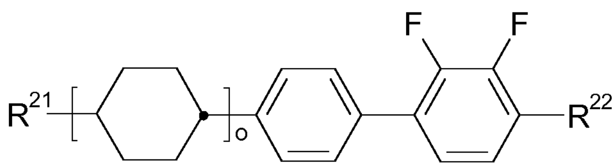

II-3

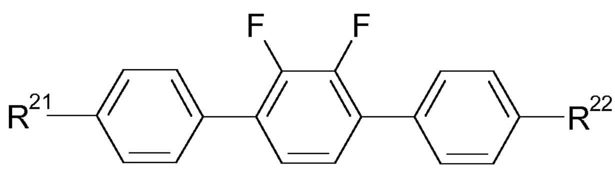

II-4

wobei

$R^{21}$ einen unsubstituierten Alkylrest mit 1 bis 7 C-Atomen,
$R^{22}$ einen unsubstituierten Alkylrest mit 1 bis 7 C-Atomen oder einen unsubstituierten Alkoxyrest mit 1 bis 6 C-Atomen,

und

m, n und o jeweils unabhängig voneinander 0 oder 1

bedeuten,
enthält.

**7.** Flüssigkristallines Medium nach Anspruch 5 oder 6, wobei es zusätzlich eine oder mehrere Verbindungen der Formel III-3 und/oder Formel IV,

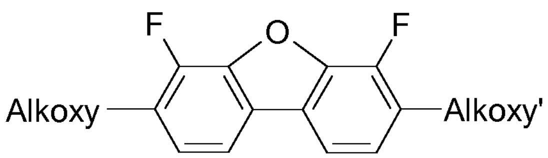

III-3

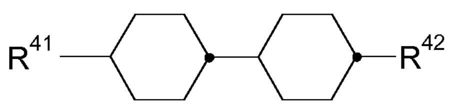

IV

wobei

Alkoxy, Alkoxy' unabhängig voneinander einen Alkoxyrest mit 1 bis 5 C-Atomen,

$R^{41}$ einen unsubstituierten Alkylrest mit 1 bis 7 C-Atomen oder einen unsubstituierten Alkenylrest mit 2 bis 7 C-Atomen,

und

$R^{42}$ einen unsubstituierten Alkylrest mit 1 bis 7 C-Atomen, einen unsubstituierten Alkoxyrest mit 1 bis 6 C-Atomen oder einen unsubstituierten Alkenylrest mit 2 bis 7 C-Atomen,

bedeuten,
enthält.

**8.** Flüssigkristallines Medium nach einem oder mehreren der Ansprüche 5 bis 7, wobei die Gesamtkonzentration der einen oder mehreren Verbindungen der Formel I im Gesamtmedium 5000 ppm oder weniger beträgt.

**9.** Elektrooptische Anzeige oder elektrooptische Komponente, wobei sie ein flüssigkristallines Medium nach einem oder mehreren der Ansprüche 5 bis 8 enthält.

## Claims

**1.** Compound of the formula I

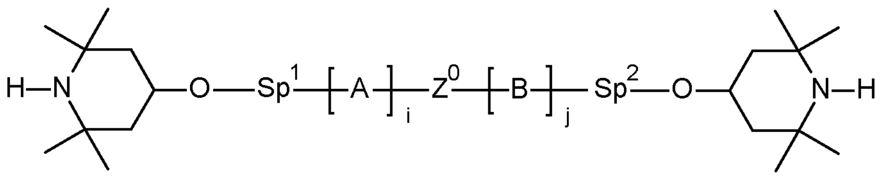 I

where

A and B in each case, independently of one another, denote a radical selected from the following groups:

a) the group consisting of trans-1,4-cyclohexylene, 1,4-cyclohexenylene and 1,4'-bicyclohexylene, in which, in addition, one or two non-adjacent $CH_2$ groups may be replaced by -O- and/or -S- and in which, in addition, one or more H atoms may be replaced by F,
b) the group consisting of 1,4-phenylene and 1,3-phenylene, in which, in addition, one or two non-adjacent CH groups may be replaced by N and in which, in addition, one or more H atoms may be replaced by L, and
c) the group consisting of

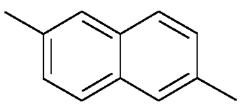 , 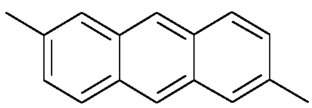 ,

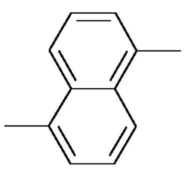 , 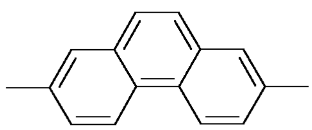 ,

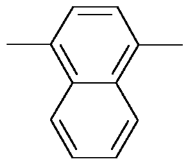 , 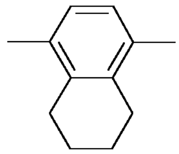 ,

where, in addition, one or more H atoms in these radicals may be replaced by L, and/or one or more double bonds may be replaced by single bonds, and/or one or more CH groups may be replaced by N,
i and j in each case, independently of one another, denote 0 or 1,
$Z^0$ denotes a single bond, $-CH_2-$, $-CF_2-$, -CO-, -O-, -NH-, -NH-(CO)-, $-CH_2CH_2-$, -CH=CH-, $-CF_2O-$, $-OCF_2-$, $-CH_2O-$, $-OCH_2-$, -COO-, -OCO-, $-C_2F_4-$ or -CF=CF-,

and

L on each occurrence, identically or differently, denotes F, Cl, CN or straight-chain or branched, in each case optionally fluorinated alkyl, alkoxy, arylalkyl or alkylarylalkyl having 1 to 15 C atoms, in which, in addition, one or more non-adjacent $CH_2$ groups may be replaced by -O- and/or -S-,

and where

$Sp^1$ and $Sp^2$ in each case, independently of one another, denote alkylene having 1 to 12 C atoms, which is optionally mono- or polysubstituted by F, Cl or CN, and in which, in addition, one or more non-adjacent $CH_2$ groups may in each case be replaced, independently of one another, by -O-, -S-, -NH-, -CH=CH- or -C≡C-, where no two O atoms in the compound are connected directly to one another.

**2.** Compound according to Claim 1, which is selected from compounds of the formula I-A

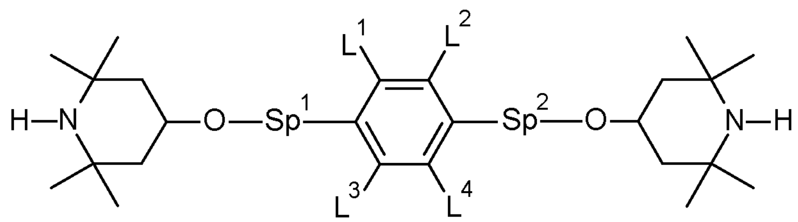 I-A

where

$Sp^1$ and $Sp^2$ have the meaning indicated in Claim 1

and

$L^1$ to $L^4$ in each case, independently of one another, denote H, F or straight-chain or branched, in each case optionally fluorinated alkyl having 1 to 7 C atoms, in which, in addition, one or more non-adjacent $CH_2$ groups may be replaced by -O-.

**3.** Use of the compound according to Claim 1 or 2 for the stabilisation of a liquid-crystalline medium.

**4.** Process for the preparation of the compound according to Claim 1 or 2, where 4-hydroxy-2,2,6,6-tetramethylpiperidinyloxyl is used as a starting material.

**5.** Liquid-crystalline medium comprising

a) one or more compounds according to Claim 1 or 2 and
b) one or more mesogenic compounds.

**6.** Liquid-crystalline medium according to Claim 5, where constituent b) comprises one or more compounds selected

from the group of the compounds of the formulae II-1 to II-4

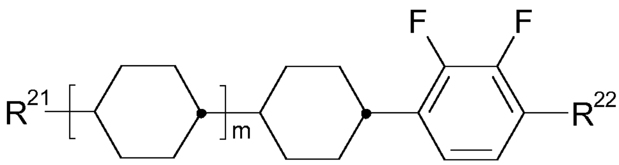

II-1

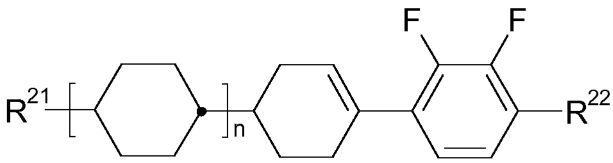

II-2

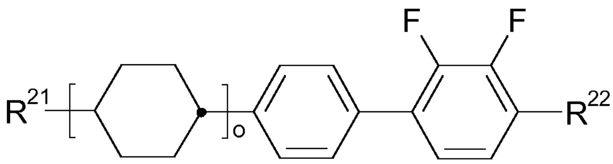

II-3

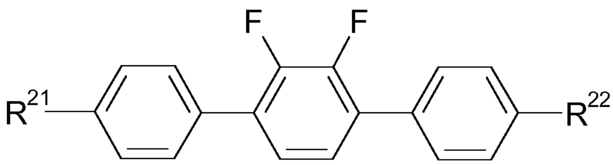

II-4

where

$R^{21}$ denotes an unsubstituted alkyl radical having 1 to 7 C atoms,
$R^{22}$ denotes an unsubstituted alkyl radical having 1 to 7 C atoms or an unsubstituted alkoxy radical having 1 to 6 C atoms,

and

m, n and o in each case, independently of one another, denote 0 or 1.

**7.** Liquid-crystalline medium according to Claim 5 or 6, which additionally comprises one or more compounds of the formula III-3 and/or formula IV

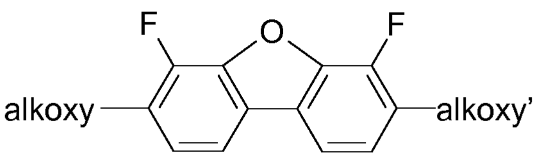

III-3

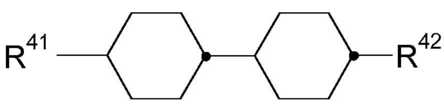

IV

where

alkoxy, alkoxy', independently of one another, denote an alkoxy radical having 1 to 5 C atoms,
$R^{41}$ denotes an unsubstituted alkyl radical having 1 to 7 C atoms or an unsubstituted alkenyl radical having 2

to 7 C atoms,

and

$R^{42}$ denotes an unsubstituted alkyl radical having 1 to 7 C atoms, an unsubstituted alkoxy radical having 1 to 6 C atoms or an unsubstituted alkenyl radical having 2 to 7 C atoms.

**8.** Liquid-crystalline medium according to one or more of Claims 5 to 7, where the total concentration of the one or more compounds of the formula I in the medium as a whole is 5000 ppm or less.

**9.** Electro-optical display or electro-optical component which contains a liquid-crystalline medium according to one or more of Claims 5 to 8.

## Revendications

**1.** Composé de la formule I

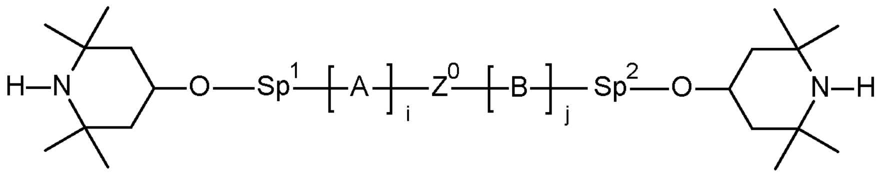 I

dans laquelle

A et B représentent, dans chaque cas de manière indépendante l'un de l'autre, un radical qui est sélectionné parmi les groupes qui suivent :

a) le groupe qui est constitué par trans-1,4-cyclohexylène, 1,4-cyclohexénylène et 1,4'-bicyclohexylène, où, en outre, un ou deux groupe(s) $CH_2$ non adjacents peut/peuvent être remplacé(s) par -O-et/ou par -S- et où, en outre, un ou plusieurs atome(s) de H peut/peuvent être remplacé(s) par F,
b) le groupe qui est constitué par 1,4-phénylène et 1,3-phénylène, où, en outre, un ou deux groupe(s) CH non adjacents peut/peuvent être remplacé(s) par N et où, en outre, un ou plusieurs atome(s) de H peut/peuvent être remplacé(s) par L, et
c) le groupe qui est constitué par

: 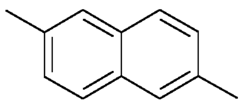 , 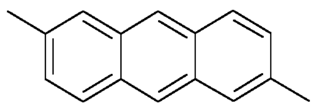 ,

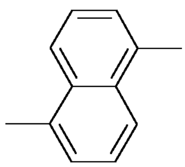 , 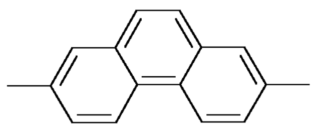 ,

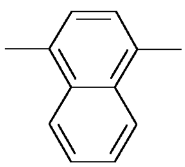 , 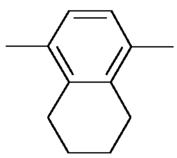 ,

où, en outre, un ou plusieurs atome(s) de H dans ces radicaux peut/peuvent être remplacé(s) par L, et/ou une

ou plusieurs liaison(s) double(s) peut/peuvent être remplacée(s) par des liaisons simples, et/ou un ou plusieurs groupe(s) CH peut/peuvent être remplacé(s) par N,
i et j représentent, dans chaque cas de manière indépendante l'un de l'autre, 0 ou 1,
$Z^0$ représente une liaison simple, $-CH_2-$, $-CF_2-$, -CO-, -O-, -NH-, -NH-(CO)-, $-CH_2CH_2-$, -CH=CH-, $-CF_2O-$, $-OCF_2-$, $-CH_2O-$, $-OCH_2-$, -COO-, -OCO-, $-C_2F_4-$ ou -CF=CF-,

et

L représente pour chaque occurrence, de manière identique ou différente, F, Cl, CN ou alkyle, alcoxy, arylalkyle ou alkyl-arylalkyle en chaîne droite ou ramifié, dans chaque cas en option fluoré qui comporte de 1 à 15 atome(s) de C, dans lequel, en outre, un ou plusieurs groupe(s) $CH_2$ non adjacents peut/peuvent être remplacé(s) par -O- et/ou par -S-,

et où

$Sp^1$ et $Sp^2$ représentent, dans chaque cas de manière indépendante l'un de l'autre, alkylène qui comporte de 1 à 12 atome(s) de C, lequel est en option mono- ou polysubstitué par F, par Cl ou par CN, et où, en outre, un ou plusieurs groupe(s) $CH_2$ non adjacents peut/peuvent dans chaque cas être remplacé(s), de manière indépendante les uns des autres, par -O-, -S-, -NH-, -CH=CH- ou -C≡C-, où deux atomes de O dans le composé sont connectés directement l'un à l'autre.

2. Composé selon la revendication 1, lequel est sélectionné parmi les composés de la formule I-A

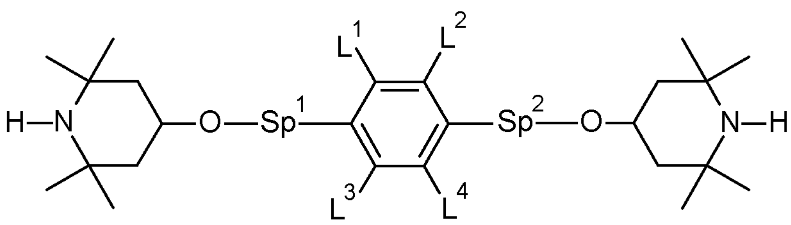
I-A

dans laquelle

$Sp^1$ et $Sp^2$ présentent la signification qui a été indiquée selon la revendication 1,

et

$L^1$ à $L^4$ représentent, dans chaque cas de manière indépendante les uns des autres, H, F ou alkyle en chaîne droite ou ramifié, dans chaque cas en option fluoré qui comporte de 1 à 7 atome(s) de C, où, en outre, un ou plusieurs groupe(s) $CH_2$ non adjacents peut/peuvent être remplacé(s) par -O-.

3. Utilisation du composé selon la revendication 1 ou 2 pour la stabilisation d'un milieu cristallin liquide.

4. Procédé pour la préparation du composé selon la revendication 1 ou 2, dans lequel du 4-hydroxy-2,2,6,6-tétraméthylpipéridinyloxyle est utilisé en tant que matériau de départ.

5. Milieu cristallin liquide comprenant

a) un ou plusieurs composé(s) selon la revendication 1 ou 2 et
b) un ou plusieurs composé(s) mésogène(s).

6. Milieu cristallin liquide selon la revendication 5, dans lequel le constituant b) comprend un ou plusieurs composé(s) qui est/sont sélectionné(s) parmi le groupe des composés des formules II-1 à II-4

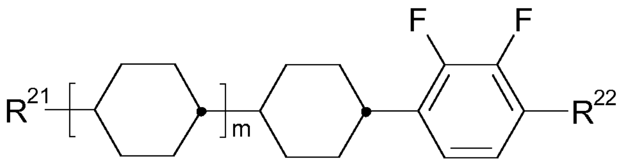

II-1

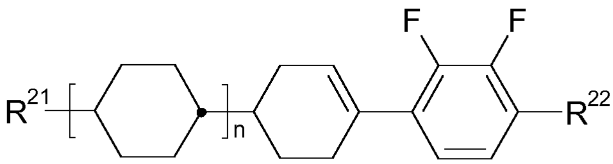

II-2

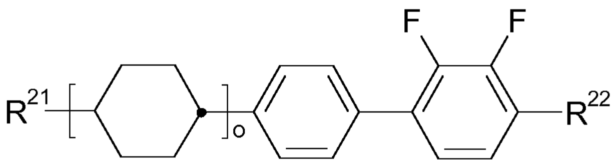

II-3

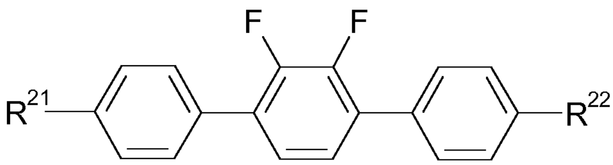

II-4

dans lesquelles

$R^{21}$ représente un radical alkyle non substitué qui comporte de 1 à 7 atome(s) de C,
$R^{22}$ représente un radical alkyle non substitué qui comporte de 1 à 7 atome(s) de C ou un radical alcoxy non substitué qui comporte de 1 à 6 atome(s) de C,

et

m, n et o représentent, dans chaque cas de manière indépendante les uns des autres, 0 ou 1.

**7.** Milieu cristallin liquide selon la revendication 5 ou 6, lequel comprend de façon additionnelle un ou plusieurs composé(s) de la formule III-3 et/ou de la formule IV

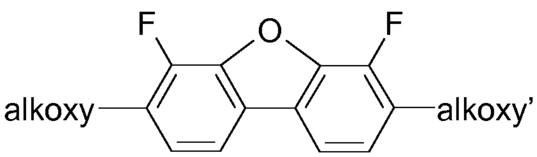

III-3

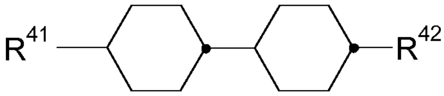

IV

dans lesquelles

alkoxy, alkoxy' représentent, de manière indépendante l'un de l'autre, un radical alcoxy qui comporte de 1 à 5 atome(s) de C,
$R^{41}$ représente un radical alkyle non substitué qui comporte de 1 à 7 atome(s) de C ou un radical alkényle non substitué qui comporte de 2 à 7 atomes de C,

et

$R^{42}$ représente un radical alkyle non substitué qui comporte de 1 à 7 atome(s) de C, un radical alcoxy non substitué qui comporte de 1 à 6 atome(s) de C ou un radical alkényle non substitué qui comporte de 2 à 7 atomes de C.

**8.** Milieu cristallin liquide selon une ou plusieurs des revendications 5 à 7, dans lequel la concentration totale des un ou plusieurs composés de la formule I dans le milieu pris dans sa globalité est de 5 000 ppm ou moins.

**9.** Affichage électro-optique ou composant électro-optique qui contient un milieu cristallin liquide selon une ou plusieurs des revendications 5 à 8.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009129911 A1
- EP 2182046 A1
- WO 2008009417 A1
- WO 2009021671 A1
- WO 2009115186 A1
- JP S55023169 A
- JP H05117324 A
- WO 0218515 A1
- JP H09291282 A
- EP 2982731 A1
- DE 102015013980 A1
- WO 2016111027 A1
- WO 2015079797 A1
- EP 0240379 A1


### In der Beschreibung aufgeführte Nicht-Patentliteratur


- **M.F. SCHIECKEL ; K. FAHRENSCHON.** Deformation of nematic liquid crystals with vertical orientation in electrical fields. *Appl. Phys. Lett.,* 1971, vol. 19, 3912
- **J.F. KAHN.** *Appl. Phys. Lett.,* 1972, vol. 20, 1193
- **G. LABRUNIE ; J. ROBERT.** *J. Appl. Phys.,* 1973, vol. 44, 4869
- **J. ROBERT ; F. CLERC.** *SID 80 Digest Techn. Papers,* 1980, 30
- **J. DUCHENE.** *Displays,* 1986, vol. 7, 3
- **H. SCHAD.** *SID 82 Digest Techn. Papers,* 1982, 244
- **S.H. LEE ; S. L. LEE ; H. Y. KIM.** *Appl. Phys. Lett.,* 1998, vol. 73 (20), 2881-2883
- **S. TOGASHI ; K. SEKIGUCHI ; H. TANABE ; E. YAMAMOTO ; K. SORIMACHI ; E. TAJIMA ; H. WATANABE ; H. SHIMIZU.** A 210-288 Matrix LCD Controlled by Double Stage Diode Rings. *Proc. Eurodisplay 84,* September 1984, 141 ff
- **M. STROMER.** Design of Thin Film Transistors for Matrix Adressing of Television Liquid Crystal Displays. *Proc. Eurodisplay 84,* September 1984, 145 ff
- **S.D. YEO.** A LC Display for the TV Application. *SID 2004 International Symposium, Digest of Technical Papers, XXXV, Buch II,* 2004, 758, , 759
- **H. YOSHIDE et al.** MVA LCD for Notebook or Mobile PCs. *SID 2004 International Symposium, Digest of Technical Papers, XXXV, Buch I,* 2004, 6-9
- **C.T. LIU et al.** A 46-inch TFT-LCD HDTV Technology. *SID 2004 International Symposium, Digest of Technical Papers, XXXV, Buch II,* 2004, 750-753
- **KIM, SANG SOO.** Super PVA Sets New State-of-the-Art for LCD-TV. *SID 2004 International Symposium, Digest of Technical Papers, XXXV, Buch II,* 2004, 760-763
- **SHIGETA, MITZUHIRO ; FUKUOKA, HIROFUMI.** Development of High Quality LCDTV. *SID 2004 International Symposium, Digest of Technical Papers, XXXV, Buch II,* 2004, 754-757
- **SOUK, JUN.** Seminar M-6: "Recent Advances in LCD Technology. *Seminar Lecture Notes,* 2004, M-6, , 1-M-6, 26
- **MILLER, IAN.** Seminar M-7: "LCD-Television. *Seminar Lecture Notes,* 2004, M-7, , 1-M-7, 32
- **KIM, HYEON KYEONG et al.** A 57-in. Wide UXGA TFT-LCD for HDTV Application. *SID 2004 International Symposium, Digest of Technical Papers, XXXV, Buch I,* 2004, 106-109
- **OHKATSU, Y.** *J. of Japan Petroleum Institute,* 2008, vol. 51, 191-204
- **MIÉVILLE, P. et al.** *Angew. Chem.,* 2010, vol. 122, 6318-6321
- *Pure Appl. Chem.,* 2001, vol. 73 (5), 888
- **C. TSCHIERSKE ; G. PELZL ; S. DIELE.** *Angew. Chem.,* 2004, vol. 116, 6340-6368
- Merck Liquid Crystals, Physical Properties of Liquid Crystals. Merck KGaA, November 1997